(19) Europäisches Patentamt
European Patent Office
Office européen des brevets

(11) Publication number: 0 526 001 A1

# (12) EUROPEAN PATENT APPLICATION

(21) Application number: 92306106.3

(22) Date of filing: 01.07.92

(51) Int. Cl.[5]: C07D 249/12, C07D 407/12, C07D 409/12, C07D 413/12, A61K 31/41

(30) Priority: 03.07.91 US 725720
20.12.91 US 812891

(43) Date of publication of application:
03.02.93 Bulletin 93/05

(84) Designated Contracting States:
AT BE CH DE DK ES FR GB GR IT LI LU NL PT SE

(71) Applicant: MERCK & CO. INC.
126, East Lincoln Avenue P.O. Box 2000
Rahway New Jersey 07065-0900 (US)

(72) Inventor: Ashton, Wallace T.
122 Sweet Briar Drive
Clark, NJ 07066 (US)
Inventor: Chang, Linda L.
45 Brandon Avenue
Wayne, NJ 07470 (US)
Inventor: Maccoss, Malcolm
48 Rose Court
Freehold, NJ 07728 (US)
Inventor: Chakravarty, Prasun K.
16 Churchill Road
Edison, NJ 08820 (US)
Inventor: Greenlee, William J.
115 Herrick Avenue
Teaneck, NJ 07666 (US)
Inventor: Patchett, Arthur A.
1090 Minisink Way
Westfield, NJ 07090 (US)
Inventor: Walsh, Thomas F.
127 Marion Avenue
Westfield, NJ 07090 (US)
Inventor: Flanagan, Kelly
1618 Raspberry Court
Edison, NJ 08820 (US)
Inventor: Rivero, Ralph A.
49 Columbia Drive
Tinton Falls, NJ 07724 (US)

(74) Representative: Thompson, John Dr. et al
Merck & Co., Inc. European Patent
Department Terlings Park Eastwick Road
Harlow, Essex CM20 2QR (GB)

## (54) Substituted triazolinones.

(57) Substituted triazolinone compounds are angiotensin II antagonists and therefore useful in the treatment of hypertension, and related cardiovascular disorders and ocular hypertension. These compounds have the general formula:

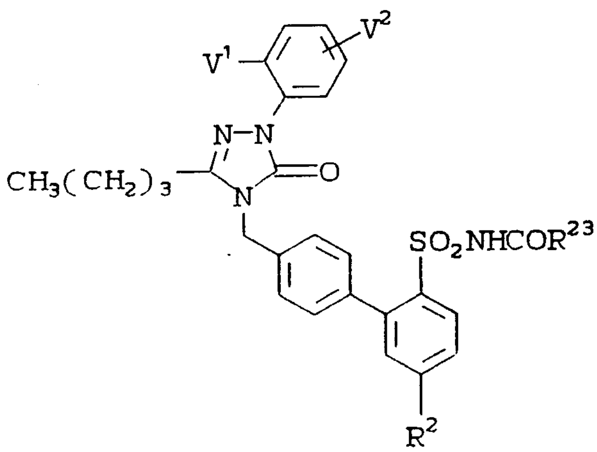

EP 0 526 001 A1

## INTRODUCTION OF THE INVENTION:

This invention relates to novel substituted triazolinone compounds and derivatives thereof which are angiotensin II antagonists useful in the treatment of hypertension and related cardiovascular disorders and in ocular hypertension.

## BACKGROUND OF THE INVENTION

Renin-angiotensin system (RAS) plays a central role in the regulation of normal blood pressure and seems to be critically involved in hypertension development and maintenance as well as congestive heart failure. Angiotensin II (A II) is an octapeptide hormone produced mainly in the blood during the cleavage of angiotensin I by angiotensin converting enzyme (ACE) localized on the endothelium of blood vessels of lung, kidney, and many other organs. It is the end product of the renin-angiotensin system (RAS) and is a powerful arterial vasoconstrictor that exerts its action by interacting with specific receptors present on cell membranes. One of the possible modes of controlling the RAS is angiotensin II receptor antagonism. Several peptide analogs of A II are known to inhibit the effect of this hormone by competitively blocking the receptors, but their experimental and clinical applications have been limited by the partial agonist activity and lack of oral absorption [M. Antonaccio. Clin. Exp. Hypertens. A4, 27-46 (1982); D. H. P. Streeten and G. H. Anderson, Jr - Handbook of Hypertension, Clinical Pharmacology of Antihypertensive Drugs, ed. A. E. Doyle, Vol 5, pp. 246-271, Elsevier Science Publisher, Amsterdam, The Netherlands, 1984].

Recently, several non-peptide compounds have been described as A II antagonists. Illustrative of such compounds are those disclosed in U.S. Patents 4,207,324; 4,340,598; 4,576,958; 4,582,847; and 4,880,804; in European Patent Applications 028,834; 245,637; 253,310; and 291,969; and in articles by A.T. Chiu, et al. [Eur. J. Pharm. Exp. Therap, 157, 13-21 (1988)] and by P.C. Wong, et al. [J. Pharm. Exp. Therap, 247, 1-7(1988)]. All of the U.S. Patents, European Patent Applications 028,834 and 253,310 and the two articles disclose substituted imidazole compounds which are generally bonded through a lower alkyl bridge to a substituted phenyl. European Patent Application 412,594 and WO/91/18888 disclose triazolinones similar to those described herein which are also A-II antagonist.

## DETAILED DESCRIPTION OF THE INVENTION

The novel compounds of this invention have the general formula (I):

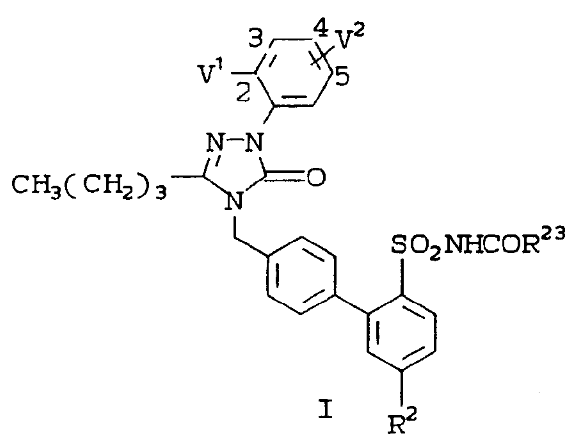

I

or a pharmaceutically acceptable salt thereof, wherein $V^1$ is H, $CH_3$, $CF_3$ or halogen with the proviso that $V^1$ is $CF_3$ when $V^2$ is H;

$V^2$ is a group at the 3-, 4-, or 5-position selected from:

(a) H;

(b) $-NO_2$;

(c) $-NR^{10}R^{21}$;

(d) $-N(CH_2CH_2)_2L$;

(e) $-NR^{21}COR^{22}$;

(f) $-NR^{21}CO_2R^{22}$;

(g) $-NR^{21}CONR^{21}R^{22}$;

(h) $-NR^{21}CON(CH_2CH_2)_2L$;
(i) $-CONR^{21}R^{22}$;
(j) $-CON(CH_2CH_2)_2L$; and
(k) $-CO-(C_1-C_5\text{-alkyl})$;

wherein L is a single bond, $CH_2$, O, $S(O)_p$, or $NR^9$, wherein p is 0 to 2;

with the proviso that $V^2$ is restricted to (e)-(k) above when $V^1$ is H;

$R^2$ is H, $C_1$-$C_4$-alkyl, $C_1$-$C_4$-alkoxy or halo;
$R^9$ is H, $C_1$-$C_5$-alkyl, aryl or $-CH_2$-aryl;
$R^{10}$ is H or $C_1$-$C_4$-alkyl;
$R^{21}$ is H or $R^{22}$ wherein
$R^{22}$ is:

(a) $C_1$-$C_6$-alkyl, $C_2$-$C_6$-alkenyl, or $C_2$-$C_6$-alkynyl, each of which is unsubstituted or substituted with one or more substituents selected from the group consisting of aryl, heteroaryl, $C_3$-$C_6$-cycloalkyl, halo, -OH, -O-$C_1$-$C_4$-alkyl, -S-$C_1$-$C_4$-alkyl, -O-phenyl, or -S-phenyl;
(b) $C_3$-$C_7$-cycloalkyl unsubstituted or substituted with one or more substituents selected from the group consisting of $C_1$-$C_4$-alkyl, halo or phenyl;
(c) aryl;
(d) heteoaryl;

$R^{23}$ is:

(a) phenyl, unsubstituted or substituted with one or two substituents selected from Cl, -Br, -F, -I, $-CH_3$ and $-CF_3$, at least one of which occupies an ortho-position;
(b) heteroalkyl, such as furan-2-yl, thiophen-2-yl, benzo[b]furan-2-yl, benzo[b]thiophen-2-yl, furan-3-yl, thiophen-3-yl, or oxazol-5-yl, unsubstituted or substituted with one or two substituents selected from -Cl, -Br, -F, -I, $-CH_3$ and $CF_3$ wherein at least one of the substituents is located adjacent to the carbonyl substituent and/or to a ring heteroatom;
(c) branched $C_3$-$C_6$-alkyl;
(d) $C_3$-$C_7$-cycloalkyl, unsubstituted or substituted at the 1-and/or 2-position with one to three substituents selected from -Cl, -Br, -F, -I, $-CH_3$ and $-CH_2CH_3$;
(e) $C_7$-$C_8$-bi- or tricycloalkyl;
(f) saturated 5- or 6-membered heterocyclyl linked through a carbon atom and containing one or two heteroatoms selected from oxygen and sulfur such as tetrahydrofuryl, 1,3-dithiolan-2-yl, or 1,3-dithian-2-yl.

The terms "alkyl", "alkenyl", "alkynyl" and the like include both the straight chain and branched chain species of these generic terms wherein the number of carbon atoms in the species permit. Unless otherwise noted, the specific names for these generic terms shall mean the straight chain species. For example, the term "butyl" shall mean the normal butyl substituent, n-butyl.

The terms "halo" and halogen mean Cl, Br, I or F.

The term "aryl" is defined as phenyl or naphthyl, unsubstituted or substituted with one, two, or three substituents selected from the group consisting of $C_1$-$C_4$-alkyl, $C_1$-$C_4$-alkoxy, halo, $CF_3$, $C_1$-$C_4$-alkyl-$S(O)_p$-, $CF_3SO_2$-, and -CN.

The term "heteroaryl" is defined as a 5- or 6-membered aromatic ring consisting of carbon and from 1 to 2 heteroatoms selected from the group consisting of N, O, and S, such as pyridine, pyrimidine, pyrazine, triazine, furan, thiophene, oxazole, thiazole, imidazole, or the like, which can be unsubstituted or substituted wherein the substituents are selected from the group consisting of $C_1$-$C_4$-alkyl,
$C_1$-$C_4$-alkoxy, $-CF_3$, and halo.

One embodiment of the novel compounds of this invention comprises compounds of formula I or a pharmaceutically acceptable salt thereof wherein:

$V^1$ is H, $CH_3$, $CF_3$, or halogen;

$V^2$ is a group at the 3-, 4-, or 5-position selected from:

(a) $-NO_2$,
(b) $-NR^{10}R^{21}$,
(c) $-N(CH_2CH_2)_2L$,
(d) $-NR^{21}COR^{22}$,
(e) $-NR^{21}CO_2R^{22}$,
(f) $-NR^{21}CONR^{21}R^{22}$,
(g) $-NR^{21}CON(CH_2CH_2)_2L$,
(h) $-CONR^{21}R^{22}$,
(i) $-CON(CH_2CH_2)_2L$ and
(j) $-CO-(C_1-C_5\text{-alkyl})$;

wherein L is a single bond, $CH_2$, O, $S(O)_p$, or $NR^9$, wherein p is 0 to 2;
with the proviso that $V^2$ is restricted to (d)-(j) above when $V^1$ is H.

A class of compounds within this embodiment comprises compounds of formula I wherein:
$V^1$ is $CH_3$, $CF_3$, or halogen;
$V^2$ is a group at the 3- or 5-position selected from:
(a) $-NR^{21}COR^{22}$,
(b) $-NR^{21}CO_2R^{22}$,
(c) $-NR^{21}CONR^{21}R^{22}$,
(d) $-NR^{21}CON(CH_2CH_2)_2L$,
(e) $-CONR^{21}R^{22}$,
(f) $-CON(CH_2CH_2)_2L$ and
(g) -CO-($C_1$-$C_5$-alkyl).

A subclass of compounds within this class comprises compounds of formula I wherein:
$V^1$ is $CF_3$, or halogen;
$V^2$ is a group at the 5-position selected from:
(a) $-NR^{21}COR^{22}$,
(b) $-NR^{21}CO_2R^{22}$,
(c) $-NR^{21}CONR^{21}R^{22}$,
(d) $-NR^{21}CON(CH_2CH_2)_2L$,
(e) $-CONR^{21}R^{22}$,
(f) $-CON(CH_2CH_2)_2L$, and
(g) -CO-($C_1$-$C_5$-alkyl);
$R^2$ is H or $C_1$-$C_4$-alkyl;
$R^{21}$ is H or $CH_3$;
$R^{22}$ is:
(a) $C_1$-$C_6$-alkyl, unsubstituted or substituted with one or more substituents selected from the group consisting of aryl, heteroaryl, $C_3$-$C_6$-cycloalkyl, -O-$C_1$-$C_4$-alkyl, -S-$C_1$-$C_4$-alkyl, -O-phenyl, or -S-phenyl;
(b) $C_3$-$C_7$-cycloalkyl unsubstituted or substituted with one or more substituents selected from the group consisting of $C_1$-$C_4$-alkyl or phenyl;
(c) aryl; or
(d) heteroaryl.

Another embodiment of the novel compounds of this invention is that wherein $V^1$ is $CF_3$ and $V^2$ is H. Compounds exemplifying this invention are:

(1) 4-[[2′-(N-benzoylsulfamoyl)biphenyl-4-yl]-methyl]-5-n-butyl-2,4-dihydro-2-[2-(trifluoromethyl)phenyl]-3H-1,2,4-triazol-3-one;
(2) 5-n-butyl-4-[[2′-[N-(cyclopropanecarbonyl)sulfamoyl]biphenyl-4-yl]methyl]-2,4-dihydro-2-[2-(trifluoromethyl)phenyl]-3H-1,2,4-triazol-3-one;
(3) 5-n-butyl-2,4-dihydro-4-[[2′-[N-[(S)-2,2-dimethylcyclopropanecarbonyl]sulfamoyl]biphenyl-4-yl]methyl]-2-[2-(trifluoromethyl)phenyl]-3H-1,2,4-triazol-3-one;
(4) 5-n-butyl-2,4-dihydro-4-[[2′-(N-pivaloylsulfamoyl)biphenyl-4-yl]methyl]-2-[2-(trifluoromethyl)phenyl]-3H-1,2,4-triazol-3-one;
(5) 5-n-butyl-2,4-dihydro-4-[[2′-[N-(2-norbornanecarbonyl)sulfamoyl]biphenyl-4-yl]methyl]-2-[2-(trifluoromethyl)phenyl]-3H-1,2,4-triazol-3-one;
(6) 5-n-butyl-4-[[2′-[N-(cycloheptanecarbonyl)-sulfamoyl]biphenyl-4-yl]methyl]-2,4-dihydro-2-[2-(trifluoromethyl)phenyl]-3H-1,2,4-triazol-3-one;
(7) 5-n-butyl-4-[[2′-[N-(2,2-dichloro-1-methylcyclopropanecarbonyl)sulfamoyl]-biphenyl-4-yl]-methyl]-2,4-dihydro-2-[2-(trifluoromethyl)-phenyl]-3H-1,2,4-triazol-3-one;
(8) 5-n-butyl-2,4-dihydro-4-[[2′-[N-(2-methylbenzoyl)sulfamoyl]biphenyl-4-yl]methyl]-2-[2-(trifluoromethyl)phenyl]-3H-1,2,4-triazol-3-one;
(9) 5-n-butyl-2,4-dihydro-4-[[2′-(N-isobutyrylsulfamoyl)biphenyl-4-yl]methyl]-2-[2-(trifluoromethyl)phenyl]-3H-1,2,4-triazol-3-one;
(10) 5-n-butyl-2,4-dihydro-4-[[2′-[N-[2-(trifluoromethyl)benzoyl]sulfamoyl]biphenyl-4-yl]methyl]-2-[2-(trifluoromethyl)phenyl]-3H-1,2,4-triazol-3-one;
(11) 5-n-butyl-2,4-dihydro-4-[[2′-[N-(2-methylcyclopropanecarbonyl)sulfamoyl]biphenyl-4-yl]-methyl]-2-[2-(trifluoromethyl)phenyl]-3H-1,2,4-triazol-3-one;
(12) 5-n-butyl-4-[[2′-[N-(2,2-difluorocyclopropanecarbonyl)sulfamoyl]biphenyl-4-yl]methyl]-2,4-dihydro-2-[2-(trifluoromethyl)phenyl]-3H-1,2,4-triazol-3-one;
(13) 5-n-butyl-4-[[2′-[N-(cyclobutanecarbonyl)-sulfamoyl]biphenyl-4-yl]methyl]-2,4-dihydro-2-[2-(trifluoromethyl)phenyl]-3H-1,2,4-triazol-3-one;

(14) 5-n-butyl-4-[[2′-[N-(cyclopentanecarbonyl)sulfamoyl]biphenyl-4-yl]methyl]-2,4-dihydro-2-[2-(trifluoromethyl) phenyl]-3H-1,2,4-triazol-3-one;

(15) 5-n-butyl-2,4-dihydro-4-[[2′-[N-(3,3-dimethylbutyryl)sulfamoyl]biphenyl-4-yl]methyl]-2-[2-(trifluoromethyl) phenyl]-3H-1,2,4-triazol-3-one;

(16) 5-n-butyl-2,4-dihydro-4-[[2′-[N-(2-furoyl)sulfamoyl]biphenyl-4-yl]methyl]-2-[2-(trifluoromethyl)phenyl]-3H-1,2,4-triazol-3-one;

(17) 5-n-butyl-2,4-dihydro-4-[[2′-[N-(3-furoyl)sulfamoyl]biphenyl-4-yl]methyl]-2-[2-(trifluoromethyl) phenyl]-3H-1,2,4-triazol-3-one;

(18) 5-n-butyl-2,4-dihydro-4-[[2′-[N-(2-thiophenecarbonyl)sulfamoyl]biphenyl-4-yl]methyl]-2-[2-(trifluoromethyl) phenyl]-3H-1,2,4-triazol-3-one;

(19) 5-n-butyl-2,4-dihydro-4-[[2′-[N-(3-thiophenecarbonyl)sulfamoyl]biphenyl-4-yl]methyl]-2-[2-(trifluoromethyl)phenyl]-3H-1,2,4-triazol-3-one;

(20) 5-n-butyl-2,4-dihydro-4-[[2′-[N-(tetrahydro-2-furoyl)sulfamoyl]biphenyl-4-yl]methyl]-2-[2-(trifluoromethyl) phenyl]-3H-1,2,4-triazol-3-one;

(21) 5-n-butyl-2,4-dihydro-4-[[2′-[N-(tetrahydro-3-furoyl)sulfamoyl]biphenyl-4-yl]methyl]-2-[2-(trifluoromethyl) phenyl]-3H-1,2,4-triazol-3-one;

(22) 5-n-butyl-2,4-dihydro-4-[[2′-[N-(3-methyl-2-furoyl)sulfamoyl]biphenyl-4-yl]methyl]-2-[2-(trifluoromethyl) phenyl]-3H-1,2,4-triazol-3-one;

(23) 5-n-butyl-2,4-dihydro-4-[[2′-[N-(3-methyl-2-thiophenecarbonyl)sulfamoyl]biphenyl-4-yl]methyl]-2-[2-(trifluoromethyl)phenyl]-3H-1,2,4-triazol-3-one;

(24) 5-n-butyl-2,4-dihydro-4-[[2′-[N-(5-methyl-2-thiophenecarbonyl)sulfamoyl]biphenyl-4-yl]-methyl]-2-[2-(trifluoromethyl)phenyl]-3H-1,2,4-triazol-3-one;

(25) 5-n-butyl-2,4-dihydro-4-[[2′-[N-(2-methyl-3-furoyl)sulfamoyl]biphenyl-4-yl]methyl]-2-[2-(trifluoromethyl) phenyl]-3H-1,2,4-triazol-3-one;

(26) 5-n-butyl-2,4-dihydro-4-[[2′-[N-(2,5-dimethyl-3-furoyl)sulfamoyl]biphenyl-4-yl]methyl]-2-[2-(trifluoromethyl) phenyl]-3H-1,2,4-triazol-3-one;

(27) 4-[[2′-[N-(5-bromo-2-furoyl)sulfamoyl]biphenyl-4-yl]methyl]-5-n-butyl-2,4-dihydro-2-[2-(trifluoromethyl)phenyl]-3H-1,2,4-triazol-3-one;

(28) 5-n-butyl-4-[[2′-[N-(3-chloro-2-thiophenecarbonyl)sulfamoyl]biphenyl-4-yl]methyl]-2,4-dihydro-2-[2-(trifluoromethyl)phenyl]-3H-1,2,4-triazol-3-one;

(29) 5-n-butyl-4-[[2′-[N-(5-chloro-2-thiophenecarbonyl)sulfamoyl]biphenyl-4-yl]methyl]-2,4-dihydro-2-[2-(trifluoromethyl)phenyl]-3H-1,2,4-triazol-3-one;

(30) 5-n-butyl-4-[[2′-[N-(2,5-dichloro-3-thiophenecarbonyl)sulfamoyl]biphenyl-4-yl]methyl]-2,4-dihydro-2-[2-(trifluoromethyl)phenyl]-3H-1,2,4-triazol-3-one;

(31) 5-n-butyl-4-[[2′-[N-(3,4-dichloro-2-furoyl)sulfamoyl]biphenyl-4-yl]methyl]-2,4-dihydro-2-[2-(trifluoromethyl)phenyl]-3H-1,2,4-triazol-3-one;

(32) 5-n-butyl-2,4-dihydro-4-[[2′-[N-(4-methyloxazole-5-carbonyl)sulfamoyl]biphenyl-4-yl]methyl]-2-[2-(trifluoromethyl)phenyl]-3H-1,2,4-triazol-3-one;

(33) 4-[[2′-[N-(benzofuran-2-carbonyl)sulfamoyl]biphenyl-4-yl]methyl]-5-n-butyl-2,4-dihydro-2-[2-(trifluoromethyl) phenyl]-3H-1,2,4-triazol-3-one;

(34) 5-n-butyl-2,4-dihydro-4-[[2′-[N-(1-methylcyclopropanecarbonyl)sulfamoyl]biphenyl-4-yl]-methyl]-2-[2-(trifluoromethyl)phenyl]-3H-1,2,4-triazol-3-one;

(35) 5-n-butyl-2,4-dihydro-4-[[2′-[N-(1,3-dithiolane-2-carbonyl)sulfamoyl]biphenyl-4-yl]methyl]-2-[2-(trifluoromethyl)phenyl]-3H-1,2,4-triazol-3-one;

(36) 5-n-butyl-2,4-dihydro-4-[[2′-[N-[5-methyl-2-(trifluoromethyl)-3-furoyl]sulfamoyl]biphenyl-4-yl]methyl]-2-[2-(trifluoromethyl)phenyl]-3H-1,2,4-triazol-3-one;

(37) 4-[[2′-[N-(benzo[b]thiophene-2-carbonyl)sulfamoyl]biphenyl-4-yl]methyl]-5-n-butyl-2,4-dihydro-2-[2-(trifluoromethyl)phenyl]-3H-1,2,4-triazol-3-one;

(38) 5-n-butyl-2,4-dihydro-4-[[2′-[N-(3-methylbenzofuran-2-carbonyl)sulfamoyl]biphenyl-4-yl]-methyl]-2-[2-(trifluoromethyl)phenyl]-3H-1,2,4-triazol-3-one;

(39) 5-n-butyl-4-[[2′-[N-(2-chlorobenzoyl)sulfamoyl]-biphenyl-4-yl]methyl]-2,4-dihydro-2-[2-(trifluoromethyl)phenyl]-3H-1,2,4-triazol-3-one;

(40) 5-n-butyl-4-[[2′-[N-(cyclohexanecarbonyl)sulfamoyl]biphenyl-4-yl]methyl]-2,4-dihydro-2-[2-(trifluoromethyl)phenyl]-3H-1,2,4-triazol-3-one;

(41) 5-n-butyl-2,4-dihydro-4-[[2′-[N-(2-ethyl-2-methylcyclopropanecarbonyl)sulfamoyl]biphenyl-4-yl]methyl]-2-[2-(trifluoromethyl)phenyl]-3H-1,2,4-triazol-3-one;

(42) 5-n-butyl-2,4-dihydro-4-[[2′-[N-(2-fluorobenzoyl)sulfamoyl]biphenyl-4-yl]methyl]-2-[2-(trifluoromethyl)phenyl]-3H-1,2,4-triazol-3-one;
(43) 5-n-butyl-2,4-dihydro-4-[[2′-[N-(2,5-dichlorobenzoyl)sulfamoyl]biphenyl-4-yl]methyl]-2-[2-(trifluoromethyl)phenyl]-3H-1,2,4-triazol-3-one;
(44) 5-n-butyl-4-[[2′-[N-(2-bromobenzoyl)sulfamoyl]-biphenyl-4-yl]methyl]-2,4-dihydro-2-[2-(trifluoromethyl)phenyl]-3H-1,2,4-triazol-3-one;
(45) 5-n-butyl-2,4-dihydro-4-[[2′-[N-(5-methyl-2-furoyl)sulfamoyl]biphenyl-4-yl]methyl]-2-[2-(trifluoromethyl) phenyl]-3H-1,2,4-triazol-3-one;
(46) 5-n-butyl-4-[[2′-[N-(2,2-dichlorocyclopropanecarbonyl)sulfamoyl]biphenyl-4-yl]methyl]-2,4-dihydro-2-[2-(trifluoromethyl)phenyl]-3H-1,2,4-triazol-3-one;
(47) 5-n-butyl-4-[[2′-[N-(3-chloro-2-furoyl)sulfamoyl]biphenyl-4-yl]methyl]-2,4-dihydro-2-[2-(trifluoromethyl)phenyl]-3H-1,2,4-triazol-3-one;
(48) 5-n-butyl-4-[[2′-[N-(3-chloro-5-methyl-2-furoyl)sulfamoyl]biphenyl-4-yl]methyl]-2,4-dihydro-2-[2-(trifluoromethyl)phenyl]-3H-1,2,4-triazol-3-one;
(49) 5-n-butyl-4-[[2′-[N-(3-chlorobenzofuran-2-carbonyl)sulfamoyl]biphenyl-4-yl]methyl]-2,4-dihydro-2-[2-(trifluoromethyl)phenyl]-3H-1,2,4-triazol-3-one;
(50) 5-n-butyl-2,4-dihydro-4-[[2′-[N-(1-methylcyclobutanecarbonyl)sulfamoyl]biphenyl-4-yl]methyl]-2-[2-(trifluoromethyl)phenyl]-3H-1,2,4-triazol-3-one;
(51) 5-n-butyl-2,4-dihydro-4-[[2′-[N-[(R)-2,2-dimethylcyclopropanecarbonyl]sulfamoyl]biphenyl-4-yl]methyl]-2-[2-(trifluoromethyl)phenyl]-3H-1,2,4-triazol-3-one;
(52) 5-n-butyl-4-[[2′-[N-(2,6-dichlorobenzoyl)sulfamoyl]biphenyl-4-yl]methyl]-2,4-dihydro-2-[2-(trifluoromethyl) phenyl]-3H-1,2,4-triazol-3-one;
(53) 5-n-butyl-4-[[2′-[N-(2-chloro-6-fluorobenzoyl)-sulfamoyl]biphenyl-4-yl]methyl]-2,4-dihydro-2-[2-(trifluoromethyl)phenyl]-3H-1,2,4-triazol-3-one;
(54) 5-n-butyl-4-[[2′-[N-(2-chloro-6-methylbenzoyl)-sulfamoyl]biphenyl-4-yl]methyl]-2,4-dihydro-2-[2-(trifluoromethyl)phenyl]-3H-1,2,4-triazol-3-one;
(55) 5-n-butyl-4-[[2′-[N-(5-chloro-2-furoyl)sulfamoyl]biphenyl-4-yl]methyl]-2,4-dihydro-2-[2-(trifluoromethyl) phenyl]-3H-1,2,4-triazol-3-one;
(56) 5-n-butyl-4-[[2′-[N-(4-chlorooxazole-5-carbonyl)sulfamoyl]biphenyl-4-yl]methyl]-2,4-dihydro-2-[2-(trifluoromethyl)phenyl]-3H-1,2,4-triazol-3-one;
(57) 5-n-butyl-2,4-dihydro-4-[[2′-[N-(1,3-dithiane-2-carbonyl)sulfamoyl]biphenyl-4-yl]methyl]-2-[2-(trifluoromethyl)phenyl]-3H-1,2,4-triazol-3-one;
(58) 4-[[2′-[N-(1-adamantanecarbonyl)sulfamoyl]biphenyl-4-yl]methyl]-5-n-butyl-2,4-dihydro-2-[2-(trifluoromethyl)phenyl]-3H-1,2,4-triazol-3-one;
(59) 5-n-butyl-4-[[2′-[N-(2-chlorobenzoyl)sulfamoyl]-biphenyl-4-yl]methyl]-2,4-dihydro-2-[4-nitro-2-(trifluoromethyl)phenyl}-3H-1,2,4-triazol-3-one;
(60) 2-[4-amino-2-(trifluoromethyl)phenyl]-4-[[2′-[N-(2-chlorobenzoyl)sulfamoyl]biphenyl-4-yl]-methyl]-5-n-butyl-2,4-dihydro-3H-1,2,4-triazol-3-one;
(61) 5-n-butyl-4-[[2′-[N-(2-chlorobenzoyl)sulfamoyl]-biphenyl-4-yl]methyl]-2,4-dihydro-2-[4-(propionylamino)-2-(trifluoromethyl)phenyl]-3H-1,2,4-triazol-3-one;
(62) 2-[4-(benzylamino)-2-(trifluoromethyl)phenyl]-5-n-butyl-4-[[2′-[N-(2-chlorobenzoyl)sulfamoyl]-biphenyl-4-yl]methyl]-2,4-dihydro-3H-1,2,4-triazol-3-one;
(63) 5-n-butyl-4-[[2′-[N-(2-chlorobenzoyl)sulfamoyl]-biphenyl-4-yl]methyl]-2-(2-chloro-5-nitrophenyl)-2,4-dihydro-3H-1,2,4-triazol-3-one;
(64) 2-(5-amino-2-chlorophenyl)-5-n-butyl-4-[[2′-[N-(2-chlorobenzoyl)sulfamoyl]biphenyl-4-yl]-methyl]-2,4-dihydro-3H-1,2,4-triazol-3-one;
(65) 5-n-butyl-4-[[2′-[N-(2-chlorobenzoyl)sulfamoyl]-biphenyl-4-yl]methyl]-2-[2-chloro-5-(propionylamino)phenyl]-2,4-dihydro-3H-1,2,4-triazol-3-one;
(66) 5-n-butyl-4-[[2′-[N-(2-chlorobenzoyl)sulfamoyl]-biphenyl-4-yl]methyl]-2-[2-chloro-5-(valerylamino)phenyl]-2,4-dihydro-3H-1,2,4-triazol-3-one;
(67) 5-n-butyl-4-[[2′-[N-(2-chlorobenzoyl)sulfamoyl]-biphenyl-4-yl]methyl]-2-[2-chloro-5-[(propoxycarbonyl)amino]phenyl]-2,4-dihydro-3H-1,2,4-triazol-3-one;
(68) 5-n-butyl-4-[[2′-[N-(2-chlorobenzoyl)sulfamoyl]-biphenyl-4-yl]methyl]-2-[2-chloro-5-($N^3$-propylureido)phenyl]-2,4-dihydro-3H-1,2,4-triazol-3-one;
(69) 5-n-butyl-2-[5-(N-n-butylcarbamoyl)-2-chlorophenyl]-4-[[2′-[N-(2-chlorobenzoyl]sulfamoyl]-biphenyl-4-yl]methyl]-2,4-dihydro-3H-1,2,4-triazol-3-one;
(70) 5-n-butyl-4-[[2′-[N-(2-chlorobenzoyl)sulfamoyl]-biphenyl-4-yl]methyl]-2,4-dihydro-2-[3-(propionylamino)phenyl]-3H-1,2,4-triazol-3-one;
(71) 5-n-butyl-4-[[2′-[N-(2-chlorobenzoyl)sulfamoyl]-biphenyl-4-yl]methyl]-2,4-dihydro-2-[3-(valerylamino)phenyl]-3H-1,2,4-triazol-3-one;
(72) 2-[5-(benzoylamino)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one;
(73) 5-n-butyl-4-[[2′-[N-(2-chlorobenzoyl)sulfamoyl]-biphenyl-4-yl]methyl]-2-[2-chloro-5-(phenylacetyl)amino]-2,4-dihydro-3H-1,2,4-triazol-3-one;
(74) 5-n-butyl-4-[[2′-[N-(2-chlorobenzoyl)sulfamoyl]-biphenyl-4-yl]methyl]-2-[2-chloro-5-(hydrocinnamoyl)amino]-2,4-dihydro-3H-1,2,4-triazol-3-one;
(75) 2-[5-(benzylamino)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one;
(76) 5-n-butyl-2-[5-(butyrylamino)-2-chlorophenyl]-4-[[2′-[N-(2-chlorobenzoyl)sulfamoyl]biphenyl-4-yl]methyl]-2,4-dihydro-3H-1,2,4-triazol-3-one;
(77) 5-n-butyl-4-[[2′-[N-(2-chlorobenzoyl)sulfamoyl]-biphenyl-4-yl]methyl]-2-[2-chloro-5-(isovalerylamino)phenyl]-2,4-dihydro-3H-1,2,4-triazol-3-one;
(78) 5-n-butyl-4-[[2′-[N-(2-chlorobenzoyl)sulfamoyl]-biphenyl-4-yl]methyl]-2,4-dihydro-2-[5-(propionylamino)-2-(trifluoromethyl)phenyl]-3H-1,2,4-triazol-3-one;
(79) 5-n-butyl-2-[5-(butyrylamino)-2-(trifluoromethyl)phenyl]-4-[[2′-[N-(2-chlorobenzoyl)sulfamoyl]biphenyl-4-yl]methyl]-2,4-dihydro-3H-1,2,4-triazol-3-one;
(80) 5-n-butyl-4-[[2′-[N-(2-chlorobenzoyl)sulfamoyl]-biphenyl-4-yl]methyl]-2,4-dihydro-2-[2-(trifluoromethyl)-5-(valerylamino)phenyl]-3H-1,2,4-triazol-3-one;
(81) 5-n-butyl-4-[[2′-[N-(2-chlorobenzoyl)sulfamoyl]-biphenyl-4-yl]methyl]-2-[2-chloro-5-(N-methylpropionylamino)phenyl]-2,4-dihydro-3H-1,2,4-triazol-3-one;
(82) 5-n-butyl-4-[[2′-[N-(2-chlorobenzoyl)sulfamoyl]-biphenyl-4-yl]methyl]-2-[2-chloro-5-(N-methylbutyrylamino)phenyl]-2,4-dihydro-3H-1,2,4-triazol-3-one;
(83) 5-n-butyl-4-[[2′-[N-(2-chlorobenzoyl)sulfamoyl]-biphenyl-4-yl]methyl]-2,4-dihydro-2-[3-(N-methylpropionylamino)phenyl]-3H-1,2,4-triazol-3-one;
(84) 5-n-butyl-4-[[2′-[N-(2-chlorobenzoyl)sulfamoyl]-biphenyl-4-yl]methyl]-2,4-dihydro-2-[3-($N^3$-isopropyl-$N^3$-methylureido)phenyl]-3H-1,2,4-triazol-3-one;
(85) 5-n-butyl-2-[5-(N-n-butyl-N-methylcarbamoyl)-2-chlorophenyl]-4-[[2′-[N-(2-chlorobenzoyl)sulfamoyl]biphenyl-4-yl]methyl]-2,4-dihydro-3H-1,2,4-triazol-3-one;
(86) 2-[2-bromo-5-(butyrylamino)phenyl]-5-n-butyl-4-[[2′-[N-(2-chlorobenzoyl)sulfamoyl]biphenyl-4-yl]methyl]-2,4-dihydro-3H-1,2,4-triazol-3-one;
(87) 2-[5-(butyrylamino)-2-methylphenyl]-5-n-butyl-4-[[2′-[N-(2-chlorobenzoyl)sulfamoyl]biphenyl-4-yl]methyl]-2,4-dihydro-3H-1,2,4-triazol-3-one;
(88) 5-n-butyl-2-[2-chloro-5-(valerylamino)phenyl]-2,4-dihydro-4-[[2′-[N-(3-methyl-2-furoyl)sulfamoyl]biphenyl-4-yl]methyl]-3H-1,2,4-triazol-3-one;
(89) 5-n-butyl-4-[[2′-[N-(3-chloro-2-thiophenecarbonyl)sulfamoyl]biphenyl-4-yl]methyl]-2-[2-chloro-5-(valerylamino)phenyl]-2,4-dihydro-3H-1,2,4-triazol-3-one;
(90) 5-n-butyl-2-[2-chloro-5-(valerylamino)phenyl]-4-[[2′-[N-(3,4-dichloro-2-furoyl)sulfamoyl]biphenyl-4-yl]methyl]-2,4-dihydro-3H-1,2,4-triazol-3-one;
(91) 5-n-butyl-4-[[2′-[N-(3-chloro-2-furoyl)sulfamoyl]biphenyl-4-yl]methyl]-2-[2-chloro-5-(valerylamino)phenyl]-2,4-dihydro-3H-1,2,4-triazol-3-one;
(92) 5-n-butyl-2-[2-chloro-5-(valerylamino)phenyl]-2,4-dihydro-4-[[2′-[N-(3,3-dimethylbutyryl)sulfamoyl]biphenyl-4-yl]methyl]-3H-1,2,4-triazol-3-one;
(93) 5-n-butyl-2-[2-chloro-5-(valerylamino)phenyl]-4-[[2′-[N-(2,5-dichlorobenzoyl)-sulfamoyl]biphenyl-4-yl]methyl]-2,4-dihydro-3H-1,2,4-triazol-3-one;
(94) 5-n-butyl-2-[2-chloro-5-(valerylamino)phenyl]-2,4-dihydro-4-[[2′-[N-(4-methyl-5-oxazolecarbonyl)sulfamoyl]biphenyl-4-yl]methyl]-3H-1,2,4-triazol-3-one;
(95) 5-n-butyl-2-[2-chloro-5-(valerylamino)phenyl]-2,4-dihydro-4-[[2′-[N-(1,3-dithiane-2-carbonyl)-sulfamoyl]biphenyl-4-yl]methyl]-3H-1,2,4-triazol-3-one;
(96) 5-n-butyl-2-(5-butyryl-2-chlorophenyl)-4-[[2′-[N-(2-chlorobenzoyl)sulfamoyl]biphenyl-4-yl]methyl]-2,4-dihydro-3H-1,2,4-triazol-3-one;
(97) 5-n-butyl-4-[[2′-[N-(2-chlorobenzoyl)sulfamoyl]biphenyl-4-yl]methyl]-2-[2-chloro-3-(valerylamino)phenyl]-2,4-dihydro-3H-1,2,4-triazol-3-one;
(98) 5-n-butyl-4-[[2′-[N-(2-chlorobenzoyl)sulfamoyl]biphenyl-4-yl]methyl]-2-[2-chloro-3-(N-n-butylcarbamoyl)phenyl]-2,4-dihydro-3H-1,2,4-triazol-3-one;
(99) 5-n-butyl-4-[[2′-[N-(2-chlorobenzoyl)sulfamoyl]biphenyl-4-yl]methyl]-2,4-dihydro-2-[2-methyl-3-(valerylamino)phenyl]-3H-1,2,4-triazol-3-one;
(100) 5-n-butyl-2-[2-chloro-5-(valerylamino)phenyl]-4-[[2′-[N-(2-chlorobenzoyl)sulfamoyl]-5′-ethylbiphenyl-yl]methyl]-2,4-dihydro-3H-1,2,4-triazol-3-one;
(101) 5-n-butyl-2-[2-chloro-5-(propionylamino)phenyl]-4-[[2′-[N-(2-chlorobenzoyl)sulfamoyl]-5′-n-propylbiphenyl-4-yl]methyl]-2,4-dihydro-3H-1,2,4-triazol-3-one;
(102) 5-n-butyl-4-[[2′-[N-(2-chlorobenzoyl)sulfamoyl]-5′-ethylbiphenyl-4-yl]methyl]-2-[2-chloro-3-(N-n-butylcarbamoyl)phenyl]-2,4-dihydro-3H-1,2,4-triazol-3-one;
(103) 2-[5-(N-benzylcarbamoyl)-2-chlorophenyl]-5-n-but yl-4-[[2′-[N-(2-chlorobenzoyl)sulfamoyl]-biphenyl-4-yl]methyl]-2,4-dihydro-3H-1,2,4-triazol-3-one;
(104) 2-[5-(N-benzyl-N-methylcarbamoyl)-2-chlorophenyl]-5-n-butyl-4-[[2′-[N-(2-chlorobenzoyl)-sulfamoyl]biphenyl-4-yl]methyl]-2,4-dihydro-3H-1,2,4-triazol-3-one.

Abbreviations used in the schemes and examples are listed in Table 1.

TABLE 1

| | |
|---|---|
| Reagents | |
| NaOEt | sodium ethoxide |
| $Et_3N$ | triethylamine |
| t-BuLi | *tert*-butyllithium |
| NBS | N-bromosuccinimide |
| TrCl | trityl chloride (triphenylmethylchoride) |
| AIBN | 2,2'-azobis(isobutyronitrile) |
| DBU | 1,8-diazabicyclo[5.4.0]undec-7-ene |

| | |
|---|---|
| DMAP | 4-(dimethylamino)pyridine |
| CDI | 1,1'-carbonyldiimidazole |
| DCC | N,N[1]'-dicyclohexylcarbodiimide |
| DIAD | diisopropyl azodicarboxylate |

Solvents

| | |
|---|---|
| EtOH | ethanol |
| DMF | dimethylformamide |
| AcOH | acetic acid |
| DMSO | dimethylsulfoxide |
| THF | tetrahydrofuran |
| EtOAc | ethyl acetate |

Others

| | |
|---|---|
| Ar (or Ar') | aryl |
| R | any group, in context, compatible with the definition of Formula I |
| X | a leaving group such as bromo, chloro, iodo, or ρ-toluenesulfonate |
| Et | ethyl |
| Me | methyl |
| iPr | isopropyl |
| t-Bu(or Bu-t) | tert-butyl |
| Bu | n-butyl |
| Im | imidazol-1-yl |
| Tr | trityl (triphenylmethyl) |
| FAB | fast atom bombardment |
| EI | electron impact |
| MS | mass spectrum |

DISCUSSION OF CHEMISTRY AND REACTION SCHEMES

The compounds of Formula I can be prepared by a variety of methods typified by those described below. General synthetic methods for 2,4,5-trisubstituted-2,4-dihydro-3H-1,2,4-triazol-3-ones and -triazole-3-thiones are discussed in books or review articles such as:

(1) C. Temple and J.A. Montgomery, "Triazoles: 1,2,4" (Vol. 37 of The Chemistry of Heterocyclic Compounds, A. Weissberger and E.C. Taylor, eds), Wiley-Interscience, New York, 1981, pp. 365-442.

(2) J.B. Polya, Comprehensive Heterocyclic Chemistry. The Structure, Reactions, Synthesis and Uses of Heterocyclic Compounds, A.R. Katritzky and C.W. Rees, eds., Vol. 5, Pergamon Press, Oxford, 1984, pp. 733-790.

(3) J.H Boyer, Heterocyclic Compounds, R.C. Elderfield, ed., Vol. 7, John Wiley & Sons, New York, 1961, pp. 384-461.

In general, the compounds of Formula I are constructed in such a way that $N^1$ and $N^2$ of the triazole ring are derived from hydrazine or a hydrazine derivative, while $N^4$ of the triazole and the 4-(arylmethyl) substituent are derived directly or indirectly from a suitably substituted benzylamine (or isocyanate) or from a benzyl halide (or methanesulfonate, p-toluenesulfonate, etc.).

Although the Reaction Schemes described below are reasonably general, it will be understood by those skilled in the art of organic synthesis that one or more functional groups present in a given compound of Formula I may render the molecule incompatible with a particular synthetic sequence. In such a case an alternative route, an altered order of steps, or a strategy of protection and deprotection may be employed. In all cases the particular reaction conditions (including reagents, solvent, temperature, and time) should be chosen so that

they are consistent with the nature of the functionality present in the molecule.

The Reaction Schemes below have been generalized for simplicity. It is to be understood that the "$ArCH_2$" substituent present at $N^4$ of the triazolinone derivatives or in their precursors is any substituted arylmethyl moiety consistent with the definition of the $N^4$ substituent in Formula I or which may be transformed to such a grouping either before or after the assembly of the triazolinone ring system Such transformations may involve protection and/or deprotection or other modifications.

It is further to be understood that in the generalized schemes below, the Ar' and R groups represent moieties consistent with the definition of Formula I or which may be transformed to such groupings before or after assembly of the heterocycle.

## REACTION SCHEME 1

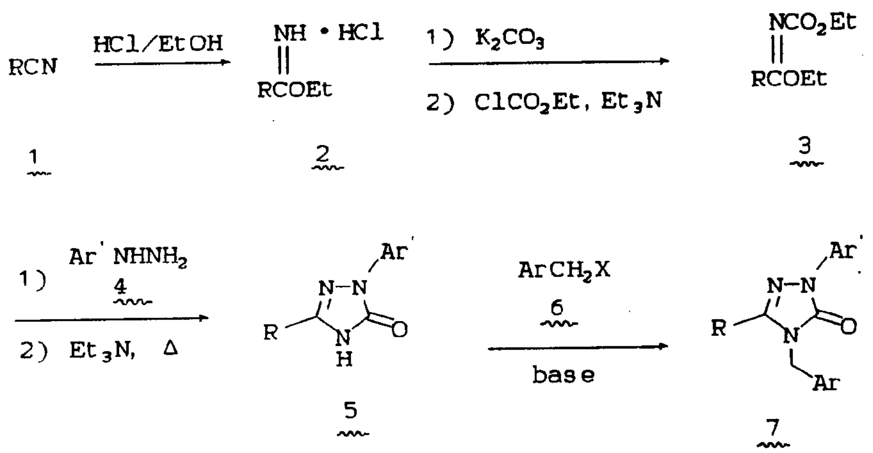


Reaction Scheme 1 presents a route patterned after that reported by K. Yabutani, K. Taninaka, M. Kajioka, K. Takagi, H. Matsui, K. Sutoh, and M. Yamamoto, European Patent Application 220,952 (1987). The N-carbethoxy imidate 3 (obtained from the alkanenitrile 1 via the imidate 2, which is reacted with ethyl chloroformate) is treated with arylhydrazine 4, typically at about 40-50°C. Without isolation of the intermediate, further heating at elevated temperature (usually in the range of 90-150°C.) in the presence of a tertiary amine such as triethylamine effects cyclization to the triazolinone 5. In the presence of a suitable base (e.g., sodium hydride, sodium alkoxide, sodium hydroxide) treatment of 5 with the appropriate $ArCH_2X$, where X = bromo, iodo, chloro, methanesulfonate, p-toluenesulfonate, and the like, yields the $N^4$-alkylated product 7. A variant of the method using a thioimidate has been described by M. Kajioka, H. Kurono, K. Okawa, and M. Harada, U.S. Patent No. 4,318,731 (1982).

## REACTION SCHEME 2

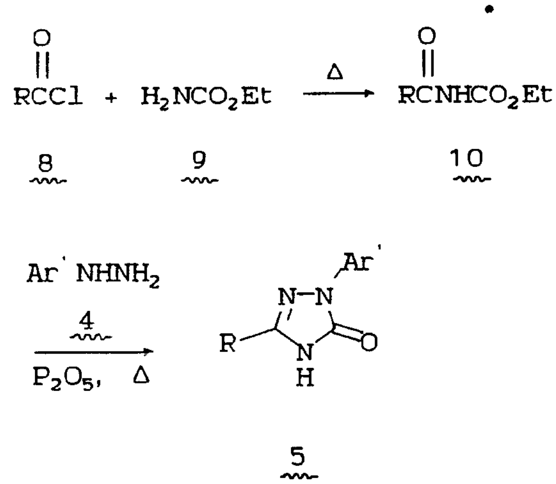


8 9 10

5

An alternative route to the $N^2$-substituted triazolinone intermediate 7 is shown in Reaction Scheme 2. This chemistry has been described by T.N. Ghosh and M.V. Betrabet, J. Indian Chem. Soc., 7, 899 (1930), S. Bellioni, Ann Chim. (Rome), 52, 187 (1962), G. Palazzo and G. Picconi, Boll. Chim. Farm., 105, 217 (1966), and British Patent 1,021,070 (1966). An acid chloride 8 is heated with urethane (9) (typically at 80-100°C.), to give the acylurethane 10. Reaction of 10 with an arylhydrazine 4 and phosphorus pentoxide (usually in toluene or xylene at reflux) gives 5, which can then be further alkylated on $N^4$ as in Reaction Scheme 1. A (thioacyl)urethane modification of this pathway has been reported by D.L. Temple, Jr., and W.G. Lobeck, Jr., U.S. Patent 4,487,773 (1984).

## REACTION SCHEME 3

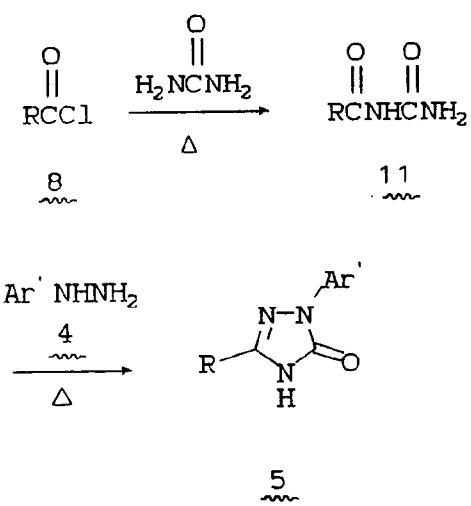


8 11

5

A variation of Reaction Scheme 2, shown in Reaction Scheme 3, has been described by P. Gold-Aubert, D. Melkonian, and L. Toribio, Helv. Chim. Acta, 47, 1188 (1964) and A.L. Langis, U.S. Patent 3,499,000 (1970). The readily prepared acylurea 11 upon heating with an arylhydrazine 4 (at about 150-200°C.) is converted to the triazolinone intermediate 5.

## REACTION SCHEME 4

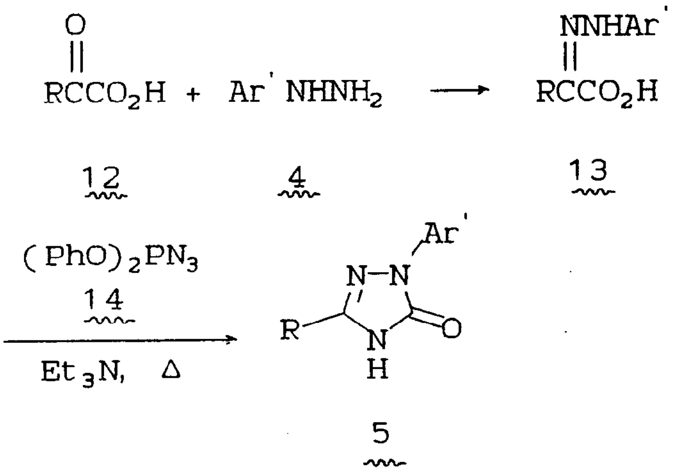


12 4 13

5

In a quite different approach (Reaction Scheme 4), L. Maravetz, U.S. Patent 4,705,557 (1987) and G Theodoridis, International Patent Application WO87/03782 (1987) disclose condensing an α-keto acid 12 with the arylhydrazine 4 to give derivatives such as 13, which can be converted to the triazolinone intermediate 5 by heating with diphenylphosphoryl azide and triethylamine (typically at 75-115°C.). In the last step, an intermediate acyl azide loses nitrogen and undergoes the Curtius rearrangement to an isocyanate, which undergoes ring closure. As shown in Reaction Scheme 1, 5 can then be alkylated on $N^4$ to give the trisubstituted triazolinone 7.

## REACTION SCHEME 5

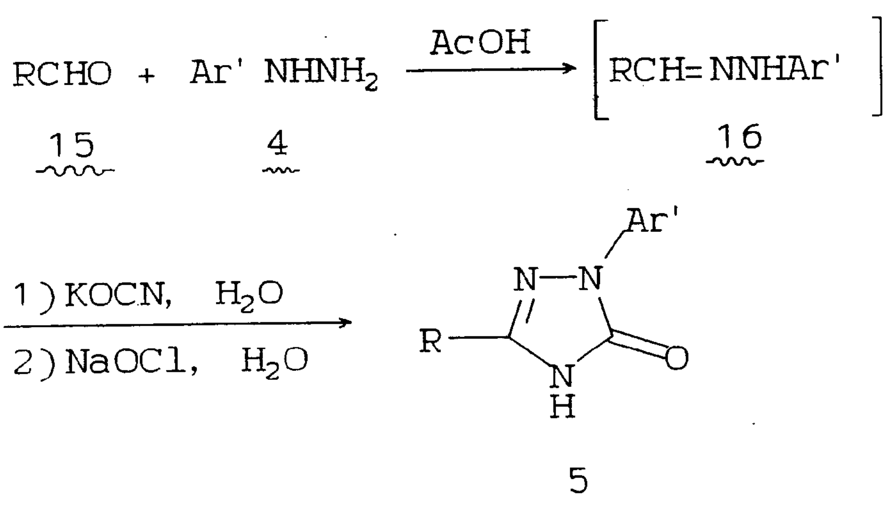


15 4 16

5

Still another route to $N^2$-aryl triazolinone intermediates of structure 5 is illustrated in Reaction Scheme 5. In this method [G. Theodoridis and J.W. Lyga, International Patent Application W091/03470 (1991)], an aldehyde 15 is reacted with the arylhydrazine 4 in glacial acetic acid. The intermediate hydrazone 16 may either be isolated or treated directly in the same pot with aqueous potassium cyanate. The aryl triazolidinone thus formed is again frequently not isolated but rather treated directly with an aqueous sodium hypochlorite solution, resulting in oxidation to the triazolinone 5.

## REACTION SCHEME 6

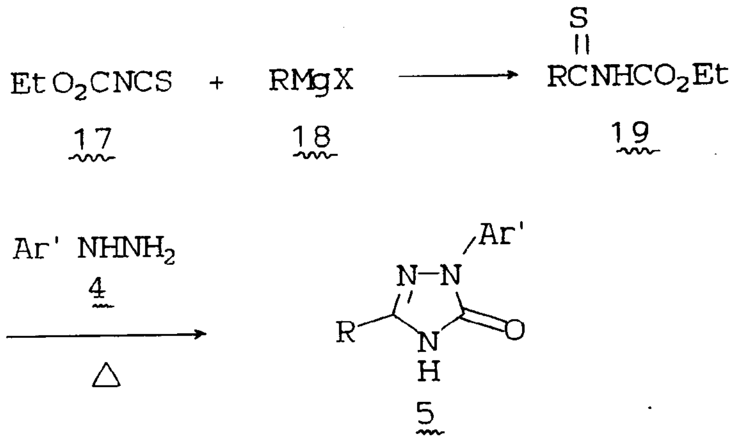


17 18 19

5

The triazolinone intermediate 5 can also be made according to reaction Scheme 6 [B. George and E.P. Papadopoulos, J. Org. Chem., 41, 3233 (1976); R.E. Manning, D.B. Reitz, and H.-C. Huang, PCT Patent Appl. WO 91/18888 (1991)]. Ethoxycarbonyl isothiocyanate (17) is reacted with an alkylmagnesium halide 18 (typically in ether at about -50°C to room temperature). The resulting N-(ethoxycarbonyl) thioamide 19 is heated with arylhydrazine 4 (for example, in ethanol or 2-methoxyethanol at reflux) to yield the triazolinone 5.

## REACTION SCHEME 7

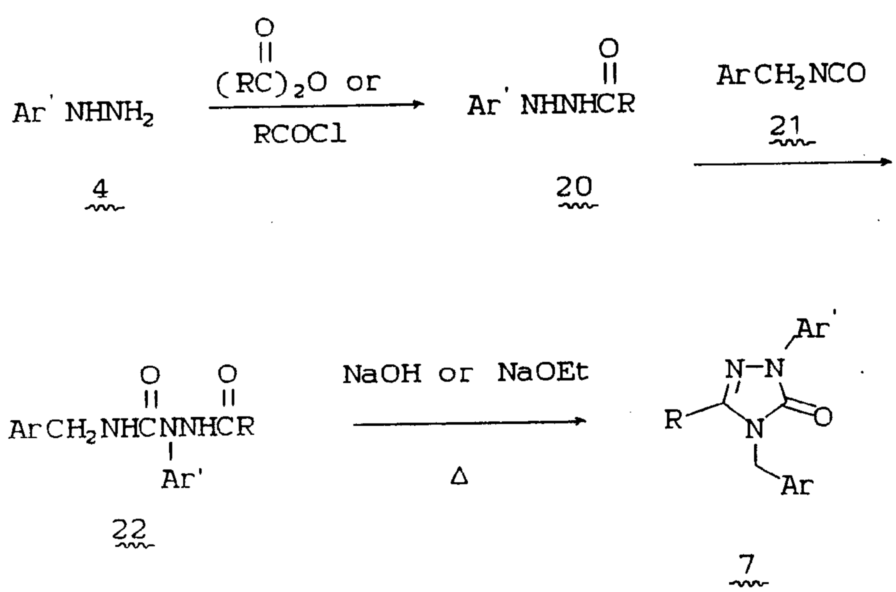


4 20 22 7

In Reaction Scheme 7, acylation of arylhydrazine 4 gives 20, which can be reacted with the isocyanate 21 to yield the 1-acyl-2,4-disubstituted-semicarbazide 22. Cyclization of 22 upon heating with hydroxide or alkoxide affords the triazolinone 7. This chemistry has been described by H. Gehlen and W. Schade, Liebigs Ann. Chem., 675, 180 (1964).

## REACTION SCHEME 8

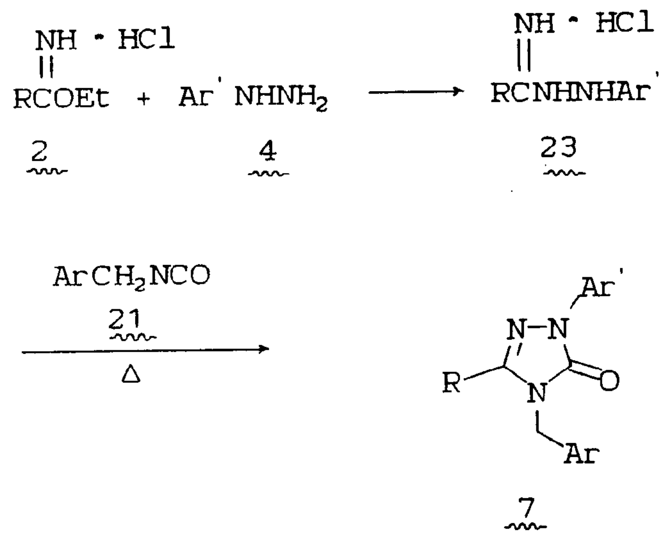


2 4 23

7

In another approach (Reaction Scheme 8), imidate 2 is treated with arylhydrazine 4 to give the amidrazone 23. Heating 23 with the isocyanate 21 gives the triazolinone 7. Syntheses of this type have been reported by T. Bany, Rocz. Chem., 42, 247 (1968).

## REACTION SCHEME 9

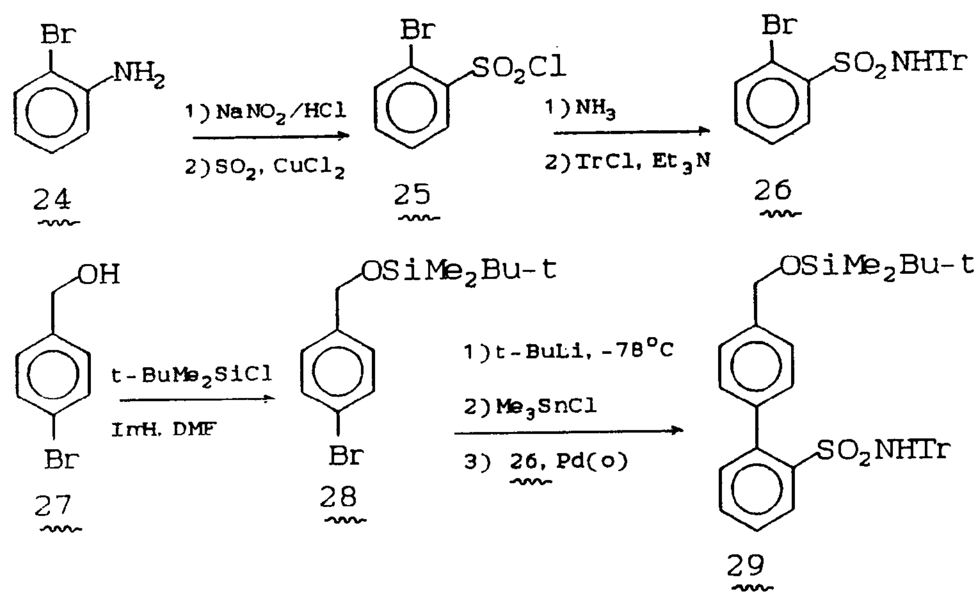


24 25 26

27 28 29

REACTION SCHEME 9 (CONT'D)

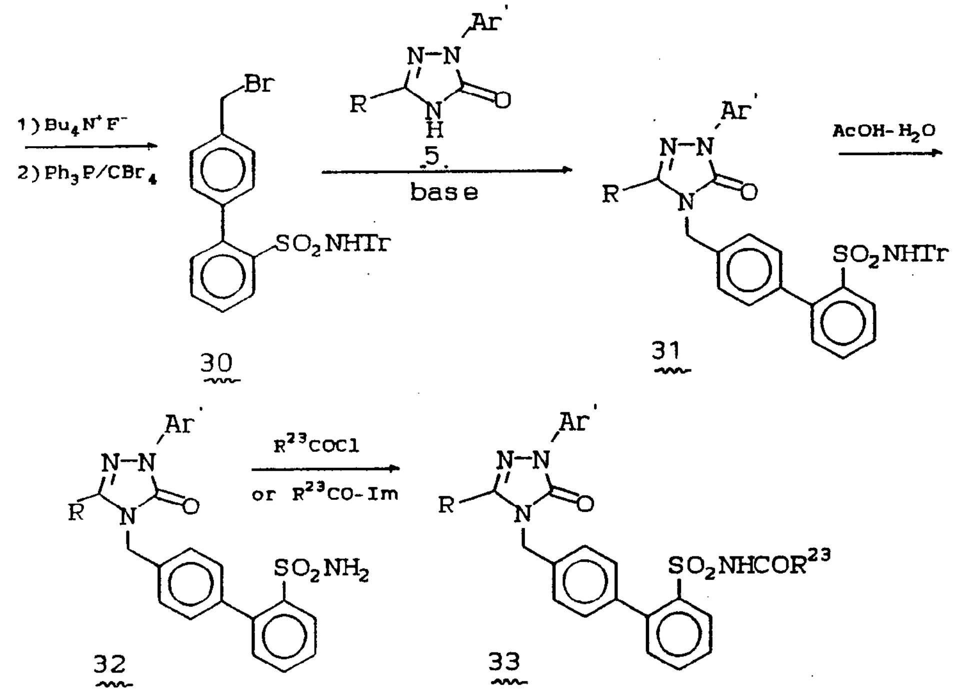


30 31 32 33

wherein:
Tr = trityl (i.e. triphenylmethyl)
Im = 1-imidazolyl.

Reaction Scheme 9 outlines the synthesis of the (2'-sulfamoylbiphenyl-4-yl)methyl side chain and elaboration to the triazolinone acylsulfonamide 33. Diazotization of o-bromoaniline (24) and reaction of the diazonium salt with sulfur dioxide in the presence of cupric chloride affords the corresponding sulfonyl chloride 25 [see H. Meerwein, et al., Chem. Ber., 90, 841 (1957); A.J. Prinsen and H. Cerfontain, Rec. Trav. Chim., 84, 24 (1965); E. E. Gilbert, Synthesis, 3 (1969); and references cited therein]. Treatment of 25 with ammonia and then with trityl chloride in the presence of triethylamine yields the N-trityl sulfonamide 26. p-Bromobenzyl alcohol (27) is t-butyl-dimethylsilylated, and the resulting 28 is coupled with 26 under the conditions described above to give the biphenyl product 29. The silyl group is removed with tetrabutylammonium fluoride, and treatment of the alcohol with triphenylphosphine/carbon tetrabromide gives the bromo derivative 30. The alykylation of the triazolinone intermediate 5 with 30 is carried out as in Reaction Scheme 1 to give 31. The trityl protecting group is removed with aqueous acetic acid to give the free sulfonamide 32 which is acylated to yield the target 33. The acylating agent may be an acid chloride or an alternative reactive acyl derivative, particulary an acylimidazole derivative (which may be prepared from the carboxylic acid by warming with 1,1'-carbonyldiimidazole in a solvent such as THF). The acylation reaction is generally conducted in the presence of a base such as pyridine (may be used as solvent, especially for the acid chloride) or DBU (in a solvent such as THF). Alternatively, the sulfonamide 32 may be deprotonated with a strong base such as sodium hydride prior to the acylation.

REACTION SCHEME 10

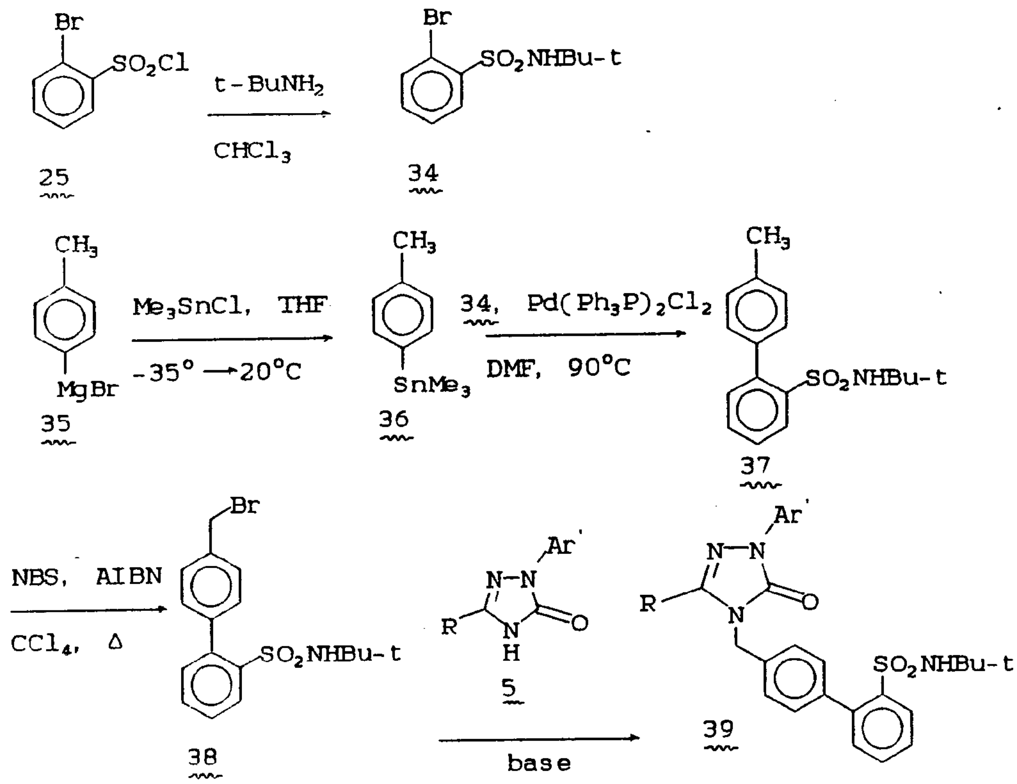


25 34 35 36 37 38 5 39

REACTION SCHEME 10 (CONT'D)

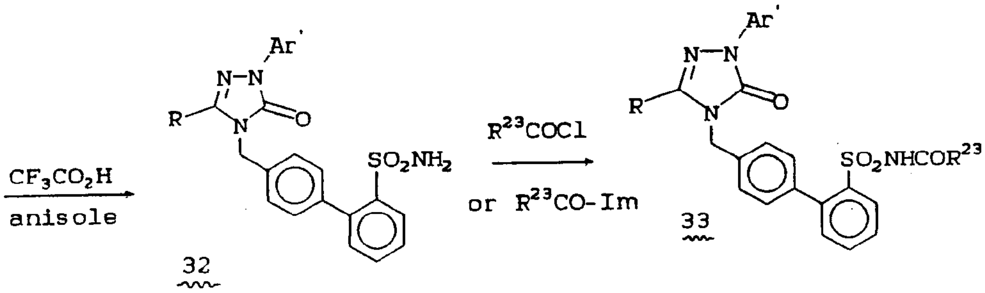


32 33

A preferred route to target compounds of structure 33 is illustrated in Reaction Scheme 10. In a variation of Scheme 9, 2-bromobenzenesulfonyl chloride (25) is reacted with an excess of t-butylamine to yield the t-butylsulfonamide 34. The trimethylstannyl derivative 36 may be conveniently prepared from p-tolylmagnesium bromide (35) by treatment with trimethyltin chloride at -35°C to room temperature. Cross-coupling of 36 with 34 catalyzed by bis(triphenylphosphine)palladium(II) chloride in DMF at about 90°C affords the biphenyl derivative 37. Such couplings have been described by J.K. Stille, Pure Appl. Chem., 57, 1771(1985); T.R. Bailey, Tetrahedron Lett., 27, 4407(1986); and D.A. Widdowson and Y.-Z. Zhang, Tetrahedron, 42, 2111(1986). The bromomethyl species 38 is obtained by heating 37 with N-bromosuccinimide (NBS) in carbon tetrachloride in the presence of an initiator such as 2,2'-azobis(isobutyronitrile) (AIBN). The triazolinone 39 is obtained by alkylation of 5 with 38 according to Reaction Scheme 1. The free sulfonamide 32 is obtained by deprotection of 39 with trifluoroacetic acid in the presence of anisole. Conversion of 32 to 33 is accomplished as in Scheme 9.

Various fuctional group transformations may be carried out on the "Ar'" moiety of the triazolinones in order to elaborate the desired $V^2$ substituent In many cases, such transformations may be accomplished either at the stage of the acylsulfonamide 33 or the t-butylsulfonamide intermediate 39. Whether there is a particular preference for carrying out the transformations at the stage of 39 vs. 33 depends on the specific reaction conditions and will be apparent to one skilled in the art. In general, the $V^1$ substituent and a precursor of the $V^2$ substituent (for example, $NO_2$ or $CO_2$-alkyl), when $V^2$ is not H, will be present on the arylhydrazine when the triazolinone is assembled as depicted in the previous reaction schemes.

## REACTION SCHEME 11

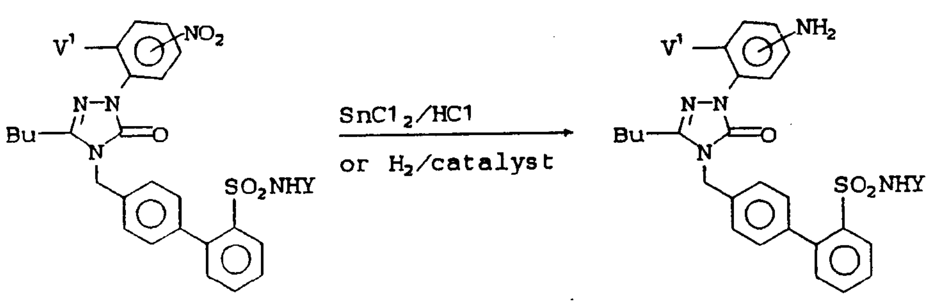


40 41

41

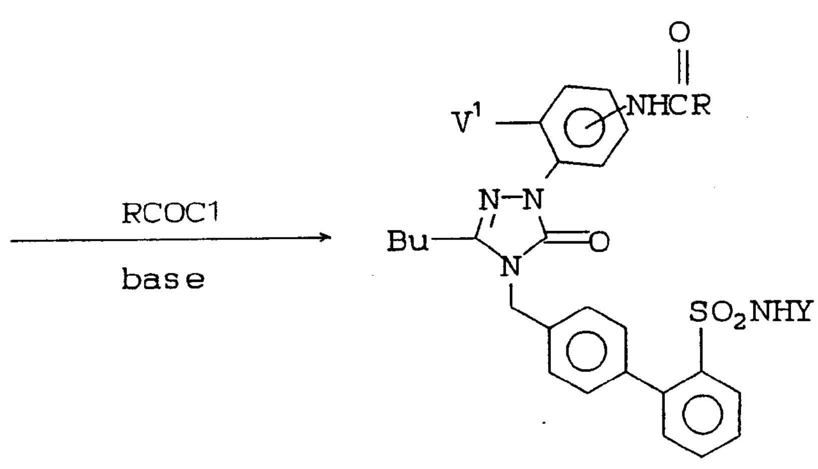


42

41
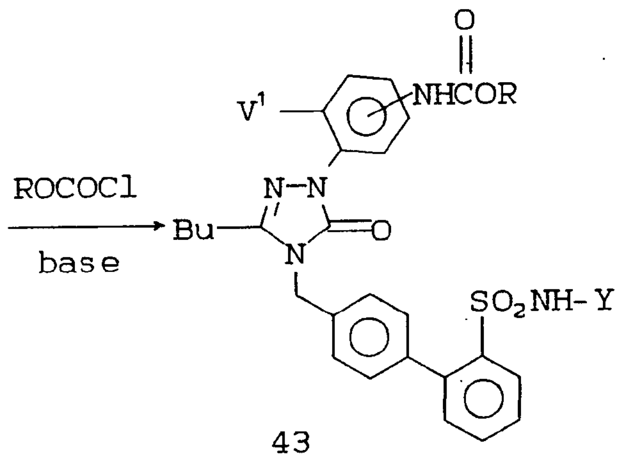


43

41
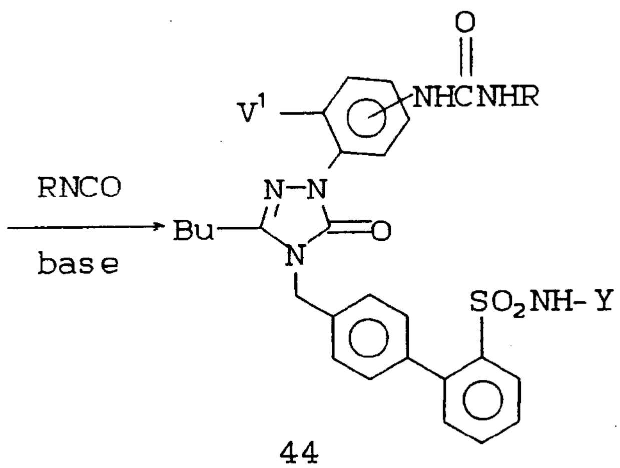


44

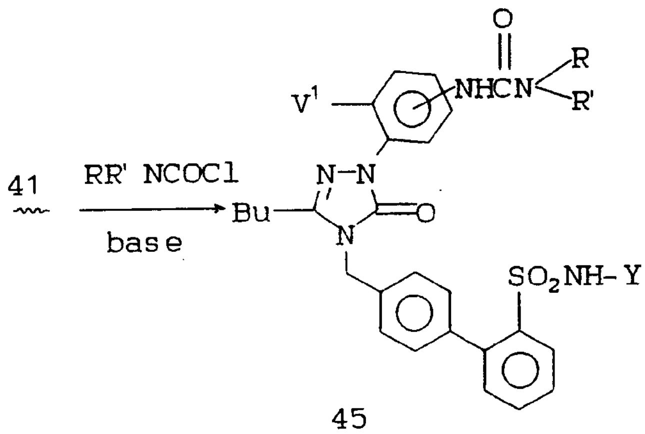


45

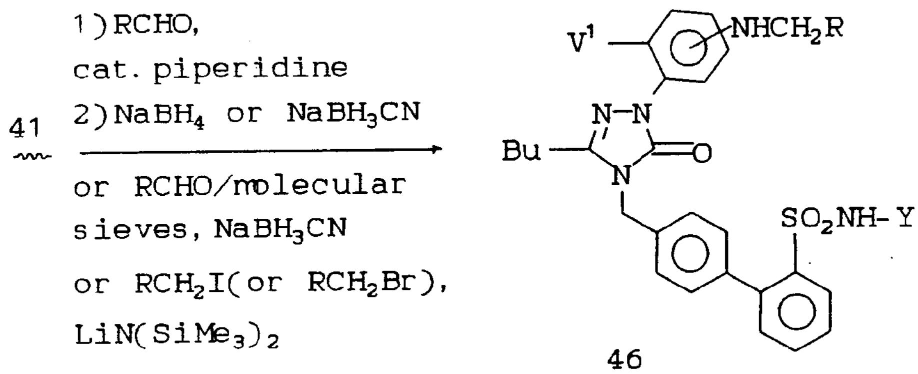


46

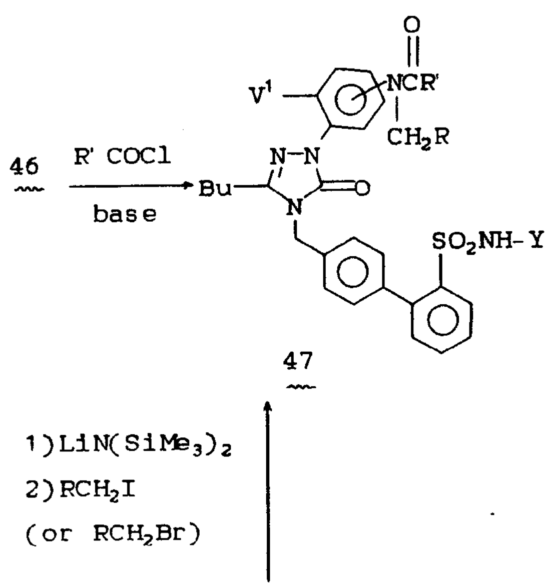


42 (R' in place of R)

wherein

Y is t-Bu or

$$-\overset{O}{C}R^{23}.$$

As shown in Reaction Scheme 11, intermediate 40, which contains a nitro substituent and is prepared according to the previous schemes, is reduced, as appropriate, with stannous chloride in the presence of concentrated hydrochloric acid or by catalytic hydrogenation to give the amino derivative 41. In the presence of a base such as sodium hydride, 41 can be reacted with an acid chloride to give the amide 42, with a chloroformate to give the carbamate 43, with an isocyanate to give the urea 44, or with a carbamoyl chloride to give a trisubstituted urea 45. Also, 41 can be converted to a substituted-amino derivative 46. For R = aryl, this may be accomplished conveniently by first heating 41 with the aldehyde in the presence of a catalytic amount of piperidine in a solvent such as isopropanol. The intermediate Schiff base is then reduced (optionally without isolation) by use of sodium borohydride in ethanol or sodium cyanoborohydride in methanol to provide 46. For R = alkyl or aralkyl, the transformation may be accomplished by reacting 41 with the aldehyde in the presence of molecular sieves and sodium cyanoborohydride (present initially or added later), preferably at about 10-40°C. Alternatively, for R = aryl, alkyl, hydrogen, etc., 41 may be deprotonated with a strong base such as lithium bis(trimethylsilyl)amide in a solvent such as DMF and then reacted with the appropriate alkyl or aralkyl iodide or bromide to afford 46. Subsequently, 46 can be acylated as described above to yield the tertiary amide 47, which may also be prepared by alkylation of 42 in the presence of a strong base such as lithium bis(trimethylsilyl)amide in a solvent such as DMF.

## REACTION SCHEME 12

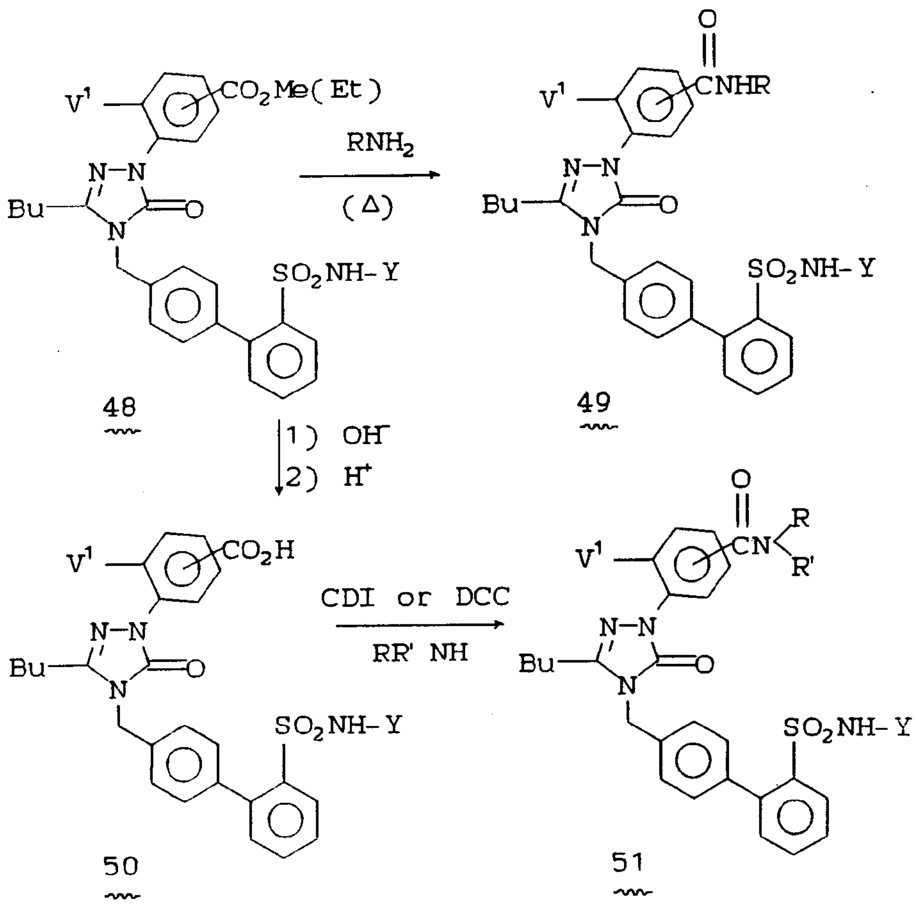


48

49

50

51

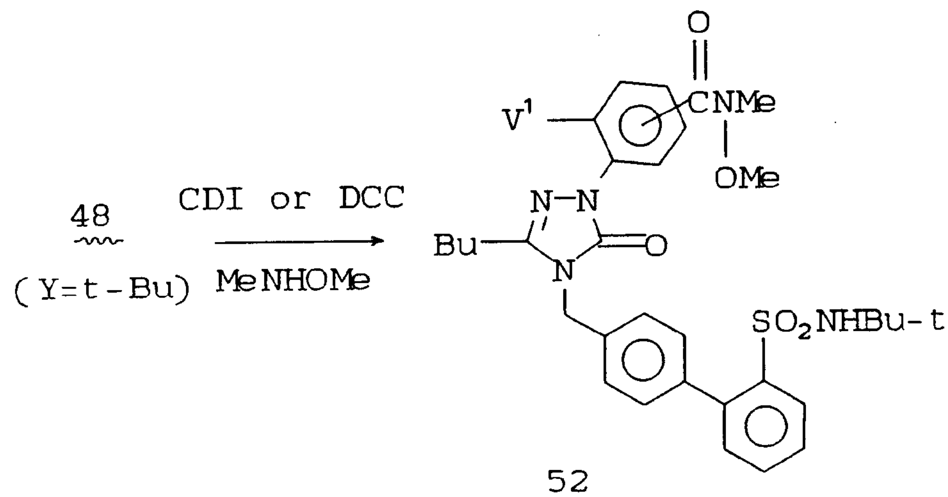


52

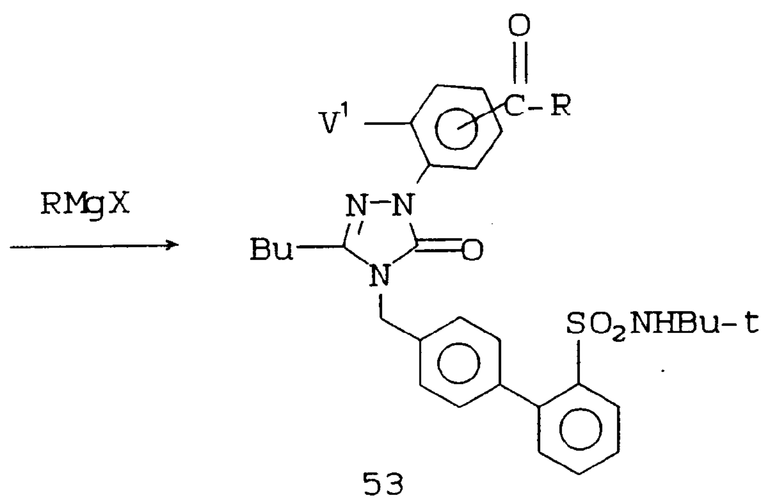


53

wherein
Y is t-Bu or

$$-\overset{O}{\overset{\|}{C}}R^{23}.$$

In Reaction Scheme 12, intermediate 48, which bears a methyl or ethyl ester substituent and is synthesized according to the previous reaction schemes, is treated with a neat amine, generally at about 20-100°C, to provide the amide 49. This method is particularly useful for primary amines. Compound 48 may also be saponified under standard conditions to the carboxylic acid 50. With use of a condensing agent such as 1,1'-carbonyldiimidazole or N,N'-dicyclohexylcarbodiimide, 50 can be reacted with an amine to give the amide product 51. This method is suitable for secondary as well as primary amines. Similarly, 48 (at the t-butylsulfonamide intermediate stage) is converted to the N-methoxy-N-methylamide 52. Following the Weinberg method [S. Nahm and S. M. Weinreb, Tetrahedron Lett., 22, 3815 (1981)], 52 can be reacted with a Grignard reagent to afford the ketone product 53.

By use of these methods and others analogous to those shown in Reaction Schemes 11 and 12, all of the $V^2$ substituents can be elaborated at the stage corresponding to the t-butylsulfonamide intermediate 39 or the acylsulfonamide 33. When the transformations are carried out at the t-butylsulfonamide stage, final conversion to the desired acylsulfonamide is accomplished as in Reaction Scheme 10.

## REACTION SCHEME 13

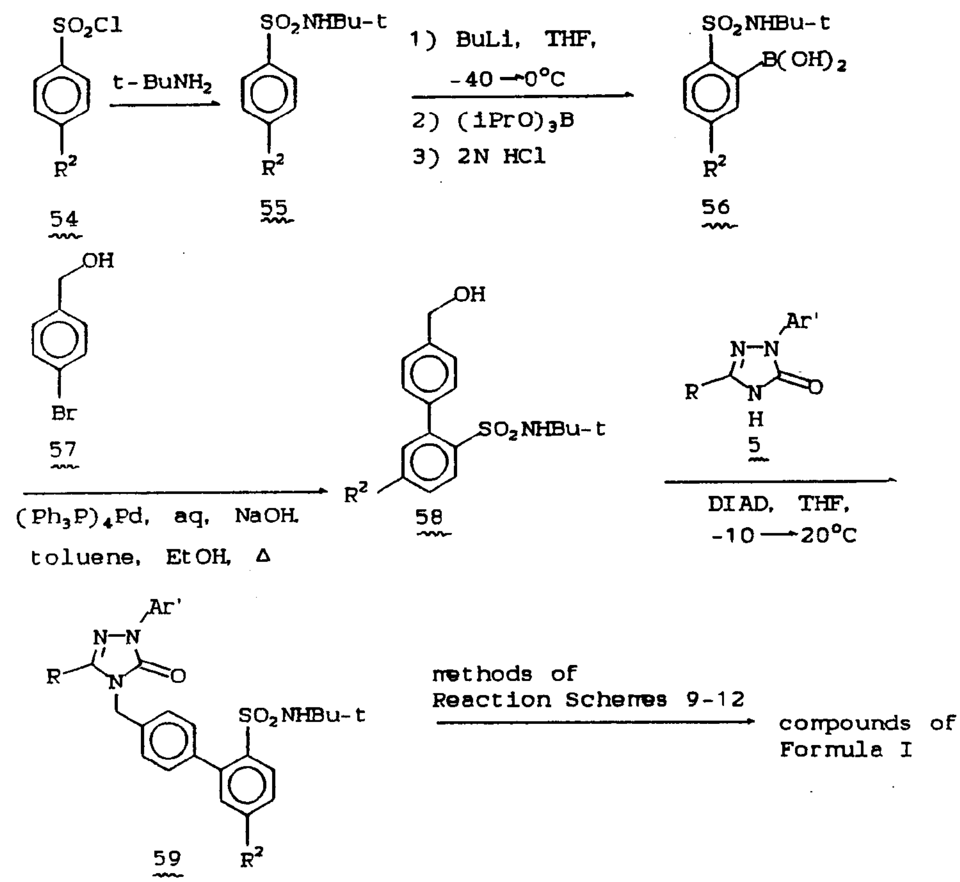


The route shown in Reaction Scheme 13 is particularly useful for preparing compounds of Formula I in which the distal ring of the biphenylmethyl side chain bears a substituent (for example, alkyl) at the 5′-position in addition to the acylsulfonamide moiety at the 2′-position. A 4-substituted benzenesulfonyl chloride 54 is converted to the N-t-butylsulfonamide 55 as in Reaction Scheme 10. Based on a literature method [M.J. Sharp, W. Cheng, and V. Snieckus, Tetrahedron Lett., 28, 5093 (1987)], metalation ortho to the sulfonamide is achieved with n-butyllithium in THF at -40° to 0°C. Then treatment with triisopropyl borate followed by acidic work-up affords the boronic acid 56. This undergoes cross-coupling with 4-bromobenzyl alcohol (57) in the presence of tetrakis(triphenylphosphine)palladium(0) according to literature methods [M.J. Sharp, et al., op. cit.; N. Miyaura, T. Yanagi, and A. Suzuki, Synth. Commun., 11, 513 (1981)] to give the biphenylmethyl alcohol 58. A triazolinone base 5, Prepared as in the previous schemes, can be directly alkylated with 58 in the presence of diiospropyl azodicarboxylate (DIAD) in THF at -10°C to room temperature, following the methods of Mitsunobu [O. Mitsunobu, Synthesis, 1 (1981)]. The product 59 can be further converted to fully elaborated compounds of Formula I by the methods of Reaction Schemes 9-12.

The compounds of this invention form salts with various inorganic and organic acids and bases which are also within the scope of the invention. Such salts include ammonium salts, alkali metal salts like sodium and potassium salts, alkaline earth metal salts like the calcium and magnesium salts, salts with organic bases; e.g., dicyclohexylamine salts, N-methyl-D-glucamine salts, salts with amino acids like arginine, lysine, and the like. Also, salts with organic and inorganic acids may be prepared; e.g., HCl, HBr, $H_2SO_4$, $H_3PO_4$, methanesulfonic, toluenesulfonic, maleic, fumaric, camphorsulfonic. The non-toxic, physiologically, acceptable salts are preferred, although other salts are also useful; e.g., in isolating or purifying the product.

The salts can be formed by conventional means such as by reacting the free acid or free base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Angiotensin II (A II) is a powerful arterial vasoconstrictor, and it exerts its action by interacting with specific

receptors present on cell membranes. The compounds described in the present invention act as competitive antagonists of A II at the receptors. In order to identify A II antagonists and determine their efficacy in vitro, the following three ligand-receptor binding assays were established.

Receptor binding assay using rabbit aortae membrane preparation:

Three frozen rabbit aortae (obtained from Pel-Freeze Biologicals) were suspended in 5mM Tris-0.25M Sucrose, pH 7.4 buffer (50 ml) homogenized, and then centrifuged. The mixture was filtered through a cheesecloth and the supernatant was centrifuged for 30 minutes at 20,000 rpm at 4°C. The pellet thus obtained was resuspended in 30 ml of 50mM Tris-5 mM $MgCl_2$ buffer containing 0.2% Bovine Serum Albumin and 0.2 mg/ml Bacitracin and the suspension was used for 100 assay tubes. Samples tested for screening were done in duplicate. To the membrane preparation (0.25 ml) there was added $^{125}$I-Sar$^1$Ile$^8$-angiotensin II [obtained from New England Nuclear] (10μl; 20,000 cpm) with or without the test sample and the mixture was incubated at 37°C for 90 minutes. The mixture was then diluted with ice-cold 50mM Tris-0.9% NaCl, pH 74 (4ml) and filtered through a glass fiber filter (GF/B Whatman 2.4″ diameter). The filter was soaked in scintillation cocktail (10 ml) and counted for radioactivity using Packard 2660 Tricarb liquid scintillation counter. The inhibitory concentration ($IC_{50}$) of potential A II antagonist which gives 50% displacement of the total specifically bound $^{125}$I-Sar$^1$Ile$^8$-angiotensin II was presented as a measure of the efficacy of such compounds as A II antagonists.

Receptor assay using Bovine adrenal cortex preparation

Bovine adrenal cortex was selected as the source of A II receptor. Weighed tissue (0.1 g is needed for 100 assay tubes) was suspended in Tris.HCl (50mM), pH 7.7 buffer and homogenized. The homogenate was centrifuged at 20,000 rpm for 15 minutes. Supernatant was discarded and pellets resuspended in buffer [$Na_2HPO_4$ (10mM)-NaCl (120mM)-disodium EDTA (5mM) containing phenylmethane sulfonyl fluoride (PMSF)(0.1mM)]. (For screening of compounds, generally duplicates of tubes are used). To the membrane preparation (0.5 ml) there was added $^3$H-angiotensin II (50mM) (10μl) with or without the test sample and the mixture was incubated at 37°C for 1 hour. The mixture was then diluted with Tris buffer (4ml) and filtered through a glass fiber filter (GF/B Whatman 2.4″ diameter). The filter was soaked in scintillation cocktail (10ml) and counted for radioactivity using Packard 2660 Tricarb liquid scintillation counter. The inhibitory concentration ($IC_{50}$) of potential A II antagonist which gives 50% displacement of the total specifically bound $^3$H-angiotensin II was presented as a measure of the efficacy of such compounds as A II antagonists.

Receptor assay using rat brain membrane preparation

Membranes from rat brain (thalamus, hypothamus and midbrain) were prepared by homogenization in 50 mM Tris HCl (pH 7.4), and centrifuged at 50,000 x g. The resulting pellets were washed twice in 100 mM NaCl, 5 mM $Na_2$·EDTA, 10 mM $Na_2HPO_4$ (pH 7.4) and 0.1 mM PMSF by resuspension and centrifugation. For binding assays, the pellets were resuspended in 160 volumes of binding assay buffer (100 mM NaCl, 10 mM $Na_2HPO_4$, 5 mM $Na_2$·EDTA, pH 7.4, 0.1 mM PMSF, 0.2 mg/ml soybean trypsin inhibitor, 0.018 mg/ml o-phenanthroline, 77 mg/ml dithiothreitol and 014 mg/ml bacitracin. For $^{125}$I.Ile$^8$-angiotensin II binding assays, 10 μl of solvent (for total binding), Sar$^1$,Ile$^8$-angiotensin II (1 μM) (for nonspecific binding) or test compounds (for displacement) and 10 μl of [$^{125}$I]Sar$^1$,Ile$^8$-angiotensin II (23-46 pM) were added to duplicate tubes. The receptor membrane preparation (500 μl) was added to each tube to initiate the binding reaction. The reaction mixtures were incubated at 37°C for 90 minutes. The reaction was then terminated by filtration under reduced pressure through glass-fiber GF/B filters and washed immediately 4 times with 4 ml of 5 mM ice-cold Tris HCl (pH 7.6) containing 0.15 M NaCl. The radioactivity trapped on the filters was counted using a gamma counter.

Using the methodology described above, representative compounds of the invention were evaluated and all were found to exhibit an activity of at least $IC_{50}$<10 μm against the $AT_1$ and $AT^2$ subtype receptors thereby demonstrating and confirming the utility of the compounds of the invention as effective A II antagonists with "balanced" $AT_1/AT_2$ activity.

The antihypertensive effects of the compounds described in the present invention may be evaluated using the methodology described below: Male Charles River Sprague-Dawley rats (300-375 gm) were anesthetized with methohexital (Brevital; 50 mg/kg i.p.) and the trachea was cannulated with PE 205 tubing A stainless steel pithing rod (1.5 mm thick., 150 mm long) was inserted into the orbit of the right eye and down th spinal column. The rats were immediately placed on a Harvard Rodent Ventilator (rate - 60 strokes per minute, volumn - 1.1 cc per 100 grams body weight). The right carotid artery was ligated, both left and right vagal nerves were cut, and the left carotid artery was cannulated with PE 50 tubing for drug administration, and body temperature

was maintained at 37°C by a thermostatically controlled heating pad which received input from a rectal temperature probe. Atropine (1 mg/kg i.v.) was then administered, and 15 minutes later propranolol (1 mg/kg i.v.). Thirty minutes later angiotensin II or other agonists were administered intravenously at 30-minute intervals and the increase in the diastolic blood pressure was recorded before and after drug or vehicle administration.

Thus, the compounds of the invention are useful in treating hypertension. They are also of value in the management of acute and chronic congestive heart failure and angina. These compounds are also expected to be useful in primary and secondary hyperaldosteronism, renal diseases such as diabetic nephropathy, glomerulonephritis, glomerular sclerosis, nephrotic syndrome, hypertensive nephrosclerosis, end stage renal disease, renal transplant therapy, renovascular hypertension, scleroderma, left ventricular dysfunction, systolic and diastolic dysfunction diabetic retinopathy, in the management of vascular disorders such as migraine or Raynaud's disease, as prophylaxis to minimize the atherosclerotic process, in neointimal hyperplasia follwoing angioplasty or vascular injury and to retard the onset of type II diabetes. The application of the compounds of this invention for these and similar disorders will be apparent to those skilled in the art.

The compounds of this invention are also useful to treat elevated intraocular pressure and to enhance retinal blood flow and can be administered to patients in need of such treatment with typical pharmaceutical formulations such as tablets, capsules, injectables and the like as well as topical ocular formulations in the form of solutions, ointments, inserts, gels, and the like. Pharmaceutical formulations prepared to treat intraocular pressure would typically contain about 0.1% to 15% by weight, preferably 0.5% to 2% by weight, of a compound of this invention. For this use, the compounds of this invention may also be used in combination with other medications for the treatment of glaucoma including choline esterase inhibitors such as physostigmine salicylate or demecarium bromide, parasympathomimetic agents such as pilocarpine nitrate, β-adrenergic antagonists such as timolol maleate, adrenergic agonists such as epinephrine and carbonic anhydrase inhibitors such as MK-507.

In the management of hypertension and the clinical conditions noted above, the compounds of this invention may be utilized in compositions such as tablets, capsules or elixirs for oral administration, suppositories for rectal administration, sterile solutions or suspensions for parenteral or intramuscular administration, and the like. The compounds of this invention can be administered to patients (animals and human) in need of such treatment in dosages that will provide optimal pharmaceutical efficacy. Although the dose will vary from patient to patient depending upon the nature and severity of disease, the patient's weight, special diets then being followed by a patient, concurrent medication, and other factors which those skilled in the art will recognize, the dosage range will generally be about 1 to 1000 mg. per patient per day which can be administered in single or multiple doses Preferably, the dosage range will be about 5.0 to 500 mg. per patient per day; more preferably about 5 to 300 mg. per patient per day.

The compounds of this invention can also be administered in combination with other antihypertensives and/or diuretics. For example, the compounds of this invention can be given in combination with diuretics such as hydrochlorothiazide, chlorothiazide, chlorthalidone, methyclothiazide, furosemide, ethacrynic acid, triamterene, amiloride, atriopeptin and spironolactone; calcium channel blockers, such as diltiazem, felodipine, nifedipine, amlodipine, nimodipine, isradipine, nitrendipine and verapamil; β-adrenergic antagonists such as timolol, atenolol, metoprolol, propanolol, nadolol and pindolol; angiotensin converting enzyme inhibitors such as enalapril, lisinopril, captopril, ramipril, quinapril and zofenopril; renin inhibitors such as A-69729 and FK 906 and FK 744; α-adrenergic antagonists such as prazosin, doxazosin, and terazosin; sympatholytic agents such as methyldopa, clonidine and guanabenz, atriopeptidase inhibitors (alone or with ANP) such as UK-79300; serotonin antagonists such as ketanserin; $A_2$-adenosine receptor agonists such as CGS 22492C; potassium channel agonists such as pinacidil and cromakalim; and various other antihypertensive drugs including reserpine, minoxidil, guanethidine, hydralazine hydrochloride and sodium nitroprusside as well as combinations of the above-named drugs.

Combinations useful in the management of congestive heart failure include, in addition, compounds of this invention with cardiac stimulants such as dobutamine and xamoterol and phosphodiesterase inhibitors including amrinone and milrinone.

Typically, the individual daily dosages for these combinations can range from about one-fifth of the minimally recommended clinical dosages to the maximum recommended levels for the entities when they are given singly.

To illustrate these combinations, one of the angiotensin II antagonists of this invention effective clinically in the 5-500 milligrams per day ranpe can be effectively combined at levels of the 10-500 milligrams per day range with the following compounds at the indicated per day dose range:

hydrochlorothiazide (6-100 mg), chlorothiazide (125-500 mg), ethacrynic acid (5-200 mg), amiloride (5-20 mg), furosemide (5-80 mg), propanolol (10-480 mg), timolol maleate (1-20 mg), methyldopa (125-2000 mg), felodipine (1-20 mg), nifedipine (5-120 mg), nitrendipine (5-60 mg) and diltiazem (30-540 mg). In addition, triple drug

combinations of hydrochlorothiazide (5-100 mg) plus amiloride (5-20 mg) plus an angiotensin II antagonist of this invention (1-500 mg) or hydrochlorothiazide (5-100 mg) plus timolol maleate (5-60) plus an angiotensin II antagonist of this invention (1-500 mg) or hydrochlorothiazide (5-200 mg) and nifedipine (5-60 mg) plus an angiotensin II antagonist of this invention (1-500 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 1 to 100 mg. of compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unitform is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occuring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The following examples illustrate the preparation of the compounds of formula (I) and their incorporation into pharmaceutical compositions and as such are not to be considered as limiting the invention set forth in the claims appended hereto.

## EXAMPLE 1

4-[[2'-(N-Benzoylsulfamoyl)biphenyl-4-yl]methyl]-5-n-butyl-2,4-dihydro-2-[2-(trifluoromethyl)phenyl]-3H-1,2,4-triazol-3-one

### Step A: Ethyl Valerimidate (Free Base)

A 127 g (76.7 mmole) sample of ethyl valerimidate hydrochloride [prepared from valeronitrile, ethanol, and hydrogen chloride gas as described by A.J. Hill and I. Rabinowitz, J. Am. Chem. Soc., 48, 734 (1926)] was dissolved in 33% (w/w) potassium carbonate solution (made by dissolving 15 g of $K_2CO_3$ in 30 ml of $H_2O$) and immediately extracted with ether (3x40 ml). The combined ether layers were dried over $Na_2SO_4$, filtered, and concentrated in vacuo to give 7.09 g (72%) of the product as a clear oil, which was used directly in the next step.

300 MHz NMR ($CDCl_3$)δ 0.88 (t, J= 7Hz, 3H), 1.24 (t, J= 7Hz, 3H), 1.31 (m, 2H), 1.50 (m, 2H), 2.19 (t, J= 7.5Hz, 2H), 4.06 (q, J= 7Hz, 2H), 6.84 (br s, 1H).

### Step B: Ethyl N-Carbethoxyvalerimidate

A solution of 6.5 g (50.3 mmole) of ethyl valerimidate (free base) in 90 ml of dry $CH_2Cl_2$ was treated with 7.71 ml (5.60 g, 55.3 mmole) of triethylamine. The resulting solution was stirred under $N_2$ at -10°C in an ice-salt bath as a solution of 4.81 ml (5.46 g, 50.3 mmole) of ethyl chloroformate in 10 ml of $CH_2Cl_2$ was added dropwise over 25 minutes. Upon completion of the addition, the cooling bath was removed, and the mixture was stirred at room temperature for 2 hours. Next, the solvent was removed by evaporation in vacuo. The residue was taken up in hexane and filtered to remove triethylamine hydrochloride. Concentration of the filtrate yielded 7.08 g (70%) of the product as a yellow oil, suitable for use in the next step without further purification. NMR indicated a mixture of syn and anti isomers. TLC (98:2 $CH_2Cl_2$-MeOH) showed a close pair of spots, $R_f$ 0.48, 0.52; mass spectrum (EI) m/e 201 ($M^+$).

200 MH: NMR ($CDCl_3$)δ 0.86 (distorted t, J= 7.5Hz, 3H), 2.15-2.35 (m, 8H), 2.4-2.65 (m, 2H), 2.19, 2.35 (t, J= 7.5Hz, 2H total), 4.0-4.2 (m, 4H).

Step C: 2-Bromo-N-(tert-butyl)benzenesulfonamide

To a stirred solution of 2-bromobenzenesulfonyl chloride (Lancaster Synthesis) (2.21 g, 8.65 mmol) in chloroform (40 ml) under nitrogen at room temperature was added tert-butylamine (Aldrich) (2.30 ml, 21.9 mmol). The orange solution was stirred at room temperature for 12 hours, then the mixture evaporated to dryness. Flash chromatography (silica gel, 15% ethyl acetate-hexane) afforded the title compound (2.12 g, 84%) as a white solid; $^1$H NMR (300 MHz, $CDCl_3$) δ 8.18 (d, J = 8.5 Hz, 1H), 7.73 (d, J = 8.5 Hz, 1H), 7.50-7.35 (m, 2H), 5.11 (s, 1H), 1.20 (s, 9H).

Step D: p-Tolyltrimethyltin

p-Tolylmagnesium bromide solution (Aldrich) (1.0M solution in diethyl ether) (53 ml, 0.0530 mol) was added dropwise to trimethyltin chloride (6.92 g, 0.0347 mol) in tetrahydrofuran (50 ml) under nitrogen at -10°C. The suspension was allowed to warm slowly to room temperature over 3 hours; then saturated ammonium chloride solution (10 ml) was added, followed by sufficient water to dissolve the precipitate. The solution was extracted three times with diethyl ether-hexane (1:1). The combined organic phase was washed with brine, dried (magnesium sulfate) and the solvents removed in vacuo. Vacuum distillation of the residue afforded a colorless liquid (bp 39-40°C, 0.1 mm Hg) which was further purified by flash chromatography (silica gel, hexane) to give p-tolyltrimethyltin (7.30 g, 82%) as a colorless liquid; $^1$H NMR (300 MHz, $CDCl_3$) δ 7.40 (d, J = 7.7 Hz, 2H), 7.19 (d, J = 7.7 Hz, 2H), 2.34 (s, 3H), 0.30 (s, 9H).

Step E: 2′-(N-t-Butylsulfamoyl)-4-methylbiphenyl

2-Bromo-N-(tert-butyl)benzenesulfonamide (from Step A) (1.00 g, 3.92 mmol), p-tolyltrimethyltin (from Step B) (1.95 g, 6.67 mmol), bis(triphenylphosphine)palladium(II) chloride (Aldrich) (165 mg, 0.235 mmol) and dimethylformamide (25 ml) were heated with stirring under nitrogen at 90°C for 5 hours. The black suspension was cooled to room temperature, then filtered through a pad of Celite which was washed with tetrahydrofuran. The colorless filtrate was evaporated to dryness then chromatographed (silica gel, 10% ethyl acetate-hexane) to give the title compound (0.88 g, 74%) as a white solid; $^1$H NMR (300 MHz, $CDCl_3$) δ 8.16 (d, J = 7.9 Hz, 1H), 7.60-7.37 (m, 4H), 7.36-7.24 (m, 3H), 3.57 (s, 1H), 2.42 (s, 3H), 0.99 (s, 9H).

Step F: [2′-(N-t-Butylsulfamoyl)biphenyl-4-yl]-methyl bromide

N-Bromosuccinimide (387 mg, 2.17 mmol), α,α′-azobis(isobutyronitrile) (catalytic), 2′-(N-t-butylsulfamoyl)-4-methylbiphenyl (from Step C) (550 mg, 1.81 mmol) and carbon tetrachloride (50 ml) were heated with stirring at reflux for 3 hours. After cooling to room temperature the mixture was filtered and the filtrate evaporated to dryness. Flash chromatography (silica gel, initially 10 and then 20% ethyl acetate-hexane) afforded the title compound [699 mg, 77% pure (the remainder of the material was the corresponding dibromo derivative)] as a white solid; $^1$H NMR (300 MHz, $CDCl_3$) δ 8.17 (dd, J = 7.5, 1.6 Hz, 1H), 7.68-7.45 (m, 6H), 7.31 (dd, J = 7.5, 1.6 Hz, 1H), 4.55 (s, 2H), 3.52 (s, 1H), 100 (s, 9H).

Step G: 5-n-Butyl-2,4-dihydro-2-[2-(trifluoromethyl)phenyl]-3H-1,2,4-triazol-3-one

To a solution of 1.56 g (8.86 mmole) of 2-(trifluoromethyl)phenylhydrazine in 18 mL of toluene was added 1.96 g (9.75 mmole) of ethyl N-carbethoxyvalerimidate (from Step B), and the solution was stirred at 50°C for 1.5 hour. Then 1.36 mL (984 mg, 9.75 mmole) of triethylamine was added, and the solution was stirred at 90°C for 15 hours. The solution was concentrated in vacuo, and the residue was re-concentrated twice from toluene. Flash chromatography of the crude product on silica gel (gradient elution with 0.5-2% MeOH in $CH_2Cl_2$) gave 1.65 g (66%) of the title compound as white crystals, mp 124-126°C; homogeneous by TLC in 19:1 $CH_2Cl_2$-MeOH; mass spectrum (FAB) m/e 286 $(M+1)^+$. 400 MHz NMR ($CDCl_3$) δ 0.88 (t, 3H), 1.34 (m, 2H), 1.62 (m, 2H), 2.52 (t, 2H), 7.5-7.6 (m, 2H), 7.66 (dd, 1H), 779 (d, 1H), 11.75 (br s, 1H).

Step H: 5-n-Butyl-4-[[2′-(N-t-butylsulfamoyl)biphenyl-4-yl]methyl]-2,4-dihydro-2-[2-(trifluoromethyl)phenyl]-3H-1,2,4-triazol-3-one

To a solution of 227 mg of 5-n-butyl-2,4-dihydro-2-[2-(trifluoromethyl)phenyl]-3H-1,2,4-triazol-3-one (from Step G) in 1.6 mL of dry DMF were added 38.2 mg (0.955 mmole) of sodium hydride (60% in oil) and 457 mg (1.12 mmole) of [2′-(N-t-butylsulfamoyl)biphenyl-4-yl]methyl bromide (from Step F). The solution was stirred

at 50°C for 1.5 hour. The mixture was then quenched by addition of 15 mL of $H_2O$ and extracted with 3 x 10 mL of ethyl acetate. The combined ethyl acetate fractions were washed with 2 x 15 mL of $H_2O$ and the 1 x 15 mL of saturated NaCl solution. The organic phase was dried over $Na_2SO_4$, filtered, and concentrated in vacuo. Flash chromatography of the crude product on silica gel (gradient elution with 0.3-10% MeOH in $CH_2Cl_2$) gave 343 mg (74%) of the title compound as white crystals, mp 168-170°C; homogeneous by TLC in 98:2 $CH_2Cl_2$-MeOH; mass spectrum (FAB) m/e 587 $(M+1)^+$.
400 MHz NMR ($CDCl_3$) δ 0.89 (t, 3H), 0.97 (s, 9H), 1.37 (m, 2H), 1.64 (m, 2H), 2.48 (t, 2H), 3.48 (s, 1H), 4.95 (s, 2H), 7.2-7.6 (m, 9H), 7.66 (dd, 1H), 7.78 (d, 1H), 8.15 (d, 1H).

Step I: 5-n-Butyl-2,4-dihydro-4-[(2′-sulfamoylbiphenyl-4-yl)methyl]-2-[2-(trifluoromethyl)phenyl]-3H-1,2,4-triazol-3-one

A solution of 200 mg (0.341 mmole) of 5-n-butyl-4-[[2′-(N-t-butylsulfamoyl)biphenyl-4-yl]-methyl]-2,4-dihydro-2-[2-(trifluoromethyl)phenyl]-3H-1,2,4-triazol-3-one (from Step H) and 3 drops of anisole in 3.4 mL of trifluoroacetic acid was purged with $N_2$ and stirred at room temperature overnight. The solvent was then evaporated under a gentle stream of $N_2$. The residue was dissolved in $CH_2Cl_2$ and washed twice with 5% $NaHCO_3$ solution. The organic phase was dried ($Na_2SO_4$), filtered, and concentrated in vacuo. The residue was re-concentrated twice from toluene and then flash chromatographed on silica gel (packed in $CH_2Cl_2$, gradient elution with 0.5-5% $CH_2Cl_2$ in MeOH) to yield 154 mg (85%) of the title compound as white crystals, mp 74-76°C; homogeneous by TLC in 98:2 $CH_2Cl_2$-MeOH; mass spectrum (FAB) m/e 531 $(M+1)^+$.
400 MHz NMR ($CDCl_3$) δ 0.88 (t, 3H), 1.36 (m, 2H), 1.63 (m, 2H), 2.50 (t, 2H), 4.23 (br s, 2H), 4.95 (s, 2H), 7.3-7.6 (m, 9H), 7.65 (dd, 1H), 7.78 (d, 1H), 8.14 (d, 1H).

Step J: 4-[[2′-(N-Benzoylsulfamoyl)biphenyl-4-yl]-methyl]-5-n-butyl-2,4-dihydro-2-[2-(trifluoromethyl)phenyl]-3H-1,2,4-triazol-3-one

To a solution of 55.7 mg (0.105 mmole) of 5-n-butyl-2,4-dihydro-4-[(2′-sulfamoylbiphenyl-4-yl)methyl]-2-[2-(trifluoromethyl)phenyl]-3H-1,2,4-triazol-3-one (from Step I) in 1.0 mL of pyridine was added 122 μL (147.5 mL, 1.05 mmole) of benzoyl chloride The light orange solution was purged with $N_2$, stoppered, and stirred at room temperature for 12 hours. The reaction was quenched by addition of 5 mL of saturated $KH_2PO_4$ solution and then extracted with 3 x 10 mL of ethyl acetate. The combined organic fractions were washed with 15 mL of saturated aqueous NaCl, then dried ($Na_2SO_4$), filtered, and concentrated in vacuo. Flash chromatography of the crude product on silica gel (gradient elution with 0.5-5% MeOH in $CH_2Cl_2$) provided 52 mg (75%) of the title compound as white crystals, mp 91-93°C; satisfactory purity by TLC in 19:1 $CH_2Cl_2$-MeOH; mass spectrum (FAB) m/e 673 $(M+K)^+$.
Analysis: ($C_{33}H_{29}F_3N_4O_4S \cdot 0.33CH_2Cl_2$)
Calcd: C, 60.39; H, 4.51; N, 8.45.
Found: C, 60.28; H, 4.49; N, 8.15.
400 MHz NMR ($CDCl_3$) δ 0.87 (t, 3H), 1.36 (m, 2H), 1.63 (m, 2H), 2.47 (t, 2H), 4.86 (s, 2H), 7.11 (d, 2H), 72-7.7 (m, 13H), 7.75 (d, 1H), 8.25 (br s, 1H), 8.39 (d, 1H).

## EXAMPLE 2

4-[[2′-(N-Benzoylsulfamoyl)biphenyl-4-yl]methyl]-5-n-butyl-2,4-dihydro-2-[2-(trifluoromethyl)phenyl]-3H-1 2,4-triazol-3-one

A solution of 145 mg (1.18 mmole) of benzoic acid and 192 mg (1.18 mmole) of 1,1′-carbonyldiimidazole in 1 mL of THF was stirred under $N_2$ at 55°C for 2 hours. Then a solution of 157 mg (0.296 mmole) of 5-n-butyl-2,4-dihydro-4-[(2′-sulfamoylbiphenyl-4-yl)methyl]-2-[2-(trifluoromethyl)phenyl]-3H-1,2,4-triazol-3-one (from Example 1, Step I) and 133 mL (135 mg, 0.888 mmole) of 1,8-diazabicyclo[5.4.0]-undec-7-ene (DBU) in 1 mL of THF was added dropwise. After being stirred overnight at 55°C, the mixture was partitioned between ethyl acetate and 5% citric acid (aqueous). The organic layer was washed with $H_2O$ and then with saturated NaCl solution. The ethyl acetate phase was then dried over $Na_2SO_4$, filtered, and concentrated in vacuo. Flash chromatography of the residue as in Example 1, Step J, gave 159 mg (85%) of the title compound, homogeneous by TLC in 98:2 $CH_2Cl_2$-MeOH and comparable in physical properties to the product made according to Example 1, Step J.

EXAMPLE 3

4-[[2'-(N-Benzoylsulfamoyl)biphenyl-4-yl]methyl]-5-n-butyl-2,4-dihydro-2-[2-(trifluoromethyl)phenyl]-3H-1,2,4-triazol-3-one, potassium salt

A 13.8 g (21.8 mmole) sample of 4-[[2'-(N-benzoylsulfamoyl)biphenyl-4-yl]methyl]-5-n-butyl-2,4-dihydro-2-[2-(trifluoromethyl)phenyl]-3H-1,2,4-triazol-3-one (prepared according to Example 1 or Example 2) was dissolved in a mixture of 100 mL of methanol and 100 mL of THF and stirred under $N_2$ as 218 mL (21.8 mL) of 1 N KOH in methanol was added. The resulting solution was stirred for 1 hour and then concentrated to dryness. The residue was dissolved in 150 mL of THF and stirred vigorously as 1650 mL of hexane was added gradually, resulting in precipitation The solid was collected on a filter and dried to give 13.8 g (94%) of the title compound as a white solid, which had high purity by HPLC ($C_{18}$ reverse phase, elution with 40:60:1 $H_2O$-acetonitrile-trifluoroacetic acid).

Analysis: ($C_{33}H_{29}F_3KN_4O_4S$)
Calcd: C, 58.92; H, 4.20; N, 8.32; K, 5.81.
Found: C, 58.76; H, 4.29; N, 8.17; K, 5.93.
400 MHz NMR ($CD_3OD$) δ 0.90 (t, J = 7.3 Hz, 3H), 1.40 (m, 2H), 1.62 (m, 2H), 2.56 (t, J = 7.5 Hz, 2H), 4.97 (s, 2H), 7.14-7.19 (m, 3H), 7.24 (dd, J = 7.2 Hz, 7.3 Hz, 2H), 7.34 (dd, J ≃ 7 Hz, 7 Hz, 1H), 7.41 (d, J = 8.2 Hz, 2H), 7.49 (m, 2H), 7.59-7.65 (m, 3H), 7.71 (dd, J = 7.0 Hz, 7.0 Hz, 1H), 7.81 (dd, J = 6.6 Hz, 6.7 Hz, 1H), 7.88 (d, J = 6.7 Hz, 1H), 8.26 (m, 1H).

EXAMPLE 4

5-n-Butyl-4-[[2'-[N-(cyclopropanecarbonyl)sulfamoyl]-biphenyl-4-yl]methyl]-2,4-dihydro-2-[2-(trifluoromethyl)phenyl]-3H-1,2,4-triazol-3-one

Following the procedure of Example 1, Step J, 5-n-butyl-2,4-dihydro-4-[(2'-sulfamoylbiphenyl-4-yl)-methyl]-2-[2-(trifluoromethyl)phenyl]-3H-1,2,4-triazol-3-one (from Example 1, Step I) was reacted with cyclopropanecarbonyl chloride Flash chromatography of the crude product on silica gel (gradient elution with 0.3-1% MeOH in $CH_2Cl_2$) gave a 51% yield of the title compound as white crystals, mp 187-189°C; satisfactory purity by TLC in 19:1 $CH_2Cl_2$-MeOH; mass spectrum (FAB) m/e 599 $(M+1)^+$.

Analysis ($C_{30}H_{29}F_3N_4O_4S\cdot 0.25H_2O$):
Calcd: C, 59.79; H, 4.85; N, 9.30.
Found: C, 59.82; H, 4.81; N, 8.90.
400 MHz NMR ($CDCl_3$) δ 0.71 (m, 2H), 0.84-0.92 (m, 5H, including t, 3H, centered at 0.89), 1.38 (m, 2H), 1.55-1.7 (m, 3H), 2.55 (t, 2H), 4.92 (s, 2H), 7.25-7.7 (m, 10H), 7.77 (d, 1H), 8.26 (d, 1H), 8.51 (br s, 1H).

EXAMPLE 5

5-n-Butyl-4-[[2'-[N-[(S)-2,2-dimethylcyclopropanecarbonyl]sulfamoyl]biphenyl-4-yl]methyl]-2,4-dihydro-2-[2-(trifluoromethyl)phenyl]-3H-1,2,4-triazol-3-one

A solution of (S)-2,2-dimethylcyclopropanecarboxylic acid (194 mg, 1.70 mmol), and carbonyldiimidazole (275 mg, 170 mmol) in THF was stirred at 50°C for 3 hrs. To this was added dropwise a solution of DBU (258 mg, 1.7 mmol) and 5-n-butyl-2,4-dihydro-4-[(2'-sulfamoylbiphenyl-4-yl)methyl]-2-[2-(trifluoromethyl)phenyl]-3H-1,2,4-triazol-3-one (300 mg, 0.57 mmol) (from Example 1, Step I) in THF. After stirring at 50°C overnight, the reaction mixture was quenched by addition of 5% citric acid and extracted with EtOAc The organic layers were combined and washed with water, 5% $NaHCO_3$, and brine, and dried over anhydrous $Na_2SO_4$. The crude material obtained after filtration and evaporation of volatiles was flash chromatographed over 60 mL silica gel (gradient elution using 0.5-5.0% MeOH/$CH_2Cl_2$) to afford the title compound in 74% yield (263 mg) as a white solid, homogeneous by TLC in 5% MeOH/$CH_2Cl_2$; mass spectrum (FAB) m/e 627 $(M+1)^+$, 649 $(M+Na)^+$; mp 93-95°C.

Analysis $C_{32}H_{33}F_3N_4O_4S\cdot 0.25\ H_2O$
Calcd: C, 60.89; H, 5.35; N, 8.88.
Found: C, 60.68; H, 5.35; N, 8.88.
400 MHz NMR ($CDCl_3$) δ 0.75(dd, J=7.5Hz, 4.0Hz, 1H), 0.89(t, J=7.4 Hz, 3H) 0.96(s, 3H), 0.99(s, 3H), 1.01-1.08(m, 2H), 1.35-1.41(m, 2H), 1.62-1.70(m, 2H), 2.54 (t, J=7.4Hz, 2H), 4.95(s, 2H), 7.27-7.38(m, 5H), 7.52-7.79(m, 6H), 7.92(s, br, 1H), 8.27(dd, J=7.9Hz, 1.3Hz, 1H)

EXAMPLE 6

5-n-Butyl-2,4-dihydro-4-[[2′-(N-pivaloylsulfamoyl)-biphenyl-4-yl]methyl]-2-[2-(trifluoromethyl)phenyl]-3H-1,2,4-triazol-3-one

The title compound was prepared from 5-n-butyl-2,4-dihydro-4-[(2′-sulfamoylbiphenyl-4-yl)-methyl]-2-[2-(trifluoromethyl)phenyl]-3H-1,2,4-triazol-3-one (from Example 1, Step I), according to the procedure of Example 5 except that pivalic acid was used as the acid. Excess pivalic acid was removed by 10% aq. $KHCO_3$. The crude product was purified by flash chromatography (gradient elution using 1-5% $MeOH/CH_2Cl_2$) to afford an 86% yield of the desired compound as a white solid, homogeneous by TLC in 5% $MeOH/CH_2Cl_2$; mass spectrum (FAB) m/e 615 $(M+1)^+$; mp 176-178°C.
Analysis: ($C_{31}H_{33}F_3N_4O_4S$)
Calcd: C, 60.57; H, 5.41; N, 9.11.
Found: C, 60.27; H, 5.55; N, 8.86.
400 MHz NMR ($CDCl_3$) δ 0.88(t, J=7.2Hz, 3H), 0.96(s, 9H), 1.40(m, 2H), 1.65(m, 2H), 2.51(t, J=7.2Hz, 2H), 4.93(s, 2H), 7.26-7.42(m, 5H), 7.53-7.79(m, 6H), 8.31(dd, J=8.0Hz, 1.4Hz, 1H).

EXAMPLE 7

5-n-Butyl-2,4-dihydro-4-[[2′-[N-(2-norbornanecarbonyl)sulfamoyl]biphenyl-4-yl]methyl]-2-[2-(trifluoromethyl)phenyl]-3H-1,2,4-triazol-3-one

A solution of 53 mg (0.375 mmole) of 2-norbornanecarboxylic acid and 61 mg (0.375 mmole) of 1,1′-carbonyldiimidazole (CDI) in 1.5 mL THF was stirred under $N_2$ at 65°C for 3 hours. Then a solution of 100 mg (0.189 mmole) of 5-n-butyl-2,4-dihydro-4-[(2′-sulfamoylbiphenyl-4-yl)methyl]-2-[2-(trifluoromethyl) phenyl]-3H-1,2,4-triazol-3-one (from Example 1, Step I) and 56.5 mL (57.5 mg, 0.378 mmole) of DBU in 1.4 mL of THF was added dropwise. After being stirred overnight at 55°C, the mixture was partitioned between ethyl acetate and 5% citric acid (aqueous) The organic layer was washed with water and then with saturated NaCl solution. The ethyl acetate phase was then dried over anhydrous $Na_2SO_4$, filtered, and concentrated in vacuo. Flash chromatography of the residue (eluting with 1% MeOH in $CH_2Cl_2$) gave 30 mg (24%) of the pure product as a glassy foam, mp 89-92°C, homogeneous by TLC in 95:5 $CH_2Cl_2$-MeOH; mass spectrum (FAB) m/e 653 $(M+1)^+$. 400 MH: NMR ($CDCl_3$) δ 0.89 (t, J=7.4 Hz, 3H), 1.02 (m, 2H), 1.12-1.85 (m, 12H), 2.18 (m, 1H), 2.53 (m, 2H), 2.80 (m, 1H), 4.95 (s, 2H), 5.95 (m, 1H), 7.20-7.82 (m, 11H), 8.29 (d, J=8.0 Hz, 1H).

EXAMPLE 8

5-n-Butyl-4-[[2′-[N-(cycloheptanecarbonyl)sulfamoyl]-biphenyl-4-yl]methyl]-2,4-dihydro-2-[2-(trifluoromethyl)phenyl]-3H-1.2,4-triazol-3-one

The title compound was prepared from cycloheptanecarboxylic acid (2.0 equivalents), CDI (2.0 equivalents), 5-n-butyl-2,4-dihydro-4-[(2′-sulfamoylbiphenyl-4-yl)methyl]-2-[2-(trifluoromethyl)phenyl]-3H-1,2,4-triazol-3-one (from Example 1, Step I) (1.0 equivalent), and DBU (2.0 equivalents) according to the procedure of Example 7, to give a 69% yield of the desired material after flash chromatography, as a glassy solid, mp 146-149°C, homogeneous by TLC in 95:5 $CH_2Cl_2$-MeOH; mass spectrum (FAB) m/e 655 $(M+1)^+$.
400 MHz NMR ($CDCl_3$) d 0.87 (t, J=7.4Hz, 3H) 1.20-1.80 (m, 14H), 1.94 (m, 2H), 2.50 (m, 3H), 4.87 (s, 2H), 7.23-7.79 (m, 11H), 8.29 (d, J=6.5Hz, 1H).

Additional 4-[[2′-(N-acylsulfamoyl)biphenyl-4-yl]methyl]-5-alkyl-2,4-dihydro-2-[2-(trifluoromethyl)phenyl]-3H-1,2,4-triazol-3-ones prepared by the methods described in the foregoing Examples are tabulated below:

TABLE II

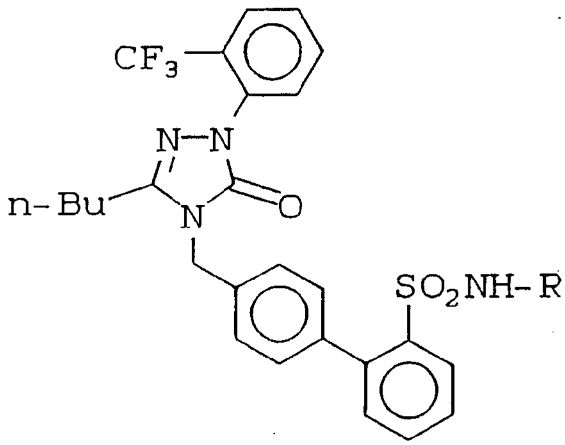

TABLE II (cont'd)

| R | mp | Formula | | Analysis C | H | N |
|---|---|---|---|---|---|---|
| (1) 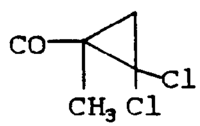 | 189-191°C | $C_{31}H_{29}Cl_2F_3N_4O_4S$ | calcd: | 54.63 | 4.29 | 8.22 |
| | | | found: | 54.63 | 4.07 | 7.96 |
| (2) 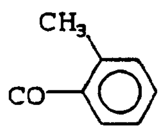 | 93- 95°C | $C_{34}H_{31}F_3N_4O_4S$ | calcd: | 62.95 | 4.82 | 8.64 |
| | | | found: | 62.66 | 4.95 | 8.44 |
| (3) $COCH(CH_3)_2$ | 93- 95°C | $C_{30}H_{31}F_3N_4O_4S \cdot 0.5\ H_2O$ | calcd: | 59.10 | 5.29 | 9.19 |
| | | | found: | 59.04 | 5.23 | 9.10 |
| (4) 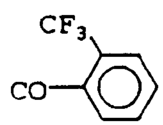 | 197-199°C | $C_{34}H_{28}F_6N_4O_4S \cdot 0.25H_2O$ | calcd: | 57.75 | 4.06 | 7.92 |
| | | | Found: | 57.93 | 3.98 | 7.80 |

**TABLE II** cont'd

| R | | mp | formula | | Analysis C | Analysis H | Analysis N |
|---|---|---|---|---|---|---|---|
| (5) | 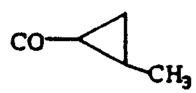 | 94- 96°C | $C_{31}H_{31}F_3N_4O_4S$ | calcd: | 60. 77 | 5. 10 | 9. 14 |
| | | | | found: | 60. 53 | 5. 06 | 9. 12 |
| (6) | 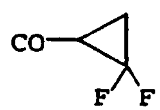 | 178-180°C | $C_{30}H_{27}F_5N_4O_4S$ | calcd: | 56. 78 | 4. 29 | 8. 83 |
| | | | | found: | 56. 54 | 4. 10 | 8. 55 |
| (7) | 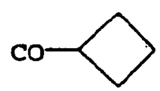 | 184-185°C | $C_{31}H_{31}F_3N_4O_4S \cdot H_2O \cdot$ 0. 05 $CHCl_3$ | calcd: | 58. 58 | 5. 23 | 8. 80 |
| | | | | found: | 58. 44 | 4. 88 | 8. 68 |
| (8) | 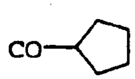 | 95- 97°C | $C_{32}H_{33}F_3N_4O_4S \cdot$ 0. 2 $H_2O$ | calcd: | 60. 98 | 5. 34 | 8. 89 |
| | | | | found: | 60. 91 | 5. 12 | 8. 92 |

**TABLE II** cont'd

| R | mp | formula | | Analysis C | H | N |
|---|---|---|---|---|---|---|
| (9) $COCH_2C(CH_3)_3$ | 94- 96°C | $C_{32}H_{35}F_3N_4O_4S$ | calcd: | 61.13 | 5.61 | 8.91 |
| | | | found: | 60.93 | 5.52 | 8.75 |
| (10) 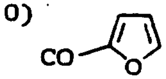 | 194-195°C | $C_{31}H_{27}F_3N_4O_5S$ | calcd: | 59.61 | 4.36 | 8.97 |
| | | | found: | 59.69 | 4.31 | 8.78 |
| (11) 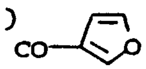 | 125-127°C | $C_{31}H_{27}F_3N_4O_5S$ | calcd: | 59.61 | 4.36 | 8.97 |
| | | | found: | 59.61 | 4.48 | 8.59 |
| (12) 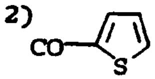 | >110°C (gradual) | $C_{31}H_{27}F_3N_4O_4S_2$ | calcd: | 58.11 | 4.25 | 8.75 |
| | | | found: | 57.94 | 4.28 | 8.54 |

**TABLE II** cont'd

| R | | mp | formula | | Analysis C | Analysis H | Analysis N |
|---|---|---|---|---|---|---|---|
| (13) | 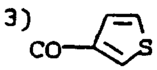 | 123-126°C | $C_{31}H_{27}F_3N_4O_4S_2$ | calcd: | 58.11 | 4.25 | 8.75 |
| | | | | found: | 58.10 | 4.35 | 8.47 |
| (14) | 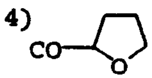 | 149-151°C | $C_{31}H_{31}F_3N_4O_5S \cdot 0.5H_2O$ | calcd: | 58.39 | 5.06 | 8.79 |
| | | | | found: | 58.47 | 4.86 | 8.58 |
| (15) | 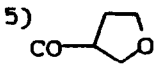 | 154-155°C | $C_{31}H_{31}F_3N_4O_5S \cdot 0.5H_2O$ | calcd: | 58.39 | 5.06 | 8.79 |
| | | | | found: | 58.43 | 4.94 | 8.65 |
| (16) | 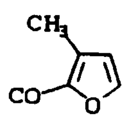 | >95°C (gradual) | $C_{32}H_{29}F_3N_4O_5S \cdot 0.2H_2O \cdot 0.3\ C_4H_{10}O$ (ether) | calcd: | 60.01 | 4.91 | 8.43 |
| | | | | found: | 59.63 | 5.00 | 8.13 |

**TABLE II** cont'd

| | R | mp | formula | | Analysis C | H | N |
|---|---|---|---|---|---|---|---|
| (17) | 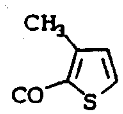 | >145°C (gradual) | $C_{32}H_{29}F_3N_4O_4S_2$ • 0.7 $CH_2Cl_2$ | calcd: | 54.92 | 4.43 | 7.84 |
| | | | | found: | 55.07 | 4.39 | 7.51 |
| (18) | 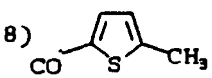 | >125°C (gradual) | $C_{32}H_{29}F_3N_4O_4S_2$ • 0.25 $CH_2Cl_2$ | calcd: | 57.30 | 4.40 | 8.29 |
| | | | | found: | 57.69 | 4.77 | 7.93 |
| (19) | 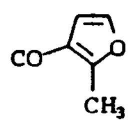 | 154-157°C | $C_{32}H_{29}F_3N_4O_5S$ • 0.75 $H_2O$ | calcd: | 58.93 | 4.71 | 8.59 |
| | | | | found: | 58.64 | 4.73 | 8.52 |
| (20) | 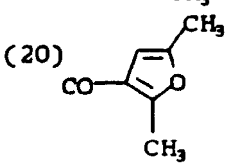 | 220-223°C | $C_{33}H_{31}F_3N_4O_5S$ • 0.75 $H_2O$ | calcd: | 59.50 | 4.92 | 8.41 |
| | | | | found: | 59.64 | 4.96 | 8.26 |

**TABLE II** cont'd

| | R | mp | formula | | Analysis C | H | N |
|---|---|---|---|---|---|---|---|
| (21) | 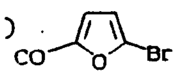 | >125°C (gradual) | $C_{31}H_{26}BrF_3N_4O_5S\ H_2O$ | calcd: | 51.60 | 3.91 | 7.77 |
| | | | | found: | 51.44 | 3.53 | 7.59 |
| (22) | 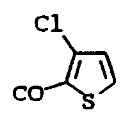 (K salt) | >200°C (gradual) | $C_{31}H_{25}ClF_3KN_4O_4S_2$ • 0.3 $C_4H_{10}O$ (ether) | calcd: | 52.59 | 3.84 | 7.62 |
| | | | | found: | 52.79 | 3.60 | 7.77 |
| (23) | 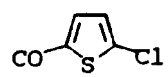 | >125°C (gradual) | $C_{31}H_{26}ClF_3N_4O_4S_2$ • 0.5 $H_2O$ | calcd: | 54.42 | 3.99 | 8.19 |
| | | | | found: | 54.76 | 4.39 | 7.79 |
| (24) | 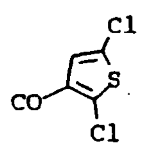 | >130°C (gradual) | $C_{31}H_{25}Cl_2F_3N_4O_4S_2$ • 0.7 $CH_2Cl_2$ | calcd: | 49.51 | 3.46 | 7.29 |
| | | | | found: | 49.46 | 3.42 | 7.19 |

**TABLE II** cont'd

| | R | mp | formula | | Analysis C | H | N |
|---|---|---|---|---|---|---|---|
| (25) | 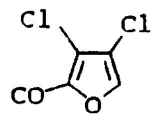 | >140°C (gradual) | $C_{31}H_{25}Cl_2F_3N_4O_5S \cdot$ 0.5 $CH_2Cl_2$ | calcd:<br>found: | 51.41<br>51.34 | 3.56<br>3.51 | 7.61<br>7.57 |
| (26) | 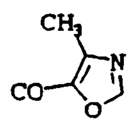 | >155°C (gradual) | $C_{31}H_{28}F_3N_5O_5S \cdot$ $H_2O \cdot 0.4$ $CH_2Cl_2$ | calcd:<br>found: | 54.53<br>54.25 | 4.49<br>4.43 | 10.13<br>10.49 |
| (27) | 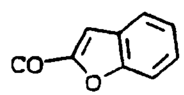 | 124-127°C | $C_{35}H_{29}F_3N_4O_5S \cdot H_2O$ 0.2 $C_4H_{10}O$ (ether) | calcd:<br>found: | 60.77<br>60.85 | 4.70<br>4.68 | 7.92<br>7.58 |
| (28) | 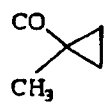 | 193-195°C | $C_{31}H_{31}F_3N_4O_4S \cdot$ 0.25 $H_2O$ | calcd:<br>found: | 60.33<br>60.29 | 5.14<br>4.98 | 9.08<br>8.83 |

**TABLE II** cont'd

| | R | mp | formula | | Analysis C | Analysis H | Analysis N |
|---|---|---|---|---|---|---|---|
| (29) | 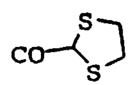 | >75°C (gradual) | $C_{30}H_{29}F_3N_4O_4S \cdot$ 0.5 $H_2O$ | calcd: | 53.64 | 4.50 | 8.34 |
| | | | | found: | 53.62 | 4.23 | 7.99 |
| (30) | 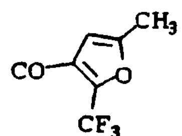 | 218-219°C | $C_{33}H_{28}F_6N_4O_5S$ | calcd: | 56.09 | 3.99 | 7.93 |
| | | | | found: | 55.81 | 3.74 | 7.76 |
| (31) | 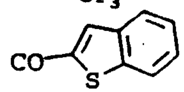 | 177-179°C | $C_{35}H_{29}F_3N_4O_4S_2 \cdot$ 0.4 $CH_2Cl_2$ | calcd: | 58.67 | 4.14 | 7.73 |
| | | | | found: | 58.64 | 3.85 | 7.68 |
| (32) | 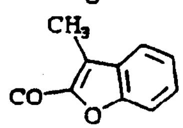 | 103-106°C | $C_{36}H_{31}F_3N_4O_5$ | calcd: | 62.78 | 4.54 | 8.14 |
| | | | | found: | 62.58 | 4.29 | 8.01 |

TABLE II cont'd

| | R | mp | formula | | Analysis C | H | N |
|---|---|---|---|---|---|---|---|
| (33) | 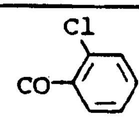 (K salt) | 168-170°C | $C_{33}H_{28}ClF_3KN_4O_4S$ | calcd: | 59.24 | 4.22 | 8.37 |
| | | | | found: | 58.99 | 4.32 | 8.11 |
| (34) | 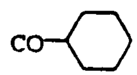 | 101-103°C | $C_{33}H_{35}F_3N_4O_4S$ | calcd: | 61.86 | 5.51 | 8.74 |
| | | | | found: | 61.63 | 5.38 | 8.52 |
| (35) | 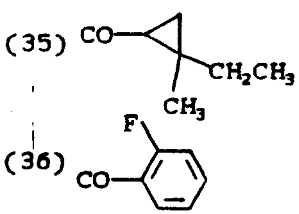 | 90-92°C | $C_{33}H_{35}F_3N_4O_4S$ | calcd: | 61.86 | 5.51 | 8.74 |
| | | | | found: | 61.74 | 5.61 | 8.49 |
| (36) | | 98-100°C | $C_{33}H_{28}F_4N_4O_4S \cdot 0.25\ H_2O$ | calcd: | 60.31 | 4.37 | 8.53 |
| | | | | found: | 60.06 | 4.12 | 8.33 |

**TABLE II cont'd**

| R | mp | Formula | | Analysis C | Analysis H | Analysis N |
|---|---|---|---|---|---|---|
| (37) 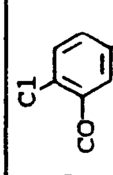 | 142-145°C | $C_{33}H_{27}Cl_2F_3N_4O_4S$ | calcd: | 55.62 | 3.96 | 7.86 |
| | | | Found: | 55.33 | 3.56 | 7.65 |
| (38) 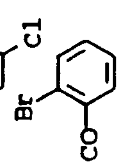 | 90-93°C | $C_{33}H_{28}BrF_3N_4O_4S$ • 0.25 $H_2O$ | calcd: | 55.20 | 4.00 | 7.80 |
| | | | Found: | 54.98 | 3.78 | 7.54 |
| (39) 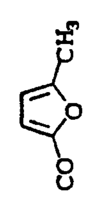 | 210-211°C | $C_{32}H_{29}F_3N_4O_5S$ | calcd: | 60.18 | 4.58 | 8.77 |
| | | | Found: | 59.89 | 4.55 | 8.62 |

## EXAMPLE 9

5-n-Butyl-4-[[2'-[N-(2-chlorobenzoyl)sulfamoyl]-biphenyl-4-yl]methyl]-2,4-dihydro-2-[4-nitro-2-(trifluoromethyl)phenyl}-3H-1,2,4-triazol-3-one

Step A: 5-n-Butyl-2,4-dihydro-2-[4-nitro-2-(trifluoromethyl)phenyl]-3H-1,2,4-triazol-3-one

By the procedure of Example 1, Step G, 4-nitro-2-(trifluoromethyl)phenylhydrazine [generated from the hydrochloride which was prepared from 4-nitro-2-(trifluoromethyl)aniline according to H. Stroh and G. Westphal, Chem. Ber., 96, 184 (1963), by partitioning between ether and IN sodium carbonate] was reacted with ethyl N-carbethoxyvalerimidate (Example 1, Step B). After work-up, the residue was purified by flash chromatography on silica gel (gradient elution with 0.5-5.0% methanol in $CH_2Cl_2$) to give a 27% yield of the title compound as an orange solid, mp 126-128°C, homogeneous by TLC (19:1 $CH_2Cl_2$-MeOH); mass spectrum (FAB) m/e 331 $(M+1)^+$. 400 MHz $^1H$ NMR ($CDCl_3$) δ 0.91 (t, J=7.3 Hz, 3H), 1.38 (m, 2H), 1.66 (m, 2H), 2.57 (t, J=7.6 Hz,

2H), 7.83 (d, J=8.8 Hz, 1H), 8.50 (dd, J=8.8, 2.6 Hz, 1H), 8.67 (d, J=2.6 Hz, 1H), 11.25 (br s, 1H).

Step B: 5-n-Butyl-4-[[2′-(N-t-butylsulfamoyl-biphenyl-4-yl]methyl]-2,4-dihydro-2-[4-nitro-2-(trifluoromethyl)phenyl]-3H-1,2,4-triazol-3-one

By the procedure of Example 1, Step H, 5-n-butyl-2,4-dihydro-2-[4-nitro-2-(trifluoromethyl)-phenyl]-3H-1,2,4-triazol-3-one.(from Step A) was alkylated with [2-(N-t-butylsulfamoyl)biphenyl-4-yl]-methyl bromide (from Example 1, Step F). Flash chromatography of the crude product on silica gel (gradient elution with 0.5-5.0% MeOH in $CH_2Cl_2$) gave an 88% yield of the title compound as an orange solid, mp >78°C (gradual), homogeneous by TLC (98:2 $CH_2Cl_2$-MeOH), mass spectrum (FAB) m/e 632 $(M+1)^+$.
400 MH: $^1$H NMR ($CDCl_3$) δ 090 (t, J=7.4 Hz, 3H), 0.98 (s, 9H), 1.40 (m, 2H), 1.66 (m, 2H), 2.50 (t, J=7.5 Hz, 2H), 3.47 (s, 1H), 4.95 (s, 2H), 7.25-7.60 (m, 7H), 7.92 (d, J=9.1 Hz, 1H), 8.15 (dd, J=7.9, 1.4 Hz, 1H) 8.48 (dd, J=8.9, 2.6 Hz, 1H), 8.66 (d, J=2.5 Hz, 1H).

Step C: 5-n-Butyl-2,4-dihydro-2-[4-nitro-2-(trifluoromethyl)phenyl]-4-[(2′-sulfamoylbiphenyl-4-yl)methyl]-3H-1,2,4-triazol-3-one

The title compound was prepared from 5-n-butyl-4-[[2′-(N-t-butylsulfamoyl)biphenyl-4-yl]-methyl]-2,4-dihydro-2-[4-nitro-2-(trifluoromethyl)-phenyl]-3H-1,2,4-triazol-3-one (from Step B) according to the procedure of Example 1, Step I, and was obtained in 69% yield as a cream-colored solid, mp 218-220°C, homogenous by TLC (19:1 $CH_2Cl_2$-MeOH); mass spectrum (FAB) m/e 576 $(M+1)^+$.
400 MHz $^1$H NMR ($CDCl_3$) δ 0.89 (t, J=7.3 Hz, 3H), 1.38 (m, 2H), 1.65 (m, 2H), 2.52 (t, J=7.5 Hz, 2H), 4.20 (s, 2H), 4.96 (s, 2H), 7.25-7.61 (m, 7H), 7.92 (d, J=8.9 Hz, 1H), 8.14 (dd, J=7.6, 1.0 Hz, 1H), 8.48 (dd, J=8.8, 2.5 Hz, 1H), 8.66 (d, J=2.5 Hz, 1H).

Step D: 5-n-Butyl-4-[[2′-[N-(2-chlorobenzoyl)sulfamoyl]biphenyl-4-yl]methyl]-2,4-dihydro-2-[4-nitro-2-(trifluoromethyl)phenyl]-3H-1.2,4-triazol-3-one

The title compound was prepared from 2-chlorobenzoic acid (2.0 equivalents), CDI (2.0 equiv), 5-n-butyl-2,4-dihydro-2-(4-nitro-2-trifluoromethyl)phenyl-4-[(2′-sulfamoylbiphenyl-4-yl)methyl]-3H-1,2,4-triazol-3-one (from Step C) (1.0 eq), and DBU (2.0 equiv) according to the procedure of Example 2, to give a 43% yield of the desired material after flash chromatography, as a glassy solid, mp 179-181°C, homogeneous by TLC in 95:5 $CH_2Cl_2$-MeOH; mass spectrum (FAB) m/e 714 $(M+1)^+$.
Analysis ($C_{33}H_{27}ClF_3N_5O_6S$)
Calcd: C, 55.50; H, 3.81; N, 9.81.
Found: C, 55.60; H, 4.03; N, 9.66.
400 MHz NMR ($CDCl_3$) δ 0.89 (t, J=7.3 Hz, 3H) 1.37 (m, 2H), 1.64 (m, 2H), 2.47 (t, J=7.5 Hz, 2H), 4.88 (s, 2H), 7.17-7.70 (m, 10H), 7.91 (d, J=8.9 Hz, 1H), 8.36 (d, J=7.9, 1.5 Hz, 1H), 8.43 (s, 1H), 8.49 (dd, J=8.8, 2.6 Hz, 1H), 8.67 (d, J=2.6 Hz, 1H)

EXAMPLE 10

2-[4-Amino-2-(trifluoromethyl)phenyl]-5-n-butyl-4-[[2′-[N-(2-chlorobenzoyl)sulfamoyl]biphenyl-4-yl]-methyl]-2,4-dihydro-3H-1,2,4-triazol-3-one

At 0°C, under nitrogen, to a solution of 82 mg (0.115 mmole) of 5-n-butyl-4-[(2′-[N-(2-chlorobenzoyl)sulfamoylbiphenyl-4-yl)methyl]-2,4-dihydro-2-(4-nitro-2-trifluoromethyl)phenyl-3H-1,2,4-triazol-3-one (from Example 9, Step D) dissolved in 1 mL of THF was added dropwise 182 mg (0.805 mmole) of stannous chloride, dissolved in 2.2 mL of concentrated HCl. After stirring at 0°C for 15 minutes the ice/water bath was removed, and stirring continued for 30 min. The reaction mixture was poured onto a mixture of 2.8 g ice, 0.8 mL of 50% NaOH, and 1.6 mL of EtOAc. After stirring for 15 min, the phases were separated and the aqueous phase was reextracted with EtOAc twice. The combined organic layer was washed with brine, dried over sodium sulfate, filtered, and evaporated. Flash chromatography of the crude residue over silica gel (gradient elution using 0.5-2.0% MeOH/$CH_2Cl_2$) gave 53 mg (67%) of a cream-colored solid, mp 134-136°C, homogeneous by TLC (9:1 MeOH/$CH_2Cl_2$), mass spectrum (FAB) m/e 684 $(M+1)^+$.
Analysis ($C_{33}H_{29}ClF_3N_5O_4S\cdot 0.5\ H_2O$)
Calcd: C, 57.20; H, 4.36; N, 10.10.
Found: C, 57.04; H, 4.48; N, 9.77.

400MHz NMR ($CD_3OD$) δ 0.87 (t, J=7.4 Hz, 3H) 1.35 (m, 2H), 1.56 (m, 2H), 2.52 (t, J=7.5 Hz, 2H), 4.99 (s, 2H), 6.89-7.71 (m, 14H), 8.29 (d, J=8.0, 1.3 Hz, 1H)

EXAMPLE 11

5-n-Butyl-4-[[2′-[N-(2-chlorobenzoyl)sulfamoyl]-biphenyl-4-yl]methyl]-2,4-dihydro-2-[4-(propionylamino)-2-(trifluoromethyl)phenyl]-3H-1,2,4-triazol-3-one

At room temperature, under $N_2$, a solution of 45 mg (0.066 mmole) of 2-[4-amino-2-(trifluoromethyl)phenyl]-5-n-butyl-4-[[2′-[N-(2-chlorobenzoyl)-sulfamoyl]biphenyl-4-yl]methyl]-2,4-dihydro-3H-1,2,4-triazol-3-one (from Example 10) in DMF (0.5 mL) was stirred with 1.7 mg (0.072 mmole) of sodium hydride for 3 h. Subsequently, 9.1 mg (0.099 mmole) of propionyl chloride was added and the resulting reaction mixture was stirred at 50°C overnight. The reaction was quenched by addition of water. The organic material was extracted with EtOAc and washed with water and brine, then dried over sodium sulfate. After filtration and removal of volatiles, the crude product was flash chromatographed over silica gel (gradient elution with 0.5-5% $MeOH/CH_2Cl_2$) to afford the desired material as a cream-colored solid, mp 204-206°C, homogeneous by TLC (9:1 $MeOH/CH_2Cl_2$), mass spectrum (FAB) m/e 741 $(M+1)^+$.
400 MHz $^1H$ NMR ($CDCl_3$) δ 0.87 (t, J=7.3 Hz, 3H), 1.19 (t, J=7.5 Hz, 3H), 1.34 (m, 2H), 1.61 (m, 2H), 2.37 (q, J=7.5 Hz, 2H), 2.47 (t, J=7.6 Hz, 2H), 4.92 (s, 2H), 6.37 (s, br, 1H), 7.14-7.75 (m, 12H), 7.89 (s, 1H), 8.19 (s, 1H), 8.36 (d, J=7.1 Hz, 1H), 8.63 (s, br, 1H).

EXAMPLE 12

2-[4-(Benzylamino)-2-(trifluoromethyl)phenyl]-5-n-butyl-4-[[2′-[N-(2-chlorobenzoyl)sulfamoyl]biphenyl-4-yl]methyl]-2,4-dihydro-3H-1.2,4-triazol-3-one

A solution of 70 mg (0.093 mmole) of 2-[4-amino-2-(trifluoromethyl)phenyl]-5-n-butyl-4-[[2′-[N-(2-chlorobenzoyl)sulfamoyl]biphenyl-4-yl]methyl]-2,4-dihydro-3H-1,2,4-triazol-3-one (from Example 10), 33 mg (0.31 mmole) of benzaldehyde, 2.1 mL of piperidine, and 2.5 mL of isopropanol was stirred at 95°C overnight. The crude material obtained after cooling and evaporation of volatiles was dissolved in 2.2 mL MeOH, charged with 1.3 mL of a 1M solution of sodium cyanoborohydride in THF (1.3 mmole), and stirred at room temperature for 2 hours. Water (160 mL) was added at 0°C, and the resulting mixture stirred at 0°C for 2 hours. After evaporation of volatiles, the crude product was flash chromatographed over silica gel (gradient elution with 0.5-5.0% $MeOH/CH_2Cl_2$) to afford 27 mg (38%) of the desired product as a cream-colored solid, mp >107°C (gradual), homogeneous by TLC (9:1 $MeOH/CH_2Cl_2$), mass spectrum (FAB) m/e 774 $(M+1)^+$.
Analysis: ($C_{40}H_{35}ClF_3N_5O_4S \cdot 2\ CH_2Cl_2$)
Calcd: C, 53.66; H, 4.18; N, 7.45.
Found: C, 53.80; H, 3.89; N, 7.47.
400 MHz $^1H$ NMR ($CD_3OD$) δ 0.87 (t, J=7.3 Hz, 3H), 1.33 (m, 2H), 1.57 (m, 2H), 2.51 (t, J=7.5 Hz, 2H), 4.41 (s, 2H), 4.98 (s, 2H), 6.82-7.73 (m, 19H), 8.29 (dd, J=8.1, 1.4 Hz, 1H).

EXAMPLE 13

5-n-Butyl-4-[[2′-[N-(2-chlorobenzoyl)sulfamoyl]-biphenyl-4-yl]methyl]-2-(2-chloro-5-nitrophenyl)-2,4-dihydro-3H-1,2,4-triazol-3-one

Step A: 5-n-Butyl-2-(2-chloro-5-nitrophenyl)-2,4-dihydro-3H-1,2,4-triazol-3-one

By the procedure of Example 1, Step G, 2-chloro-5-nitrophenylhydrazine [generated from the hydrochloride, which was prepared from 2-chloro-5-nitroaniline according to H. Stroh and G. Westphal, Chem. Ber. 96, 184 (1963), by partitioning between ether and IN sodium carbonate] was reacted with ethyl N-carbethoxyvalerimidate (from Example 1, Step B). Enough THF was added to the reaction mixture to ensure dissolution of all starting material. After work-up, the residue was purified by flash chromatography on silica gel (gradient elution with 0.5-5.0% methanol in $CH_2Cl_2$) to give a 19% yield of the title compound as an orange solid, mp 145-147°C, homogeneous by TLC (19:1 $CH_2Cl_2$-MeOH), mass spectrum (FAB) m/e 297 $(M+1)^+$.
400 MHz $^1H$ NMR ($CDCl_3$) δ 0.91 (t, J=7.3 Hz, 3H), 1.39 (m, 2H), 1.67 (m, 2H), 2.58 (t, J=7.7 Hz, 2H), 7.70 (d, J=8.9 Hz, 1H), 8.22 (dd, J=8.8, 2.6 Hz, 1H), 8.38 (d, J=2.6 Hz, 1H), 11.62 (s, 1H).

Step B: 5-n-Butyl-4-[[2′-(N-t-butylsulfamoyl)-biphenyl-4-yl]methyl]-2-(2-chloro-5-nitrophenyl)-2,4-dihydro-3H-1,2,4-triazol-3-one

By the procedure of Example 1, Step H, 5-n-butyl-2-(2-chloro-5-nitrophenyl)-2,4-dihydro-3H-1,2,4-triazol-3-one (from Step A) was alkylated with [2-(N-t-butylsulfamoyl)biphenyl-4-yl]methyl bromide (from Example 1, Step F). Flash chromatography of the crude product on silica gel (gradient elution with 0.5-5.0% MeOH in $CH_2Cl_2$) gave an 83% yield of the title compound as an orange solid, mp >75°C (gradual), homogeneous by TLC (98:2 $CH_2Cl_2$-MeOH), mass spectrum (FAB) m/e 598 $(M+1)^+$.
400 MH: $^1H$ NMR ($CDCl_3$) δ 0.91 (t, J=7.4 Hz, 3H), 0.98 (s, 9H), 1.40 (m, 2H), 1.66 (m, 2H), 2.52 (t, J=7.6 Hz, 2H), 3.49 (s, 1H), 4.96 (s, 2H), 7.25-7.60 (m, 7H), 7.69 (d, J=8.8 Hz, 1H), 8.15 (dd, J=7.7, 1.5 Hz, 1H) 8.21 (dd, J=8.8, 2.6 Hz, 1H), 8.39 (d, J=2.6 Hz, 1H)

Step C: 5-n-Butyl-2-(2-chloro-5-nitrophenyl)-2,4-dihydro-4-[(2′-sulfamoylbiphenyl-4-yl)methyl]-3H-1,2,4-triazol-3-one

The title compound was prepared from 5-n-butyl-4-[[2′-(N-t-butylsulfamoyl)biphenyl-4-yl]methyl]-2-(2-chloro-5-nitrophenyl)-2,4-dihydro-3H-1,2,4-triazol-3-one (from Step B) according to the procedure of Example 1, Step I, and was obtained in 79% yield as a pale yellow solid after flash chromatographic purification, mp >90°C (gradual), homogenous by TLC (19:1 $CH_2Cl_2$-MeOH); mass spectrum (FAB) m/e 542 $(M+1)^+$.

400 MHz $^1H$ NMR ($CDCl_3$) δ 0.90 (t, J=7.4 Hz, 3H), 1.39 (m, 2H), 1.67 (m, 2H), 2.53 (t, J=7.6 Hz, 2H), 4.23 (s, 2H), 4.96 (s, 2H), 7.25-7.61 (m, 7H), 7.69 (d, J=8.8 Hz, 1H), 8.14 (dd, J=7.9, 1.2 Hz, 1H), 8.20 (dd, J=8.8, 2.6 Hz, 1H), 8.39 (d, J=2.6 Hz, 1H).

Step D: 5-n-Butyl-4-[[2′-[N-(2-chlorobenzoyl)sulfamoyl ]biphenyl-4-yl]methyl]-2-(2-chloro-5-nitrophenyl)-2,4-dihydro-3H-1,2,4-triazol-3-one

The title compound was prepared from 5-n-butyl-2-(2-chloro-5-nitrophenyl)-2,4-dihydro-4-[(2′-sulfamoylbiphenyl-4-yl)methyl]-3H-1,2,4-triazol-3-one (from Step C) and 2-chlorobenzoic acid (2 eq), CDI (2 eq), and DBU (2 eq) according to the procedure of Example 2 to give a 48% yield of the desired material as a cream-colored solid after flash chromatography, mp >160°C (gradual), homogeneous by TLC in 95:5 $CH_2Cl_2$-MeOH; mass spectrum (FAB) m/e 681 $(M+1)^+$.
400 MHz $^1H$ NMR ($CD_3OD$) δ 0.90 (t, J=7.3 Hz, 3H), 1.39 (m, 2H), 1.63 (m, 2H), 2.58 (t, J=7.4 Hz, 2H), 5.02 (s, 2H), 712-7.64 (m, 11H), 7.90 (dd, J=8.9, 2.8 Hz, 1H), 8.29 (dd, J=7.9, 1.2 Hz, 1H), 8.35 (dd, J=8.8, 2.6 Hz, 1H), 8.44 (d, J=2.7 Hz, 1H).

EXAMPLE 14

2-(5-Amino-2-chlorophenyl)-5-n-butyl-4-[[2′-[N-(2-chlorobenzoyl)sulfamoyl]biphenyl-4-yl]methyl]-2,4-dihydro-3H-1,2,4-triazol-3-one

This material was prepared from 5-n-butyl-4-[[2′-[N-(2-chlorobenzoyl)sulfamoyl]biphenyl-4-yl]methyl]-2-(2-chloro-5-nitrophenyl)-2,4-dihydro-3H-1,2,4-triazol-3-one (from Example 13, Step D) and stannous chloride dihydrate according to the procedure of Example 10. The crude product was flash chromatographed over silica gel to afford the title compound in 70% yield as a cream-colored solid, mp 155-157°C, homogeneous by TLC (9:1 MeOH/$CH_2Cl_2$), mass spectrum (FAB) m/e 651 $(M+1)^+$.
400 MHz $^1H$ NMR ($CD_3OD$) δ 0.88 (t, J=7.4 Hz, 3H), 1.36 (m, 2H), 1.59 (m, 2H), 2.54 (t, J=7.4 Hz, 2H), 5.00 (s, 2H), 6.75-6.79 (m, 2H), 7.10-7.65 (m, 12H), 8.29 (dd, J=8.0, 1.3 Hz, 1H).

EXAMPLE 15

5-n-Butyl-4-[[2′-[N-(2-chlorobenzoyl)sulfamoyl]biphenyl-4-yl]methyl]-2-[2-chloro-5-(propionylamino)-phenyl]-2,4-dihydro-3H-1.2,4-triazol-3-one

At room temperature, under $N_2$, a solution of 59 mg (0.091 mmole) of 2-(5-amino-2-chlorophenyl)-5-n-butyl-4-[[2′-[N-(2-chlorobenzoyl)sulfamoyl]biphenyl-4-yl]methyl]-2,4-dihydro-3H-1,2,4-triazol-3-one (from Example 14) in DMF was stirred with 2.4 mg (0.10 mmole) of sodium hydride for 3 hours. Subsequently, 25 mg (0.18 mmole) of propionyl bromide was added and the resulting reaction mixture was stirred at 50°C overnight. The reaction was quenched by addition of water. The organic material was extracted with EtOAc, washed with water

and brine, and dried over sodium sulfate. After filtration and removal of volatiles, the crude product was flash chromatographed over silica gel (gradient elution with 1-5% $MeOH/CH_2Cl_2$) to afford 26 mg (40%) of the desired material as a cream-colored solid, mp 160-162°C, homogeneous by TLC (9:1 $MeOH/CH_2Cl_2$), mass spectrum (FAB) m/e 707 $(M+1)^+$.
400 MHz $^1H$ NMR ($CD_3OD$) δ 0.89 (t, J=7.3 Hz, 3H), 1.18 (t, J=7.6 Hz, 3H), 1.36 (m, 2H), 1.61 (m, 2H), 2.39 (q, J=7.6 Hz, 2H), 2.56 (t, J=7.4 Hz, 2H), 5.01 (s, 2H), 7.12-7.69 (m, 13H), 7.93 (d, J=3.9 Hz, 1H), 8.29 (d, J=8.0 Hz, 1H).

EXAMPLE 16

5-n-Butyl-4-[[2′-[N-(2-chlorobenzoyl)sulfamoyl]biphenyl-4-yl]methyl]-2-[2-chloro-5-(valerylamino)-phenyl]-2.4-dihydro-3H-1,2,4-triazol-3-one

The title compound was prepared from 2-(5-amino-2-chlorophenyl)-5-n-butyl-4-[[2′-[N-(2-chlorobenzoyl)sulfamoyl]biphenyl-4-yl]methyl]-2,4-dihydro-3H-1,2,4-triazol-3-one (from Example 14), sodium hydride, and valeryl chloride according the procedure of Example 11 The crude product was flash chromatographed over silica gel (gradient elution with 0.5-2.0%. $MeOH/CH_2Cl_2$) to give the desired compound as a cream-colored solid in 48% yield, mp >110°C (gradual), homogeneous by TLC (9:1 $MeOH/CH_2Cl_2$), mass spectrum (FAB) m/e 734 $(M+1)^+$.
400 MH: $^1H$ NMR ($CD_3OD$) δ 0.89 (t, J=7.3 Hz, 3H), 0.95 (t, J=7.4 Hz, 3H), 1.39 (m, 4H), 1.61 (m, 4H), 2.38 (t, J=7.4Hz, 2H), 2.56 (t, J=7.4 Hz, 2H), 5.01 (s, 2H), 7.10-7.68 (m, 13H), 7.91 (d, J=2.4 Hz, 1H), 8.29 (dd, J=8.0, 1.3 Hz, 1H).

EXAMPLE 17

5-n-Butyl-4-[[2′-[N-(2-chlorobenzoyl)sulfamoyl]biphenyl-4-yl]methyl]-2-[2-chloro-5-[(propoxycarbonyl)-amino]phenyl]-2,4-dihydro-3H-1,2,4-triazol-3-one

At room temperature, under $N_2$, a solution of 20 mg (0.031 mmole) of 2-(5-amino-2-chlorophenyl)-5-n-butyl-4-[[2′-[N-(2-chlorobenzoyl)sulfamoyl]biphenyl-4-yl]methyl]-2,4-dihydro-3H-1,2,4-triazol-3-one (from Example 14), 3.8 mg (0.031 mmole) of DMAP, 18.9 mg (0.154 mmole) of propyl chloroformate, and 1 mL pyridine was sitrred overnight. After quenching with methanol and water, the organic material was extracted with EtOAc, washed with water and brine, and dried over sodium sulfate. The crude product obtained after filtration and removal of volatiles was flash chromatographed over silica gel (gradient elution with 0.5-5% $MeOH/CH_2Cl_2$) to give 15 mg (65%) of the desired compound as a white solid, mp >119°C (gradual), homogeneous by TLC (9:1 $MeOH/CH_2Cl_2$), mass spectrum (FAB) m/e 736 $(M+1)^+$.
Analysis: ($C_{36}H_{35}Cl_2N_5O_6S \cdot 0.5\ CH_2Cl_2$)
Calcd: C, 56.34; H, 4.66; N, 9.00.
Found: C, 56.26; H, 4.42; N, 8.68.
400 MHz $^1H$ NMR ($CD_3OD$) δ 0.89 (t, J=7.3 Hz, 3H), 0.99 (t, J=7.4 Hz, 3H), 1.37 (m, 2H), 1.61 (m, 2H), 1.69 (m, 2H), 2.55 (t, J=7.8 Hz, 2H), 4.09 (t, J=6.5 Hz, 2H), 5.01 (s, 2H), 7.13-7.75 (m, 14H), 8.28 (d, J=8.0 Hz, 1H).

EXAMPLE 18

5-n-Butyl-4-[[2′-[N-(2-chlorobenzoyl)sulfamoyl]biphenyl-4-yl]methyl]-2-[2-chloro-5-($N^3$-propylureido)phenyl]-2,4-dihydro-3H-1,2,4-triazol-3-one

At room temperature, under $N_2$, a solution of 34 mg (0.052 mmole) of 2-(5-amino-2-chlorophenyl)-5-n-butyl-4-[[2′-[N-(2-chlorobenzoyl)sulfamoyl]-biphenyl-4-yl]methyl]-2,4-dihydro-3H-1,2,4-triazol-3-one (from Example 14), 6.4 mg (0.052 mmole) of DMAP, 23 mg (0.26 mmole) of propyl isocyanate, and 1 mL of pyridine was sitrred overnight. After quenching with water, the organic material was extracted with EtOAc, washed with water and brine, and dried over sodium sulfate. The crude product obtained after filtration and removal of volatiles was flash chromatographed over silica gel (gradient elution with 0.5-5% $MeOH/CH_2Cl_2$) to give 23 mg (61%) of the desired compound as a white solid, mp >208°C (gradual), homogeneous by TLC (9:1 $MeOH/CH_2Cl_2$), mass spectrum (FAB) m/e 773 $(M+K)^+$.
Analysis: ($C_{36}H_{36}Cl_2N_6O_5S \cdot 0.5\ CH_2Cl_2$)
Calcd: C, 56.34; H, 4.79; N, 10.80.
Found: C, 56.22; H, 4.68; N, 10.86.

400 MHz $^1H$ NMR ($CD_3OD$) δ 0.91 (m, 6H), 1.38 (m, 2H), 1.51 (m, 2H), 1.61 (m, 2H), 2.55 (t, J=7.8 Hz, 2H), 3.12 (m, 2H), 4.99 (s, 2H), 7.12-7.70 (m, 14H), 8.28 (dd, J=7.9, 1.2 Hz, 1H).

EXAMPLE 19

5-n-Butyl-2-[5-(N-n-butylcarbamoyl)-2-chlorophenyl]-4-[[2′-[N-(2-chlorobenzoyl)sulfamoyl]biphenyl-4-yl]-methyl]-2,4-dihydro-3H-1,2,4-triazol-3-one

Step A: 5-n-Butyl-2-[5-(carbomethoxy)-2-chlorophenyl)-2,4-dihydro-3H-1,2,4-triazol-3-one

By the procedure of Example 1, Step G, 5-(carbomethoxy)-2-chlorophenylhydrazine [generated from the hydrochloride, which was prepared from 5-(carbomethoxy)-2-chloroaniline according to H. Stroh and G. Westphal, Chem. Ber. 96, 184 (1963), by partitioning between EtOAc and IN sodium carbonate] was reacted with ethyl N-carbethoxyvalerimidate (from Example 1, Step B). After work-up, the residue was purified by flash chromatography on silica gel (gradient elution with 0.5-10% methanol in $CH_2Cl_2$) to give a 34% yield of the title compound as an orange gum, homogeneous by TLC (19:1 $CH_2Cl_2$-MeOH), mass spectrum (FAB) m/e 310 $(M+1)^+$.
400 MHz $^1H$ NMR ($CDCl_3$) δ 0.88 (t, J=7.3 Hz, 3H), 1.34 (m, 2H), 1.64 (m, 2H), 2.54 (t, J=7.5 Hz, 28), 3.85 (s, 3H), 7.58 (d, J=8.5 Hz, 1H), 8.01 (dd, J=8.4, 2.1 Hz, 1H), 8.14 (d, J=2.0 Hz, 1H), 11.93 (s, 1H).

Step B: 5-n-Butyl-4-[[2′-(N-t-butylsulfamoyl)biphenyl-4-yl]methyl]-2-[5-(carbomethoxy)-2-chlorophenyl)-2,4-dihydro-3H-1,2,4-triazol-3-one

By the procedure of Example 1, Step H, 5-n-butyl-2-[5-(carbomethoxy)-2-chlorophenyl)-2,4-dihydro-3H-1,2,4-triazol-3-one (from Step A) was alkylated with [2-(N-t-butylsulfamoyl)biphenyl-4-yl]methyl bromide (from Example 1, Step F). Flash chromatography of the crude product on silica gel (gradient elution with 0.5-5.0% MeOH in $CH_2Cl_2$) gave an 85% yield of the title compound as a white solid, mp 65-67°C, homogeneous by TLC (98:2 $CH_2Cl_2$-MeOH), mass spectrum (FAB) m/e 555 $[M-(t-Bu)]^+$.
400 MH: $^1H$ NMR ($CDCl_3$) δ 0.90 (t, J=7.1 Hz, 3H), 0.98 (s, 9H), 1.38 (m, 2H), 1.64 (m, 2H), 2.50 (m, 2H), 3.50 (s, 1H), 3.90 (s, 3H), 4.96 (s, 2H), 7.26-7.62 (m, 8H), 7.99-8.20 (m, 3H).

Step C: 5-n-Butyl-2-[5-(carbomethoxy)-2-chlorophenyl]-2,4-dihydro-4-[(2′-sulfamoylbiphenyl-4-yl)methyl]-3H-1,2,4-triazol-3-one

The title compound was prepared from 5-n-butyl-4-[[2′-(N-t-butylsulfamoyl)biphenyl-4-yl]-methyl]-2-[5-(carbomethoxy)-2-chlorophenyl)-2,4-dihydro-3H-1,2,4-triazol-3-one (from Step B) according to the procedure of Example 1, Step I, and was obtained in 65% yield as a white solid after flash chromatography, mp 182-184°C, homogenous by TLC (19:1 $CH_2Cl_2$-MeOH); mass spectrum (FAB) m/e 555 $(M+1)^+$.
400 MHz $^1H$ NMR ($CDCl_3$) δ 0.89 (t, J=7.2 Hz, 3H), 1.40 (m, 2H), 1.66 (m, 2H), 2.51 (t, J=7.5 Hz, 2H), 3.90 (s, 3H), 4.21 (s, 2H), 4.96 (s, 2H), 7.30-7.61 (m, 9H), 8.00 (dd, J=8.4, 2.1 Hz, 1H), 8.15 (m, 1H).

Step D: 5-n-Butyl-2-[5-(carbomethoxy)-2-chlorophenyl]-4-[[2′-[N-(2-chlorobenzoyl)sulfamoyl]-biphenyl-4-yl]methyl]-2,4-dihydro-3H-1,2,4-triazol-3-one

The title compound was prepared from 5-n-butyl-2-[5-(carbomethoxy)-2-chlorophenyl]-2,4-dihydro-4-[(2′-sulfamoylbiphenyl-4-yl)methyl]-3H-1,2,4-triazol-3-one (from Step C) and 2-chlorobenzoic acid (2 equivalents), CDI (2 equiv), and DBU (2 equiv) according to the procedure of Example 2 to give an 88% yield of the desired material as a white solid after flash chromatography, mp 98-101°C, homogeneous by TLC in 95:5 $CH_2Cl_2$-MeOH; mass spectrum (FAB) m/e 693 $(M+1)^+$.
Analysis: ($C_{34}H_{30}Cl_2N_4O_6S \cdot 0.5\ H_2O$)
Calcd: C, 58.12; H, 4.45; N, 7.97.
Found: C, 57.95; H, 4.38; N, 7.84.
400 MHz $^1H$ NMR ($CDCl_3$) δ 0.90 (t, J=7.2 Hz, 3H), 1.39 (m, 2H), 1.63 (m, 2H), 2.47 (t, J=7.6 Hz, 2H), 3.91 (s, 3H), 4.88 (s, 2H), 7.19-7.69 (m, 11H), 8.00 (dd, J=8.4, 2.3 Hz, 1H), 8.15 (d, J=2.0 Hz, 1H), 8.36 (dd, J=7.8, 1.5 Hz, 1H), 8.45 (s, 1H).

Step E: 5-n-Butyl-2-[5-(N-n-butylcarbamoyl)-2-chlorophenyl]-4-[[2′-[N-(2-chlorobenzoyl)sulfamoyl]biphenyl-4-yl]methyl]-2,4-dihydro-3H-1,2,4-triazol-3-one

A solution of 100 mg (0.14 mmole) of 5-n-butyl-2-[5-(carbomethoxy)-2-chlorophenyl]-4-[[2′-[N-(2-chlorobenzoyl)sulfamoyl]biphenyl-4-yl]methyl]-2,4-dihydro-3H-1,2,4-triazol-3-one (from Step D), in 1 mL of n-butylamine was stirred at 65°C overnight. After removal of excess n-butylamine, the crude product was flash chromatographed over silica gel (gradient elution with 0.5-5.0% MeOH/$CH_2Cl_2$) to give 96 mg (91%) of the desired product as a white solid, mp >155°C (gradual), homogeneous by TLC in 95:5 $CH_2Cl_2$-MeOH; mass spectrum (FAB) m/e 734 $(M+1)^+$.
Analysis: ($C_{37}H_{37}Cl_2N_5O_5S\cdot 0.33\ CH_2Cl_2$)
Calcd: C, 58.77; H, 4.98; N, 9.18.
Found: C, 58.60; H, 4.69; N, 8.80.
400 MHz $^1H$ NMR ($CD_3OD$) δ 0.90 (t, J=7.3 Hz, 3H), 0.96 (t, J=7.4 Hz, 3H), 1.39 (m, 4H), 1.62 (m, 4H), 2.58 (t, J=7.5 Hz, 2H), 3.37 (t, J=7.2 Hz, 2H), 5.02 (s, 2H), 7.11-7.73 (m, 12H), 7.93 (dd, J=8.4, 2.2 Hz, 1H), 7.98 (d, J=2.2 Hz, 1H), 8.28 (d, J=8.0 Hz, 1H).

EXAMPLE 20

5-n-Butyl-4-[[2′-[N-(2-chlorobenzoyl)sulfamoyl]biphenyl-4-yl]methyl]-2-[3-(propionylamino)phenyl]-2,4-dihydro-3H-1.2.4-triazol-3-one

Step A: 5-n-Butyl-2,4-dihydro-2-(3-nitrophenyl)-3H-1,2,4-triazol-3-one

By the procedure of Example 1, Step G, 3-nitrophenylhydrazine [generated from the hydrochloride by partitioning between EtOAc and IN sodium carbonate] was reacted with ethyl N-carbethoxyvalerimidate (from Example 1, Step B). After work-up, the residue was purified by flash chromatography on silica gel (gradient elution with 0.5-1.0% methanol in $CH_2Cl_2$) to give a 75% yield of the title compound as a tan solid, homogeneous by TLC (98:2 $CH_2Cl_2$-MeOH), mass spectrum (FAB) m/e 263 $(M+1)^+$ mp 158-159°C,.
200 MHz $^1H$ NMR ($CDCl_3$) δ 1.00 (t, J=7.2 Hz, 3H), 1.46 (m, 2H), 1.75 (m, 2H), 2.69 (t, J=7.5 Hz, 2H), 7.60 (t, J=8.2 Hz, 1H), 8.06 (m, 1H), 8.36 (m, 1H), 8.92 (m, 1H), 11.55 (s, 1H).

Step B: 5-n-Butyl-4-[[2′-(N-t-butylsulfamoyl)biphenyl-4-yl]methyl]-2,4-dihydro-2-(3-nitrophenyl)-3H-1,2,4-triazol-3-one

By the procedure of Example 1, Step H, 5-n-butyl-2,4-dihydro-2-(3-nitrophenyl)-3H-1,2,4-triazol-3-one (from Step A) was alkylated with [2-(N-t-butylsulfamoyl)biphenyl-4-yl]methyl bromide (from Example 1, Step F). Flash chromatography of the crude product on silica gel (gradient elution with 0.5-10% MeOH in $CH_2Cl_2$) gave a 74% yield of the title compound as an off-white solid, mp 164-165°C, homogeneous by TLC (19:1 $CH_2Cl_2$-MeOH), mass spectrum (FAB) m/e 564 $(M+1)^+$.
400 MHz $^1H$ NMR ($CDCl_3$) δ 0.94 (t, J=7.3 Hz, 3H), 0.98 (s, 9H), 1.41 (m, 2H), 1.70 (m, 2H), 2.53 (t, J=7.6 Hz, 2H), 3.50 (s, 1H), 4.94 (s, 2H), 7.26-7.60 (m, 7H), 8.03 (m, 1H), 8.14 (dd, J=8.0, 0.8 Hz, 1H) 8.45 (m, 1H), 8.87 (m, 1H).

Step C: 5-n-Butyl-2,4-dihydro-2-(3-nitrophenyl)-4-[(2′-sulfamoylbiphenyl-4-yl)methyl]-3H-1,2,4-triazol-3-one

The title compound was prepared from 5-n-butyl-4-[[2′-(N-t-butylsulfamoyl)biphenyl-4-yl]-methyl]-2,4-dihydro-2-(3-nitrophenyl)-3H-1,2,4-triazol-3-one (from Step B) according to the procedure of Example 1, Step I, and was obtained in 94% yield as a pale yellow solid after flash chromatography, mp 73-76°C, homogenous by TLC (19:1 $CH_2Cl_2$-MeOH); mass spectrum (FAB) m/e 508 $(M+1)^+$.
400 MHz $^1H$ NMR ($CDCl_3$) δ 0.93 (t, J=7.3 Hz, 3H), 1.43 (m, 2H), 1.70 (m, 2H), 2.55 (t, J=7.4 Hz, 2H), 4.29 (s, 2H), 4.95 (s, 2H), 7.27-7.59 (m, 8H), 8.03 (m, 1H), 8.11 (dd, J=8.0, 1.3 Hz, 1H), 8.43 (m, 1H), 8.86 (m, 1H).

Step D: 5-n-Butyl-4-[[2′-[N-(2-chlorobenzoyl)sulfamoyl]biphenyl-4-yl]methyl]-2,4-dihydro-2-(3-nitrophenyl)-3H-1,2,4-triazol-3-one

The title compound was prepared from 5-n-butyl-2,4-dihydro-2-(3-nitrophenyl)-4-[(2′-sulfamoylbiphenyl-4-yl)methyl]-3H-1,2,4-triazol-3-one (from Step C) and 2-chlorobenzoic acid (2.5 equivalents), CDI (2.5 equiv), and DBU (2.5 equiv) according to the procedure of Example 2 to give a 95% yield of the desired material as a

cream-colored solid after flash chromatography, mp 92-95°C, homogeneous by TLC in 95:5 $CH_2Cl_2$-MeOH; mass spectrum (FAB) m/e 645 $(M+1)^+$.
Analysis: ($C_{32}H_{28}ClN_5O_6S \cdot 0.4\ CH_2Cl_2$)
Calcd: C, 57.22; H, 4.27; N, 10.30.
Found: C, 57.57; H, 4.14; N, 10.04.
400 MHz $^1H$ NMR ($CDCl_3$) $\delta$ 0.94 (t, J=7.3 Hz, 3H), 1.43 (m, 2H), 1.69 (m, 2H), 2.52 (t, J=7.4 Hz, 2H), 4.87 (s, 2H), 7.19-7.36 (m, 8H), 7.56-7.66 (m, 4H), 8.03 (m, 1H), 8.34 (dd, J=7.9, 1.3 Hz, 1H), 8.42 (m, 1H), 8.62 (s, 1H), 8.86 (m, 1H).

Step E: 2-(3-Aminophenyl)-5-n-butyl-4-[[2′-[N-(2-chlorobenzoyl)sulfamoyl]biphenyl-4-yl]-methyl]-2,4-dihydro-3H-1,2,4-triazol-3-one

This material was prepared from 5-n-butyl-4-[[2′-[N-(2-chlorobenzoyl)sulfamoyl]biphenyl-4-yl]-methyl]-2,4-dihydro-2-(3-nitrophenyl)-3H-1,2,4-triazol -3-one (from Example 13, Step D) and stannous chloride dihydrate according to the procedure of Example 10. The crude product was flash chromatographed over silica gel to afford the title compound in 95% yield as a cream-colored solid, mp 137-139°C, homogeneous by TLC (9:1 $MeOH/CH_2Cl_2$), mass spectrum (FAB) m/e 616 $(M+1)^+$.
Analysis: ($C_{32}H_{30}ClN_5O_4S \cdot 0.5\ H_2O \cdot 0.25\ CH_2Cl_2$)
Calcd: C, 59.93; H, 4.91; N, 10.84.
Found: C, 59.86; H, 4.52; N, 11.03.
400 MHz $^1H$ NMR ($CDCl_3$) $\delta$ 0.90 (t, J=7.2 Hz, 3H), 1.38 (m, 2H), 1.64 (m, 2H), 2.47 (t, J=7.6 Hz, 2H), 4.80 (s, 2H), 6.45 (d, J=6.8 Hz, 1H), 6.75-6.79 (m, 2H), 7.00-7.60 (m, 14H), 8.28 (d, J=7.1 Hz, 1H).

Step F: 5-n-Butyl-4-[[2′-[N-(2-chlorobenzoyl)sulfamoyl]biphenyl-4-yl]methyl]-2,4-dihydro-2-[3-(propionylamino)phenyl]-3H-1,2,4-triazol-3-one

This material was prepared from 2-(3-aminophenyl)-5-n-butyl-4-[[2′-[N-(2-chlorobenzoyl)sulfamoyl]biphenyl-4-yl]methyl]-2,4-dihydro-3H-1,2,4-triazol-3-one (from Example 13, Step E), sodium hydride, and propionyl bromide according to the procedure of Example 15 The crude product was flash chromatographed over silica gel to afford the title compound in 60% yield as a cream-colored solid, mp 105-107°C, homogeneous by TLC (9:1 $MeOH/CH_2Cl_2$), mass spectrum (FAB) m/e 672 $(M+1)^+$.
Analysis: ($C_{35}H_{34}ClN_5O_5S \cdot 0.3\ CH_2Cl_2$)
Calcd: C, 60.77; H, 5.00; N, 10.04.
Found: C, 60.46; H, 4.75; N, 9.70.
400 MHz $^1H$ NMR ($CDCl_3$) $\delta$ 0.93 (t, J=7.3 Hz, 3H), 1.24 (t, J=7.6 Hz, 3H), 1.43 (m, 2H), 1.68 (m, 2H), 2.38 (q, J=7.6Hz, 2H), 2.51 (t, J=7.6 Hz, 2H), 4.82 (s, 2H), 7.17-7.72 (m, 14H), 8.03 (d, J=1.6 Hz, 1H), 8.35 (dd, J=7.8, 1.6 Hz, 1H), 8.76 (s, 1H).

## EXAMPLE 21

5-n-Butyl-4-[[2′-[N-(2-chlorobenzoyl)sulfamoyl]-biphenyl-4-yl]methyl]-2,4-dihydro-2-[3-(valerylamino)-phenyl]-3H-1,2,4-triazol-3-one

This material was prepared from 2-(3-aminophenyl)-5-n-butyl-4-[[2′-[N-(2-chlorobenzoyl)sulfamoyl ]biphenyl-4-yl]methyl]-2,4-dihydro-3H-1,2,4-triazol-3-one (from Example 13, Step E), sodium hydride, and valeryl chloride according to the procedure of Example 16. The crude product was flash chromatographed over silica gel to afford the title compound in 67% yield as a cream-colored solid, mp 102-105°C, homogeneous by TLC (9:1 $MeOH/CH_2Cl_2$), mass spectrum (FAB) m/e 700 $(M+1)^+$.
Analysis: ($C_{37}H_{38}ClN_5O_5S \cdot 0.25\ CH_2Cl_2$)
Calcd: C, 62.01; H, 5.38; N, 9.71.
Found: C, 62.17; H, 5.32; N, 9.34.
400 MHz $^1H$ NMR ($CDCl_3$) $\delta$ 0.93 (t, J=7.4 Hz, 6H), 1.39 (m, 4H), 1.66 (m, 4H), 2.34 (t, J=7.3 Hz, 2H), 2.51 (t, J=7.6 Hz, 2H), 4.82 (s, 2H), 7.17-7.72 (m, 14H), 8.02 (d, J=1.9 Hz, 1H), 8.36 (dd, J=7.9, 1.4 Hz, 1H), 8.77 (s, 1H).

EXAMPLE 22

5-n-Butyl-4-[[2′-[N-(2-chlorobenzoyl)sulfamoyl]-5′-n-propylbiphenyl-4-yl]methyl]-2,4-dihydro-2-[2-(trifluoromethyl]-3H-1,2,4-triazol-3-one

Step A: N-t-Butyl-4-n-propylbenzenesulfonamide

To a solution of 4-n-propylbenzenesulfonyl chloride (Lancaster) in anhydrous $CH_2Cl_2$ (0.5 M solution) cooled to 0°C under $N_2$ was added t-butylamine (2.2 equiv) slowly through a dropping funnel. After complete addition, the reaction was stirred at room temperature for 12 hours. The $CH_2Cl_2$ was removed under reduced pressure, and the residue was extracted into ether and washed with 2N NaOH, $H_2O$ and brine. The organic phase was dried over anhydrous $MgSO_4$ and concentrated in vacuo to afford the titled product; $R_f$ = 0.46 (3:1 hexane-EtOAc).

$^1$H NMR (200 MHz, $CDCl_3$) δ 0.93 (t, 3H), 1.22 (s, 9H), 1.62 (m, 2H), 2.65 (t, 2H), 4.67 (bs, 1H), 7.27 (d, 2H), 7.79 (d, 2H).

Step B: 2-(N-t-butylsulfamoyl)-5-n-propylphenylboronic acid

To a solution of 2.85 g (11.2 mmol) of N-t-butyl-4-n-propylbenzenesulfonamide (from Step A) in anhydrous THF (20 mL) cooled to -40°C under $N_2$ was added 2.5M n-BuLi solution (11.2 mL, 2.5 equiv). The mixture was warmed to room temperature and stirred for 2 hours. To the mixture, containing the bright red dianion at 0°C, was added triisopropyl borate (3.9 mL, 1.5 equiv) The next day, 2N HCl (3 mL) was added and the mixture was stirred for 1 hour. The solvent was removed under reduced pressure and the residue was extracted with ethyl acetate. The organic solution was washed with 2N HCl, $H_2O$ and brine. The organic phase was dried over anhydrous $MgSO_4$ and concentrated in vacuo to afford the titled compound; $R_f$ = 0.5 (1:1 EtOAc-hexane). The material was used in the next step without further purification.

Step C: [2′-(N-t-Butylsulfamoyl)-5′n-propylbiphenyl-4-yl]methanol

To a solution of 2.80 g (9.36 mmol) of 2-(N-t-butylsulfamoyl)-5-n-propylphenylboronic acid (from Step B) and 4-bromobenzyl alcohol (5.25 g, 3 equiv) in toluene (125 mL) was added 1.25N NaOH (32 mL), EtOH (86 mL) and tetrakis(triphenylphosphine)-palladium(0) (325 mg, 3 mol %). The mixture was stirred at 100°C under $N_2$ for 3 hours. The reaction was concentrated and the residue was extracted with ethyl acetate. The organic solution was washed with IN NaOH, $H_2O$ and brine. Next, the organic phase was dried over anhydrous $MgSO_4$ and concentrated in vacuo. The product was purified by flash chromatography, eluting with 2:1 hexane-EtOAc, to provide the titled compound; $R_f$ = 0.42 (1:1 EtOAc-hexane).

$^1$H NMR (400 MHz, $CDCl_3$) δ 0.92 (t, 3H), 0.98 (s, 9H), 1.63 (m, 2H), 1.83 (bs, 1H), 2.63 (t, 2H), 3.57 (bs, 1H), 4.74 (s, 2H), 7.07 (d, 1H), 7.23 (dd, 1H), 7.42 (d, 2H), 7.49 (d, 2H), 8.02 (d, 1H).

Step D: 5-n-Butyl-4-[[2′-(N-t-butylsulfamoyl)-5′-n-propylbiphenyl-4-yl]methyl]-2,4-dihydro-2-[2-(trifluoromethyl)phenyl]-3H-1,2,4-triazol-3-one

Under nitrogen, to a solution of 110 mg (0.386 mmole) of 5-n-butyl-2,4-dihydro-2-[2-(trifluoromethyl)phenyl]-3H-1,2,4-triazol-3-one (from Example 13, Step A), 100 mg (0.257 mmole) of [2′-(N-t-butylsulfamoyl)-5′-n-propylbiphenyl-4-yl]-methanol (from Step C), and 101 mg (0.386 mmole) of triphenylphosphine in 1.2 mL of THF at -10°C, was added dropwise 78 mg (0.386 mmole) of diisopropyl azodicarboxylate. The reaction mixture was warmed up to room temperature, stirred overnight, and concentrated in vacuo. The crude product thus obtained was flash chromatographed over 40 mL of silica gel (gradient elution, 5:1 to 3:1 hexane/ethyl acetate) to afford a foam, homogeneous by TLC (1:1 hexane-EtOAc); mass spectrum (FAB) m/e 629 $(M+1)^+$.

400 MH: NMR ($CDCl_3$) δ 0.89 (t, J=7.2 Hz, 3H), 0.93 (t, J=7.2 Hz, 3H), 0.97 (s, 9H), 1.38 (m, 2H), 1.64 (m, 4H), 2.46 (t, J=7.9 Hz, 2H), 2.63 (t, J=7.6 Hz, 2H), 3.46 (s, 1H), 4.94 (s, 2H), 7.06 - 7.79 (m, 10H), 8.04 (d, J=8.3 Hz, 1H).

Step E: 5-n-Butyl-2,4-dihydro-4-[(2′-sulfamoyl-5′-n-propylbiphenyl-4-yl)methyl]-2-[2-(trifluoromethyl)phenyl]-3H-1,2,4-triazol-3-one

The title compound was prepared from 5-n-butyl-4-[(2′-sulfamoyl-5′-n-propylbiphenyl-4-yl)-methyl]-2-[2-(trifluoromethyl)phenyl]-3H-1,2,4-triazol-3-one (from Step D) according to the procedure of Example 8, Step

B. The crude product was flash chromatographed over silica gel (gradient elution with 0.5 - 1.0% MeOH in $CH_2Cl_2$), to provide the titled compound; homogeneous by TLC in 19:1 $CH_2Cl_2$-MeOH; mass spectrum (FAB) m/e 573 $(M+1)^+$.

400 MHz NMR ($CDCl_3$) δ 0.87 (t, J=7.3 Hz, 3H), 0.93 (t, J=7.3 Hz, 3H), 1.35 (m, 2H), 1.64 (m, 4H), 2.49 (t, J=7.6 Hz, 2H), 2.63 (t, J=7.5 Hz, 2H), 4.31 (s, 2H), 4.94 (s, 2H), 7.09 - 7.78 (m, 10H), 7.99 (d, J=8.1 Hz, 1H).

Step F: 5-n-Butyl-4-[[2′-[N-(2-chlorobenzoyl)-sulfamoyl]-5′-n-propylbiphenyl-4-yl]methyl]-2,4-dihydro-2-[2-(trifluoromethyl)phenyl]-3H-1,2,4-triazol-3-one

The title compound was prepared from 5-n-butyl-2,4-dihydro-2-[2-(trifluoromethyl)phenyl]-4-[(2′-sulfamoyl-5′-propylbiphenyl-4-yl)methyl]-3H-1,2,4-triazol-3-one (from Step E) and 2-chlorobenzoic acid (2.0 equivalents), CDI (2.0 equiv), and DBU (2.0 equiv) according to the procedure of Example 2 to give an 86% yield of the desired material as a cream-colored solid after flash chromatography, mp 67-69°C; homogeneous by TLC in 9:1 $CH_2Cl_2$-MeOH; mass spectrum (FAB) m/e 711 $(M+1)^+$.

400 MHz $^1H$ NMR ($CDCl_3$) δ 0.88 (t, J=7.3 Hz, 3H), 0.94 (t, J=7.3 Hz, 3H), 1.35 (m, 2H), 1.63 (m, 4H), 2.46 (t, J=7.6 Hz, 2H), 2.66 (t, J=7.9 Hz, 2H), 4.89 (s, 2H), 7.08-7.80 (m, 14H), 8.25 (d, J=8.3 Hz, 1H), 8.65 (s, 1H).

The following additional representative compounds of formula (I) can be prepared using the procedures of the foregoing Examples and Reaction Schemes:

(1) 5-n-butyl-4-[[2′-[N-(2,2-dichlorocyclopropanecarbonyl)sulfamoyl]biphenyl-4-yl]methyl]-2,4-dihydro-2-[2-(trifluoromethyl)phenyl]-3H-1,2,4-triazol-3-one;

(2) 5-n-butyl-4-[[2′-[N-(3-chloro-2-furoyl)sulfamoyl]biphenyl-4-yl]methyl]-2,4-dihydro-2-[2-(trifluoromethyl)phenyl]-3H-1,2,4-triazol-3-one;

(3) 5-n-butyl-4-[[2′-[N-(3-chloro-5-methyl-2-furoyl)sulfamoyl]biphenyl-4-yl]methyl]-2,4-dihydro-2-[2-(trifluoromethyl)phenyl]-3H-1,2,4-triazol-3-one;

(4) 5-n-butyl-4-[[2′-[N-(3-chlorobenzofuran-2-carbonyl)sulfamoyl]biphenyl-4-yl]methyl]-2,4-dihydro-2-[2-(trifluoromethyl)phenyl]-3H-1,2,4-triazol-3-one;

(5) 5-n-butyl-2,4-dihydro-4-[[2′-[N-(1-methylcyclobutanecarbonyl)sulfamoyl]biphenyl-4-yl]-methyl]-2-[2-(trifluoromethyl)phenyl]-3H-1,2,4-triazol-3-one;

(6) 5-n-butyl-2,4-dihydro-4-[[2′-[N-[(R)-2,2-dimethylcyclopropanecarbonyl]sulfamoyl]biphenyl-4-yl]methyl]-2-[2-(trifluoromethyl)phenyl]-3H-1,2,4-triazol-3-one;

(7) 5-n-butyl-4-[[2′-[N-(2,6-dichlorobenzoyl)sulfamoyl]biphenyl-4-yl]methyl]-2,4-dihydro-2-[2-(trifluoromethyl)phenyl]-3H-1,2,4-triazol-3-one;

(8) 5-n-butyl-4-[[2′-[N-(2-chloro-6-fluorobenzoyl)-sulfamoyl]biphenyl-4-yl]methyl]-2,4-dihydro-2-[2-(trifluoromethyl)phenyl]-3H-1,2,4-triazol-3-one;

(9) 5-n-butyl-4-[[2′-[N-(2-chloro-6-methylbenzoyl)-sulfamoyl]biphenyl-4-yl]methyl]-2,4-dihydro-2-[2-(trifluoromethyl)phenyl]-3H-1,2,4-triazol-3-one;

(10) 5-n-butyl-4-[[2′-[N-(5-chloro-2-furoyl)sulfamoyl]biphenyl-4-yl]methyl]-2,4-dihydro-2-[2-(trifluoromethyl)phenyl]-3H-1,2,4-triazol-3-one;

(11) 5-n-butyl-4-[[2′-[N-(4-chlorooxazole-5-carbonyl)sulfamoyl]biphenyl-4-yl]methyl]-2,4-dihydro-2-[2-(trifluoromethyl)phenyl]-3H-1,2,4-triazol-3-one;

(12) 5-n-butyl-2,4-dihydro-4-[[2′-[N-(1,3-dithiane-2-carbonyl)sulfamoyl]biphenyl-4-yl]methyl]-2-[2-(trifluoromethyl)phenyl]-3H-1,2,4-triazol-3-one;

(13) 4-[[2′-[N-(1-adamantanecarbonyl)sulfamoyl]biphenyl-4-yl]methyl]-5-n-butyl-2,4-dihydro-2-[2-(trifluoromethyl)phenyl]-3H-1,2,4-triazol-3-one;

(14) 2-[5-(benzoylamino)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one;

(15) 5-n-butyl-4-[[2′-[N-(2-chlorobenzoyl)sulfamoyl]-biphenyl-4-yl]methyl]-2-[2-chloro-5-(phenylacetyl)amino]-2,4-dihydro-3H-1,2,4-triazol-3-one;

(16) 5-n-butyl-4-[[2′-[N-(2-chlorobenzoyl)sulfamoyl]-biphenyl-4-yl]methyl]-2-[2-chloro-5-(hydrocinnamoyl)amino]-2,4-dihydro-3H-1,2,4-triazol-3-one;

(17) 2-[5-(benzylamino)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one;

(18) 5-n-butyl-2-[5-(butyrylamino)-2-chlorophenyl]-4-[[2′-[N-(2-chlorobenzoyl)sulfamoyl]biphenyl-4-yl]methyl]-2,4-dihydro-3H-1,2,4-triazol-3-one;

(19) 5-n-butyl-4-[[2′-[N-(2-chlorobenzoyl)sulfamoyl]-biphenyl-4-yl]methyl]-2-[2-chloro-5-(isovalerylamino)phenyl]-2,4-dihydro-3H-1,2,4-triazol-3-one;

(20) 5-n-butyl-4-[[2′-[N-(2-chlorobenzoyl)sulfamoyl]-biphenyl-4-yl]methyl]-2,4-dihydro-2-[5-(propionylamino)-2-(trifluoromethyl)phenyl]-3H-1,2,4-triazol-3-one;

(21) 5-n-butyl-2-[5-(butyrylamino)-2-(trifluoromethyl)phenyl]-4-[[2′-[N-(2-chlorobenzoyl)sulfamoyl ]biphenyl-4-yl]methyl]-2,4-dihydro-3H-1,2,4-triazol-3-one;
(22) 5-n-butyl-4-[[2′-[N-(2-chlorobenzoyl)sulfamoyl]-biphenyl-4-yl]methyl]-2,4-dihydro-2-[2-(trifluoromethyl)-5-(valerylamino)phenyl]-3H-1,2,4-triazol-3-one;
(23) 5-n-butyl-4-[[2′-[N-(2-chlorobenzoyl)sulfamoyl]biphenyl-4-yl]methyl]-2-[2-chloro-5-(N-methylpropionylamino )phenyl]-2,4-dihydro-3H-1,2,4-triazol-3-one;
(24) 5-n-butyl-4-[[2′-[N-(2-chlorobenzoyl)sulfamoyl]-biphenyl-4-yl]methyl]-2-[2-chloro-5-(N-methylbutyrylamino)phenyl]-2,4-dihydro-3H-1,2,4-triazol-3-one;
(25) 5-n-butyl-4-[[2′-[N-(2-chlorobenzoyl)sulfamoyl]biphenyl-4-yl]methyl]-2,4-dihydro-2-[3-(N-methylpropionylamino)phenyl]-3H-1,2,4-triazol-3-one;
(26) 5-n-butyl-4-[[2′-[N-(2-chlorobenzoyl)sulfamoyl]biphenyl-4-yl]methyl]-2,4-dihydro-2-[3-($N^3$-isopropyl-$N^3$-methylureido)phenyl]-3H-1,2,4-triazol-3-one;
(27) 5-n-butyl-2-[5-(N-n-butyl-N-methylcarbamoyl)-2-chlorophenyl]-4-[[2′-[N-(2-chlorobenzoyl)sulfamoyl]biphenyl-4-yl]methyl]-2,4-dihydro-3H-1,2,4-triazol-3-one;
(28) 2-[2-bromo-5-(butyrylamino)phenyl]-5-n-butyl-4-[[2′-[N-(2-chlorobenzoyl)sulfamoyl]biphenyl-4-yl]methyl]-2,4-dihydro-3H-1,2,4-triazol-3-one;
(29) 2-[5-(butyrylamino)-2-methylphenyl]-5-n-butyl-4-[[2′-[N-(2-chlorobenzoyl)sulfamoyl]biphenyl-4-yl]methyl]-2,4-dihydro-3H-1,2,4-triazol-3-one;
(30) 5-n-butyl-2-[2-chloro-5-(valerylamino)phenyl]-2,4-dihydro-4-[[2′-[N-(3-methyl-2-furoyl)sulfamoyl]biphenyl-4-yl]methyl]-3H-1,2,4-triazol-3-one;
(31) 5-n-butyl-4-[[2′-[N-(3-chloro-2-thiophenecarbonyl)sulfamoyl]biphenyl-4-yl]methyl]-2-[2-chloro-5-(valerylamino)phenyl]-2,4-dihydro-3H-1,2,4-triazol-3-one;
(32) 5-n-butyl-2-[2-chloro-5-(valerylamino)phenyl]-4-[[2′-[N-(3,4-dichloro-2-furoyl)sulfamoyl]biphenyl-4-yl]methyl]-2,4-dihydro-3H-1,2,4-triazol-3-one;
(33) 5-n-butyl-4-[[2′-[N-(3-chloro-2-furoyl)sulfamoyl]biphenyl-4-yl]methyl]-2-[2-chloro-5-(valerylamino)phenyl]-2,4-dihydro-3H-1,2,4-triazol-3-one;
(34) 5-n-butyl-2-[2-chloro-5-(valerylamino)phenyl]-2,4-dihydro-4-[[2′-[N-(3,3-dimethylbutyryl)sulfamoyl]biphenyl-4-yl]methyl]-3H-1,2,4-triazol-3-one;
(35) 5-n-butyl-2-[2-chloro-5-(valerylamino)phenyl]-4-[[2′-[N-(2,5-dichlorobenzoyl)-sulfamoyl]biphenyl-4-yl]methyl]-2,4-dihydro-3H-1,2,4-triazol-3-one;
(36) 5-n-butyl-2-[2-chloro-5-(valerylamino)phenyl]-2,4-dihydro-4-[[2′-[N-(4-methyl-5-oxazolecarbonyl)sulfamoyl]biphenyl-4-yl]methyl]-3H-1,2,4-triazol-3-one;
(37) 5-n-butyl-2-[2-chloro-5-(valerylamino)phenyl]-2,4-dihydro-4-[[2′-[N-(1,3-dithiane-2-carbonyl)sulfamoyl]biphenyl-4-yl]methyl]-3H-1,2,4-triazol-3-one;
(38) 5-n-butyl-2-(5-butyryl-2-chlorophenyl)-4-[[2′-[N-(2-chlorobenzoyl)sulfamoyl]biphenyl-4-yl]-methyl]-2,4-dihydro-3H-1,2,4-triazol-3-one;
(39) 5-n-butyl-4-[[2′-[N-(2-chlorobenzoyl)sulfamoyl]-biphenyl-4-yl]methyl]-2-[2-chloro-3-(valerylamino)phenyl]-2,4-dihydro-3H-1,2,4-triazol-3-one;
(40) 5-n-butyl-4-[[2′-[N-(2-chlorobenzoyl)sulfamoyl]-biphenyl-4-yl]methyl]-2-[2-chloro-3-(N-n-butylcarbamoyl) phenyl]-2,4-dihydro-3H-1,2,4-triazol-3-one;
(41) 5-n-butyl-4-[[2′-[N-(2-chlorobenzoyl)sulfamoyl]-biphenyl-4-yl]methyl]-2,4-dihydro-2-[2-methyl-3-(valerylamino) phenyl]-3H-1,2,4-triazol-3-one;
(42) 5-n-butyl-2-[2-chloro-5-(valerylamino)phenyl]-4-[[2′-N-(2-chlorobenzoyl)sulfamoyl]-5′-ethylbiphenyl-4-yl]methyl]-2,4-dihydro-3H-1,2,4-triazol-3-one;
(43) 5-n-butyl-2-[2-chloro-5-(propionylamino)phenyl]-4-[[2′-[N-(2-chlorobenzoyl)sulfamoyl]-5′-n-propylbiphenyl-4-yl]methyl]-2,4-dihydro-3H-1,2,4-triazol-3-one;
(44) 5-n-butyl-4-[[2′-[N-(2-chlorobenzoyl)sulfamoyl]-5′-ethylbiphenyl-4-yl]methyl]-2-[2-chloro-5-(N-n-butylcarbamoyl)phenyl]-2,4-dihydro-3H-1,2,4-triazol-3-one;
(45) 2-[5-(N-benzylcarbamoyl)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one;
(46) 2-[5-(N-benzyl-N-methylcarbamoyl)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one,

EXAMPLE 23

Typical Pharmaceutical Compositions Containing a Compound of the Invention

**A**: Dry Filled Capsules Containing 50 mg of Active Ingredient Per Capsule

| Ingredient | Amount per capsule (mg) |
|---|---|
| 4-[[2'-(N-Benzoylsulfamoyl)-biphenyl-4-yl]methyl]-5-n-butyl-2,4-dihydro-2-[2-(trifluoromethyl)phenyl]-3H-1,2,4-triazole-3-one, | 50 |
| Lactose | 149 |
| Magnesium stearate | 1 |
| Capsule (size No. 1) | 200 |

The active compound, 4-[[2'-(N-benzoylsulfamoyl)biphenyl-4-yl]methyl]-5-n-butyl-2,4-dihydro-2-[2-(trifluoromethyl)phenyl]-3H-1,2,4-triazole-3-one, can be reduced to a No. 60 powder and the lactose and magnesium stearate can then be passed through a No. 60 blotting cloth onto the powder. The combined ingredients can then be mixed for about 10 minutes and filled into a No. 1 dry gelatin capsule.

**B**: Tablet

A typical tablet contains the active compound (25 mg), pregelatinized starch USP (82 mg), microcrystalline cellulose (82 mg) and magnesium stearate (1 mg).

**C**: Combination Tablet

A typical combination tablet contains, for example, a diuretic such as hydrochlorothiazide and consist of the active compound of this invention (7.5 mg), hydrochlorothiazide (50 mg) pregelatinized starch USP (82 mg), microcrystalline cellulose (82 mg) and magnesium stearate (1 mg).

**D**: Suppository

Typical suppository formulations for rectal administration contains the active compound (1-25 mg), butylated hydroxyanisole (0.08-1.0 mg), disodium calcium edetate (0.25-0.5 mg), and polyethylene glycol (775-1600 mg). Other suppository formulations can be made by substituting, for example, butylated hydroxytoluene (0.04-0.08 mg) for the disodium calcium edetate and a hydrogenated vegetable oil (675-1400 mg) such as Suppocire L, Wecobee FS, Wecobee M, Witepsols, and the like, for the polyethylene glycol. Further, these suppository formulations can also include another active ingredient such as another antihypertensive and/or a diuretic and/or an angiotensin converting enzyme and/or a calcium channel blocker in pharmaceutically effective amounts as described, for example, in C above.

**E**: Injection

A typical injectible formulation contains the active compound (5.42 mg), sodium phosphate dibasic anhydrous (11.4 mg) benzyl alcohol (0.01 ml) and water for injection (1.0 ml). Such an injectible formulation can also include a pharmaceutically effective amount of another active ingredient such as another antihypertensive and/or a diuretic and/or an angiotensin converting enzyme inhibitor and/or a calcium channel blocker.

## Claims

1. A compound of structural formula:

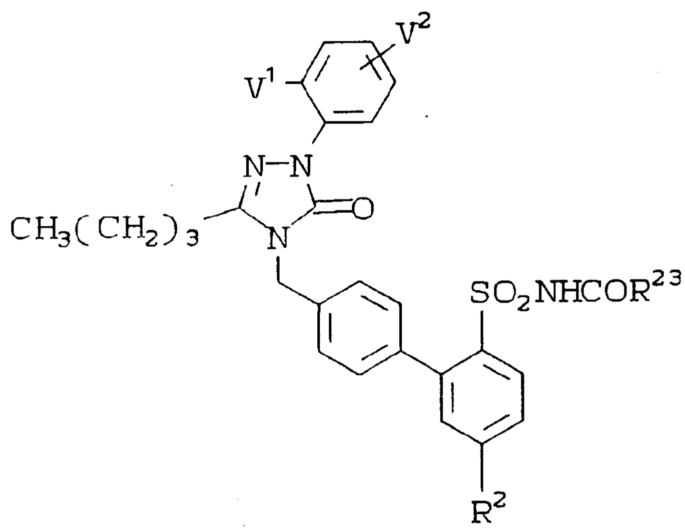

or a pharmaceutically acceptable salt thereof, wherein

$V^1$ is H, $CH_3$, $CF_3$, or halogen with the proviso that $V^1$ is $CF_3$ when $V_2$ is H;

$V^2$ is a group at the 3-, 4-, or 5-position selected from:

(a) H;
(b) $-NO_2$;
(c) $-NR^{10}R^{21}$;
(d) $-N(CH_2CH_2)_2L$;
(e) $-NR^{21}COR^{22}$:
(f) $-NR^{21}CO_2R^{22}$;
(g) $-NR^{21}CONR^{21}R^{22}$;
(h) $-NR^{21}CON(CH_2CH_2)_2L$;
(i) $-CONR^{21}R^{22}$;
(j) $-CON(CH_2CH_2)_2L$; and
(k) $-CO-(C_1-C_5\text{-alkyl})$;

wherein L is a single bond, $CH_2$, O, $S(O)_p$, or $NR^9$ wherein p is 0 to 2;

with the proviso that $V^2$ is restricred to (e)-(k) above when $V^1$ is H;

$R^2$ is H, $C_1$-$C_4$-alkyl, $C_1$-$C_4$-alkoxy or halo;

$R^9$ is H, $C_1$-$C_5$-alkyl, aryl or $-CH_2$-aryl;

$R^{10}$ is H or $C_1$-$C_4$-alkyl;

$R^{21}$ is H or $R^{22}$ wherein

$R^{22}$ is:

(a) $C_1$-$C_6$-alkyl, $C_2$-$C_6$-alkenyl, or $C_2$-$C_6$-alkynyl, each of which is unsubstituted or substituted with one or more substituents selected from the group consisting of aryl, heteroaryl, $C_3$-$C_6$-cycloalkyl, halo, -OH, $-O-C_1-C_4$-alkyl, $-S-C_1-C_4$-alkyl, -O-phenyl, or S-phenyl,
(b) $C_3$-$C_7$-cycloalkyl unsubstituted or substituted with one or more substituents selected from the group consisting of $C_1$-$C_4$-alkyl, halo or phenyl;
(c) aryl;
(d) heteroaryl;

$R^{23}$ is:

(a) phenyl, unsubstituted or substituted with one or two substituents selected from Cl, -Br, -F, -I, $-CH_3$ and $-CF_3$, at least one of which occupies an ortho-position;
(b) heteroaryl, such as furan-2-yl, thiophen-2-yl, benzo[b]furan-2-yl, benzo[b]thiophen-2-yl, furan-3-yl, thiopen-3-yl, or oxazol-5-yl, unsubstituted or substituted with one or two substituents selected from -Cl, -Br, -F, -I, $-CH_3$ and $CF_3$ wherein at least one of the substituents is located adjacent to the carbonyl substituent and/or to a ring heteroatom;
(c) branched $C_3$-$C_6$-alkyl;

(d) $C_3$-$C_7$-cycloalkyl, unsubstituted or substituted at the 1-and/or 2-position with one to three substituents selected from -Cl, -Br, -F, -I, $-CH_3$ and $-CH_2CH_3$;
(e) $C_7$-$C_8$-bi- or tricycloalkyl;
(f) saturated 5- or 6- membered heterocyclyl linked through a carbon atom and containing one or two heteroatoms selected from oxygen and sulfur such as tetrahydrofuryl, 1,3-dithiolan-2-yl, or 1,3-dithian-2-yl.

**2.** The compound of Claim 1 or a pharmaceutically acceptable salt thereof wherein:
$V^1$ is H, $CH_3$, $CF_3$, or halogen;
$V^2$ is a group at the 3-, 4-, or 5-position selected from:
(a) $-NO_2$,
(b) $-NR^{10}R^{21}$,
(c) $-N(CH_2CH_2)_2L$,
(d) $-NR^{21}COR^{22}$,
(e) $-NR^{21}CO_2R^{22}$,
(f) $-NR^{21}CONR^{21}R^{22}$,
(g) $-NR^{21}CON(CH_2CH_2)_2L$,
(h) $-CONR^{21}R^{22}$,
(i) $-CON(CH_2CH_2)_2L$ and
(j) -CO-($C_1$-$C_5$-alkyl);
wherein L is a single bond, $CH_2$, O, S(O)p, or $NR^9$ and p is 0 to 2; with the proviso that $V^2$ is restricted to (d)-(j) above when $V^1$ is H.

**3.** The compound of Claim 2 or a pharmaceutically acceptable salt thereof, wherein:
$V^1$ is $CH_3$, $CF_3$, or halogen;
$V^2$ is a group at the 3- or 5-position selected from:
(a) $-NR^{21}COR^{22}$,
(b) $-NR^{21}CO_2R^{22}$,
(c) $-NR^{21}CONR^{21}R^{22}$,
(d) $-NR^{21}CON(CH_2CH_2)_2L$,
(e) $-CONR^{21}R^{22}$,
(f) $-CON(CH_2CH_2)_2L$ and
(g) -CO-($C_1$-$C_5$-alkyl).

**4.** The compound of Claim 3, or a pharmaceutically acceptable salt thereof, wherein:
$V^1$ is $CF_3$, or halogen;
$V^2$ is a group at the 5-position selected from:
(a) $-NR^{21}COR^{22}$,
(b) $-NR^{21}CO_2R^{22}$,
(c) $-NR^{21}CONR^{21}R^{22}$,
(d) $-NR^{21}CON(CH_2CH_2)_2L$,
(e) $-CONR^{21}R^{22}$,
(f) $-CON(CH_2CH_2)_2L$ and
(g) -CO-($C_1$-$C_5$-alkyl);
$R^2$ is H or $C_1$-$C_4$-alkyl;
$R^{21}$ is H or $CH_3$;
$R^{22}$ is
(a) $C_1$-$C_6$-alkyl, unsubstituted or substituted with one or more substituents selected from the group consisting of aryl, heteroaryl, $C_3$-$C_6$-cycloalkyl, -O-$C_1$-$C_4$-alkyl, -S-$C_1$-$C_4$-alkyl, -O-phenyl, or S-phenyl;
(b) $C_3$-$C_7$-cycloalkyl unsubstituted or substituted with one or more substituents selected from the group consisting of $C_1$-$C_4$-alkyl or phenyl;
(c) aryl; or
(d) heteroaryl.

**5.** The compound of Claim 1 or a pharmaceutically acceptable salt thereof, wherein
$V^1$ is $CF_3$ and $V^2$ is H.

**6.** The compound of Claim 1 which is:

(1) 4-[[2′-(N-benzoylsulfamoyl)biphenyl-4-yl)methyl)-5-n-butyl-2,4-dihydro-2-[2-(trifluoromethyl)-phenyl)-3H-1,2,4-triazol-3-one;
(2) 5-n-butyl-2,4-dihydro-4-[[2′-[N-[(S)-2,2-dimethylcyclopropanecarbonyl]sulfamoyl]biphenyl-4-yl]methyl]-2-[2-(trifluoromethyl)phenyl]-3H-1,2,4-triazol-3-one;
(3) 5-n-butyl-2,4-dihydro-4-[[2′-[N-(2-furoyl)sulfamoyl]biphenyl-4-yl]methyl]-2-[2-trifluoromethyl)phenyl]-3H-1,2,4-triazol-3-one;
(4) 5-n-butyl-2,4-dihydro-4-[[2′-[N-(3-methyl-2-furoyl)sulfamoyl]biphenyl-4-yl]methyl]-2-[2-(trifluoromethyl)phenyl]-3H-1,2,4-triazol-3-one;
(5) 5-n-butyl-2,4-dihydro-4-[[2′-[N-(2-thiophenecarbonyl)sulfamoyl]biphenyl-4-yl]methyl]-2-[2-(trifluoromethyl)phenyl]-3H-1,2,4-triazol-3-one;
(6) 5-n-butyl-2,4-dihydro-4-[[2′-[N-(5-methyl-2-thiophenecarbonyl)sulfamoyl]biphenyl-4-yl]methyl]-2-[2-(trifluoromethyl)phenyl]-3H-1,2,4-triazol-3-one;
(7) 5-n-butyl-2,4-dihydro-4-[[2′-[N-(2-methylbenzoyl)sulfamoyl]biphenyl-4-yl]methyl]-2-[2-(trifluoromethyl)phenyl]-3H-1,2,4-triazol-3-one;
(8) 5-n-butyl-4-[[2′-[N-(3-chloro-2-thiophenecarbonyl)sulfamoyl]biphenyl-4-yl]methyl]-2,4-dihydro-2-[2-(trifluoromethyl)phenyl]-3H-1,2,4-triazol-3-one;
(9) 5-n-butyl-4-[[2′-[N-(3,4-dichloro-2-furoyl)sulfamoyl]biphenyl-4-yl]methyl]-2,4-dihydro-2-[2-(trifluoromethyl)phenyl]-3H-1,2,4-triazol-3-one;
(10) 5-n-butyl-4-[[2′-[N-(2,2-difluorocyclopropanecarbonyl)sulfamoyl]biphenyl-4-yl]methyl]-2,4-dihydro-2-[2-(trifluoromethyl]phenyl]-3H-1,2,4-triazol-3-one;
(11) 5-n-butyl-2,4-dihydro-4-[[2′-[N-[2-(trifluoromethyl)benzoyl]sulfamoyl]biphenyl-4-yl]methyl]-2-[2-(trifluoromethyl)phenyl]-3H-1,2,4-triazol-3-one;
(12) 5-n-butyl-2,4-dihydro-4-[[2′-[N-(3,3-dimethylbutyryl)sulfamoyl]biphenyl-4-yl]methyl]-2-[2-trifluoromethyl)phenyl]-3H-1,2,4-triazol-3-one;
(13) 4-[[2′-[N-(benzofuran-2-carbonyl)sulfamoyl)biphenyl-4-yl]methyl]-5-n-butyl-2,4-dihydro-3-[2-(trifluoromethyl)phenyl]-3H-1,2,4-triazol-3-one;
(14) 5-n-butyl-2,4-dihydro-4-[[2′-[N-(1-methylcyclopropanecarbonyl)sulfamoyl]biphenyl-4-yl-methyl]-2-[2-(trifluoromethyl)phenyl]-3H-1,2,4-triazol-3-one;
(15) 5-n-butyl-4-[[2′-[N-(2-chlorobenzoyl)sulfamoyl)biphenyl-4-yl]methyl]-2,4-dihydro-2-[2-(trifluoromethyl)phenyl]-3H-1,2,4-triazol-3-one;
(16) 5-n-butyl-2,4-dihydro-4-[[2′-[N-(2-fluorobenzoyl)sulfamoyl]biphenyl-4-yl]methyl]-2-[2-(trifluoromethyl)phenyl]-3H-1,2,4-triazol-3-one;
(17) 5-n-butyl-4-[[2′-[N-(2-chlorobenzoyl)sulfamoyl]-biphenyl-4-yl]methyl]-2-[2-chloro-5-(propionylamino)phenyl]-2,4-dihydro-3H-1,2,4-triazol-3-one;
(18) 5-n-butyl-4-[[2′-[N-(2-chlorobenzoyl)sulfamoyl]-biphenyl-4-yl]methyl]-2-[2-chloro-5-(valerylamino)phenyl]-2,4-dihydro-3H-1,2,4-triazol-3-one;
(19) 5-n-butyl-4-[[2′-[N-(2-chlorobenzoyl)sulfamoyl]-biphenyl-4-yl]methyl]-2-[2-chloro-5-[(propoxycarbonyl)amino]phenyl]-2,4-dihydro-3H-1,2,4-triazol-3-one;
(20) 5-n-butyl-4-[[2′-[N-(2-chlorobenzoyl)sulfamoyl]-biphenyl-4-yl]methyl]-2-[2-chloro-5-($N^3$-propylureido)phenyl]-2,4-dihydro-3H-1,2,4-triazol-3-one;
(21) 5-n-butyl-2-[5-(N-n-butylcarbamoyl)-2-chlorophenyl]-4-[[2′-[N-(2-chlorobenzoyl)sulfamoyl]biphenyl-4-yl]methyl]-2,4-dihydro-3H-1,2,4-triazol-3-one;
(22) 2-[5-(benzoylamino)-2-chlorophenyl]-5-n-butyl-4-[[2′-[N-(2-chlorobenzoyl)sulfamoyl]biphenyl-4-yl]methyl]-2,4-dihydro-3H-1,2,4-triazol-3-one;
(23) 5-n-butyl-4-[[2′-[N-(3-chloro-2-furoyl)sulfamoyl]biphenyl-4-yl]methyl]-2,4-dihydro-2-[2-(trifluoromethyl)phenyl]-3H-1,2,4-triazol-3-one;
(24) 5-n-butyl-4-[[2′-[N-(2-chlorobenzoyl)sulfamoyl]-biphenyl-4-yl]methyl]-2-[2-chloro-5-(phenylacetyl)amino]-2,4-dihydro-3H-1,2,4-triazol-3-one;
(25) 5-n-butyl-4-[[2′-[N-(2-chlorobenzoyl)sulfamoyl]-biphenyl-4-yl]methyl]-2-[2-chloro-5-(hydrocinnamoyl)amino]-2,4-dihydro-3H-1,2,4-triazol-3-one;
(26) 5-n-butyl-2-[5-(butyrylamino)-2-chlorophenyl]-4-[[2′-[N-(2-chlorobenzoyl)sulfamoyl]biphenyl-4-yl]methyl]-2,4-dihydro-3H-1,2,4-triazol-3-one;
(27) 5-n-butyl-4-[[2′-[N-(2-chlorobenzoyl)sulfamoyl]-biphenyl-4-yl]methyl]-2-[2-chloro-5-(isovalerylamino)phenyl]-2,4-dihydro-3H-1,2,4-triazol-3-one;
(28) 5-n-butyl-4-[[2′-[N-(2-chlorobenzoyl)sulfamoyl]-biphenyl-4-yl]methyl]-2,4-dihydro-2-[2-(trifluoromethyl)-5-(valerylamino)phenyl]-3H-1,2,4-triazol-3-one;
(29) 5-n-butyl-4-[[2′-[N-(3-chloro-2-furoyl)sulfamoyl)biphenyl-4-yl]methyl]-2-[2-chloro-5-(valerylamino)phenyl]-2,4-dihydro-3H-1,2,4-triazol-3-one;

(30) 5-n-butyl-2-[2-chloro-5-(valerylamino)phenyl]-4-[[2′-[N-(2-chlorobenzoyl)sulfamoyl]-5′-ethylbiphenyl-4-yl]methyl]-2,4-dihydro-3H-1,2,4-triazol-3-one;
(31) 5-n-butyl-4-[[2′-[N-(2-chlorobenzoyl)sulfamoyl]-5′-ethylbiphenyl-4-yl]methyl]-2-[2-chloro-5-(N-n-butylcarbamoyl)phenyl]-2,4-dihydro-3H-1,2,4-triazol-3-one;
(32) 2-[5-(N-benzylcarbamoyl)-2-chlorophenyl]-5-n-butyl-4-[[2′-[N-(2-chlorobenzoyl)sulfamoyl)biphenyl-4-yl]methyl]-2,4-dihydro-3H-1,2,4-triazol-3-one:
(33) 2-[5-(N-benzyl-N-methylcarbamoyl)-2-chlorophenyl]-5-n-butyl-4-[[2′-[N-(2-chlorobenzoyl]sulfamoyl)biphenyl-4-yl]methyl]-2,4-dihydro-3H-1,2,4-triazol-3-one.

**7.** A pharmaceutical composition useful in the treatment of hypertension which comprises a pharmaceutically acceptable carrier and a pharmaceutically effective amount of a compound of Claim 1.

**8.** The composition of Claim 7 which includes another antihypertensive selected from a diuretic selected from hydrochlorothiazide, chlorothiazide, chlorthalidone, methyclothiazide, furosemide, ethacrynic acid, triamterene, amiloride and spironolactone; a calcium channel blocker, selected from diltiazem, felodipine, nifedipine, nitrendipine and verapamil; a β-adrenergic antagonist selected from timolol, atenolol, metoprolol, propanolol, nadolol and pindolol; an angiotensin converting enzyme inhibitor selected from enalapril, lisinopril, captopril, ramipril, quinapril and zofenopril; a renin inhibitor selected from A-69729 and FK 744; an α-adrenergic antagonist selected from prazosin, doxazosin, and terazosin; a sympatholytic agent selected from methyldopa, clonidine and guanabenz the atriopeptidase inhibitor, UK-79300; the serotonin antagonist, ketanserin; the $A_2$-adenosine receptor agonist CGS 22492C; a potassium channel agonist selected from pinacidil and cromakalim; or another antihypertensive drug selected from reserpine, minoxidil, guanethidine, hydralazine hydrochloride and sodium nitroprusside; or combinations of the above-named drugs.

**9.** The use of a compound of Claim 1 for the manufacture of a medicament for treating hypertension.

**10.** An ophthalmological formulation for the treatment of ocular hypertension comprising an ophthalmologically acceptable carrier and an effective ocular antihypertensive amount of a compound of Claim 1.

**11.** The use of a compound of Claim 1 for the manufacture of a medicament for treating ocular hypertension.

European Patent Office

# EUROPEAN SEARCH REPORT

Application Number

EP 92 30 6106

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | EP-A-0 412 594 (MERCK & CO INC)<br>* page 59, line 52 - line 58; claims *<br>--- | 1,7-11 | C07D249/12<br>C07D407/12<br>C07D409/12<br>C07D413/12<br>A61K31/41 |
| A | EP-A-0 409 332 (MERCK & CO INC)<br>* claims *<br>----- | 1,7-11 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| BERLIN | 01 SEPTEMBER 1992 | VAN AMSTERDAM L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)